# EUROPEAN PATENT APPLICATION

(11) **EP 3 978 497 A1**
(43) Date of publication of application: **06.04.2022**
(21) Application number: 20813589.7
(22) Date of filing: 29.05.2020
(51) Int. Cl.: C07D 487/04, C07D 409/04, C07D 209/52, C07D 405/04, A61K 31/407, A61K 31/55, A61K 31/403, A61K 31/4709, A61P 25/04, A61P 25/22, A61P 25/24, A61P 25/00

(54) **FUSED RING COMPOUND, PREPARATION METHOD THEREFOR AND USE THEREOF**

(30) Priority: 30.05.2019 CN 201910462455
(71) Applicant: Shanghai Institute of Materia Medica, Chinese Academy of Sciences, Pudong, Shanghai 201203 (CN); Topharman Shanghai Co., Ltd., Pudong, Shanghai 201203 (CN); Lian V Nantong Co., Ltd., Linjiang Town, Haimen District Nantong, Jiangsu 226100 (CN)
(72) Inventor: SHEN, Jingshan, Shanghai 201203 (CN); HE, Yang, Shanghai 201203 (CN); WU, Chunhui, Shanghai 201203 (CN); YANG, Feipu, Shanghai 201203 (CN); WANG, Zhen, Shanghai 201203 (CN); ZHANG, Junchi, Shanghai 201203 (CN); ZHU, Fuqiang, Shanghai 201203 (CN); QIN, Hongjian, Shanghai 201203 (CN)
(74) Representative: Petraz, Gilberto Luigi
(86) International application number: PCT/CN2020/093279
(87) International publication number: WO 2020/239073

(57) **Abstract**

The present invention relates to a fused ring compound represented by general formula (I), a stereoisomer or a pharmaceutically acceptable salt thereof, a preparation method therefor, a pharmaceutical composition and use thereof. The compound provided in the present invention is useful for treating, preventing and/or controlling various nervous system disorders. The compound provided in the present invention modulates one or more monoamine transporters, inhibits the reuptake of endogenous monoamines such as dopamine, 5-hydroxytryptamine, and norepinephrine (e.g., from the synaptic cleft), and/or modulates the 5-HT₃ receptor.

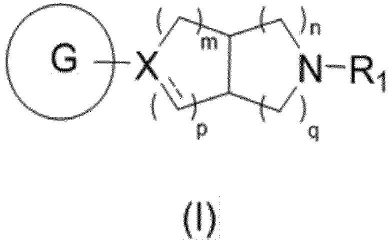

## Description

### Technical field

The invention belongs to the field of medicinal chemistry. Specifically, the present invention relates to a fused ring compound represented by formula (I), a stereoisomer or pharmaceutically acceptable salt thereof, a preparation method therefor, a pharmaceutical composition containing the same and its use in preparation of a drug for treatment of a central nervous system disease.

### Background Art

Depression is a widespread and persistent chronic disease, with a lifetime incidence of approximately 16% worldwide. In the 1950s, isoniazid and imipramine were accidentally found to have antidepressant effects in clinic, and they became the first-generation drugs to treat depression. Subsequently, the monoamine hypothesis was proposed in pharmacological research, and it postulates that the hypofunction of serotoninergic (5-HT), dopaminergic (DA) and noradrenergic (NA) nerves in the brain is responsible for depression, and the drugs which can improve the above-mentioned neurological function have antidepressant effects. The first-line therapeutic drugs currently used in clinic, such as selective 5-HT reuptake inhibitors (SSRIs) and 5-HT and NA reuptake inhibitors (SNRIs), are all based on the monoamine hypothesis. Although the development of antidepressants has made great progress, there are still many clinical needs, such as rapid onset of drugs, prevention of relapse, and improvement of cognitive damage in patients with depression. Although quiet a few drugs had been marketed in the field of major depression, only about 30-40% of patients respond to first-line treatment. These drugs still have no efficacy or low efficacy in more than 30% of patients. Therefore, it is still necessary to find a new type of antidepressant with good curative effect, low side effects and broad therapeutic spectrum.

### Summary of the Invention

### Object of the invention

It is an object of the present invention to provide a fused ring compound represented by formula (I), a stereoisomer or pharmaceutically acceptable salt thereof.

It is another object of the present invention to provide a method for preparing the fused ring compound represented by formula (I).

It is a further object of the present invention to provide a pharmaceutical composition comprising a therapeutically effective amount of one or more selected from the group consisting of the above fused ring compound represented by formula (I), a stereoisomer or pharmaceutically acceptable salt thereof.

It is yet another object of the present invention to provide use of the above fused ring compound represented by formula (I), a stereoisomer or pharmaceutically acceptable salt thereof, or the above pharmaceutical composition in preparation of a drug for prevention and/or treatment of a central nervous system disease.

It is yet another object of the present invention to provide a method for preventing and/or treating a central nervous system disease, characterized in that a therapeutically effective amount of one or more selected from the group consisting of the above compound represented by formula (I), a stereoisomer or pharmaceutically acceptable salt thereof, or the above pharmaceutical composition is administered to a subject.

According to one aspect of the present invention, provided is a fused ring compound represented by formula (I), a stereoisomer or pharmaceutically acceptable salt thereof: 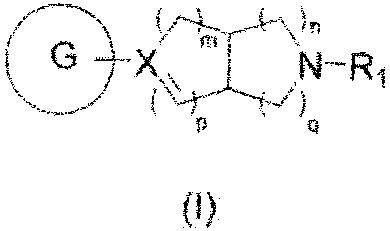 wherein
R₁ is hydrogen or C1 to C20 alkyl; preferably hydrogen or C1 to C10 alkyl; more preferably hydrogen or C1 to C6 alkyl;
m, n, p, q are each independently 0 or 1 or 2 or 3; preferably, m, n, p, q are each independently 1 or 2; provided that m+p is 1, 2, 3 or 4, N+q is 1, 2, 3 or 4, and m+p+n+q is 3, 4, 5, 6 or 7;
X is CR₂, N or C; when X is CR₂ or N, " 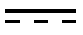 " connected with X represents a single bond, R₂ is hydrogen, hydroxyl or C1 to C6 alkoxy; when X is C, " " connected with X represents a double bond;
G ring is phenyl, biphenyl group, naphthyl, tetrahydronaphthyl, dihydroindenyl, monocyclic heterocyclyl or benzoheterocyclyl;
further, G ring is optionally substituted by one or more identical or different substituents; the substituent on G ring is halogen, oxo (=O), hydroxy, halo-Cl to C6 alkyl, C1 to C6 alkanoyl, C1 to C6 alkyl substituted by C1 to C6 alkoxy, C1 to C6 alkyl or C1 to C6 alkoxy, preferably, the substituent on the G ring is halogen, oxo (=O), hydroxy, halo-Cl to C4 alkyl, C1 to C4 alkanoyl, C1 to C4 alkyl substituted by C1 to C6 alkoxy, C1 to C4 alkyl or C1 to C4 alkoxy, more preferably, the substituent on the G ring is halogen, oxo (=O), hydroxy, acetyl, trifluoromethyl, methoxymethyl, methyl, ethyl, methoxy or ethoxy.

Preferably, the G ring is 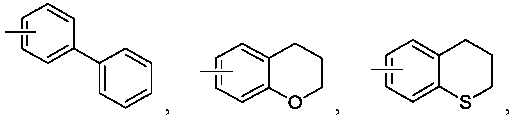 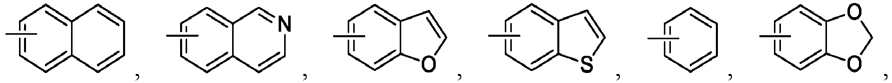 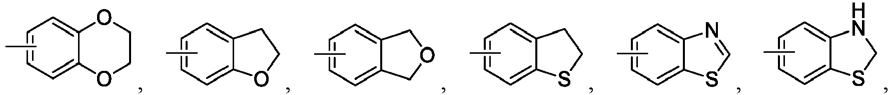 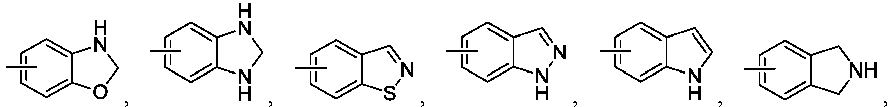 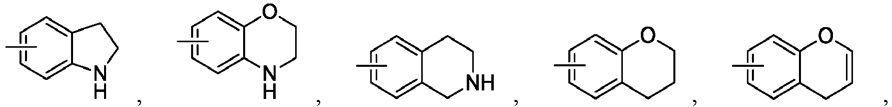 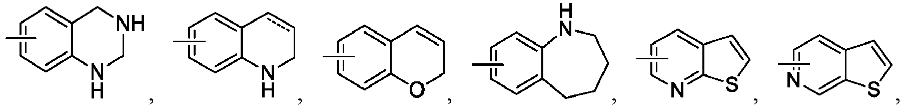 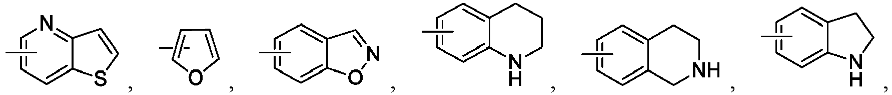 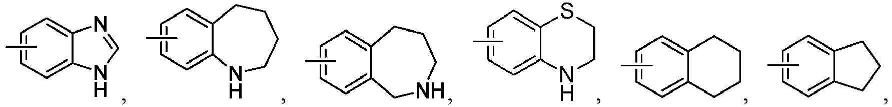 and the G ring is optionally substituted by one or more identical or different substituents, the substituent on the G ring is defined the same as previously defined.

The G ring is more preferably 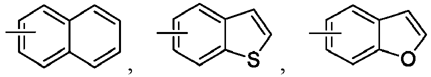 or 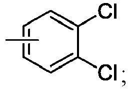 and the G ring is optionally substituted by one or more identical or different substituents, the substituent on the G ring is defined the same as previously defined.

The connection position of the above substituents may be any position that is available for connection on the ring, for example, the substituent on 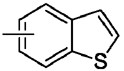 can represent 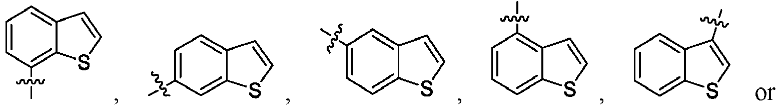 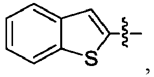 wherein, 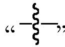 indicates the position of the connection.

In a preferred embodiment, in the fused ring compound represented by the formula (I) of the present invention: 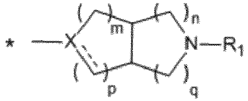 is a group selected from the group consisting of formulae S-1 to S-41: 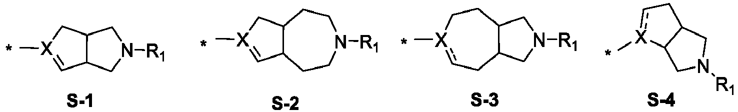 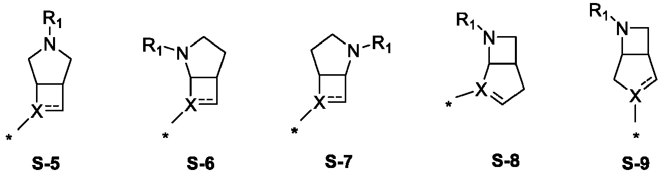 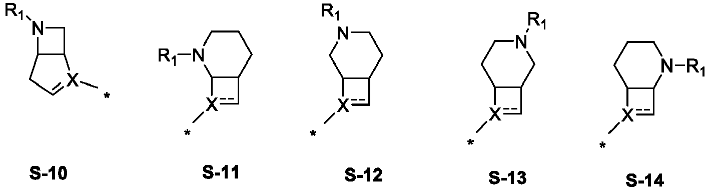 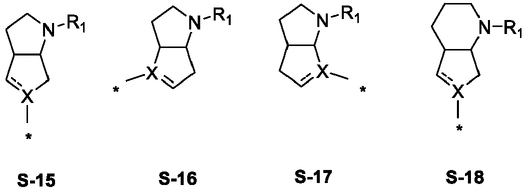 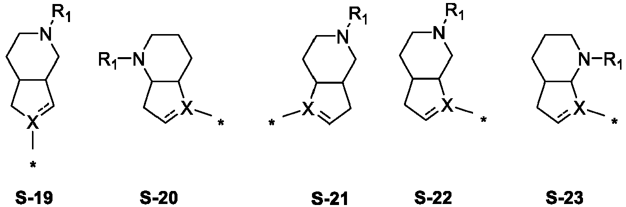 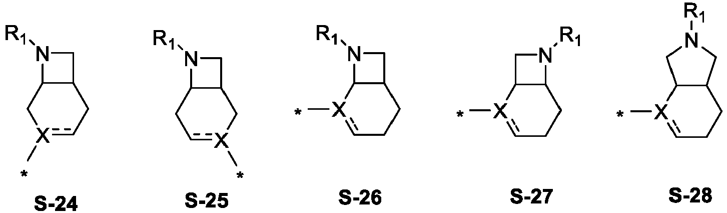 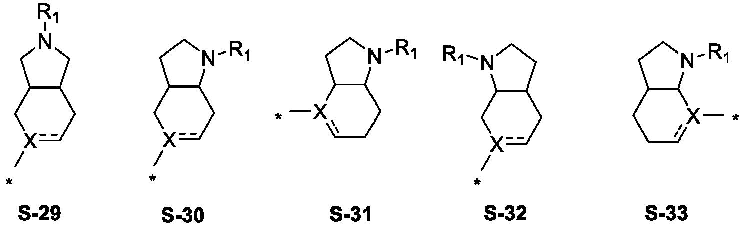 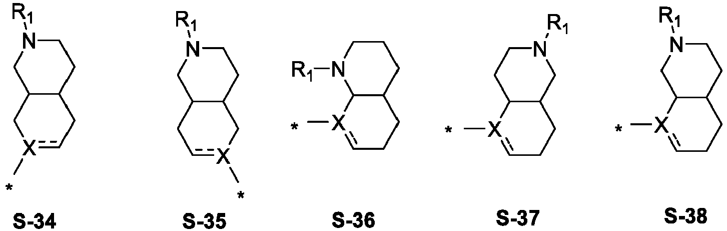 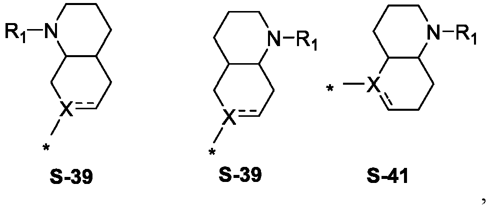 wherein, X and R₁ are defined the same as above.

In a preferred embodiment, in the fused ring compound represented by the formula (I) of the present invention: 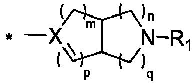 is a group selected from the group consisting of formulae S-1a to S-41a: 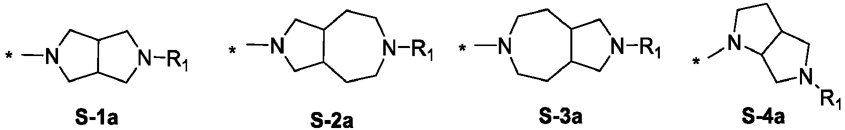 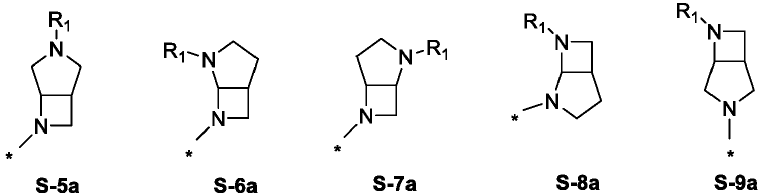 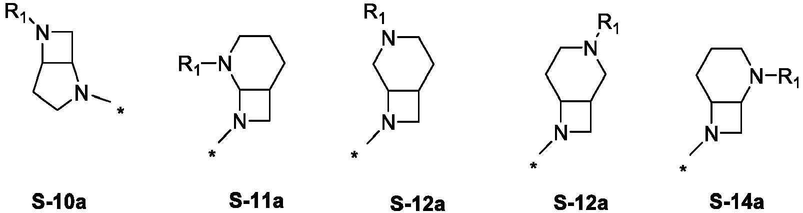 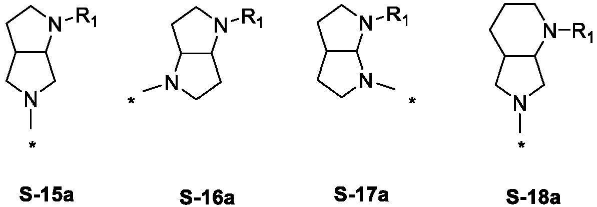 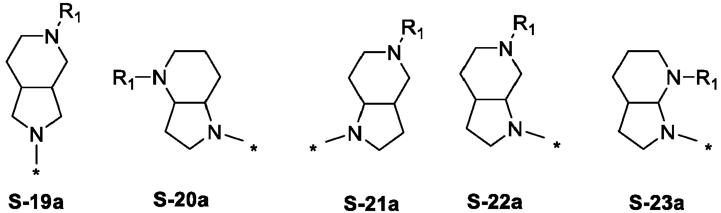 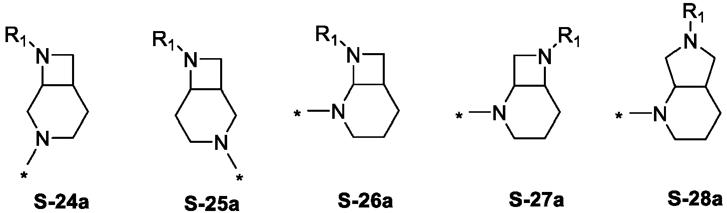 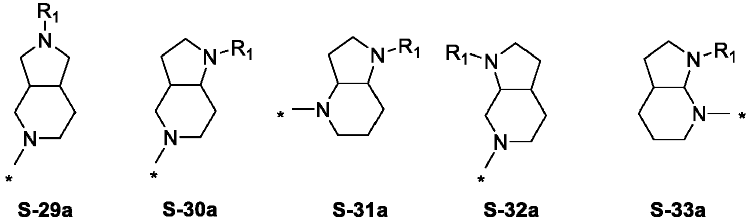 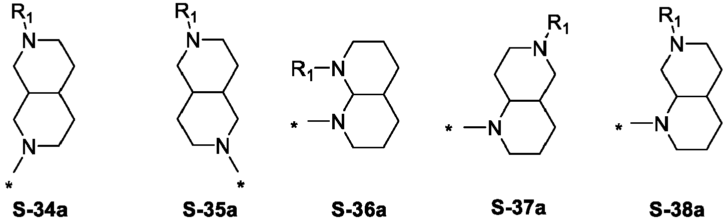 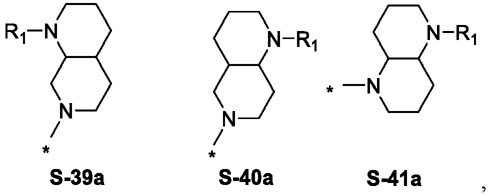 wherein, R₁ is defined the same as above.

In a preferred embodiment, in the fused ring compound represented by the formula (I) of the present invention: 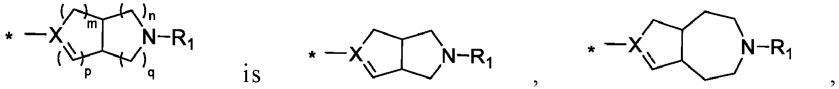 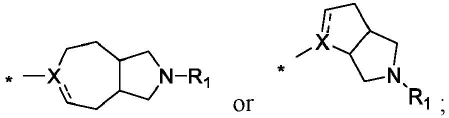 more preferably, 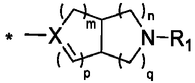 is 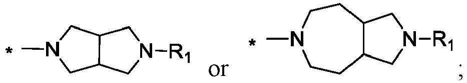 most preferably, 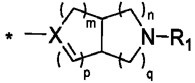 is 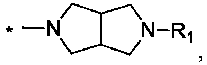 wherein, R₁, X and G are defined the same as above, and ^{∗} indicates the position binding to the G ring part.

In 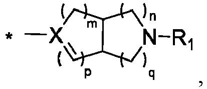 the bridging carbon atoms can provide a pair of chiral centers. The present invention relates to a compound wherein 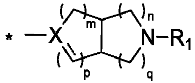 is a mixture of enantiomers, and a compound wherein 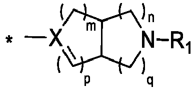 is enantiomerically rich or is enantiomerically pure. For example, the possible enantiomers of the groups S-9a, S-15a, S-18a and S-29a are shown as follows:

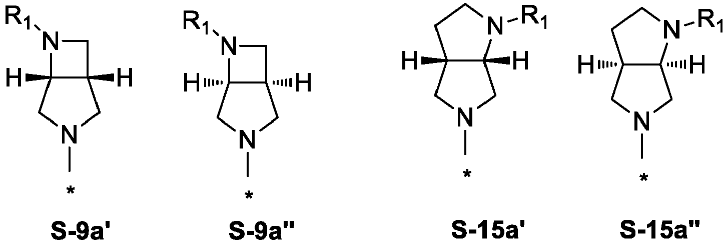

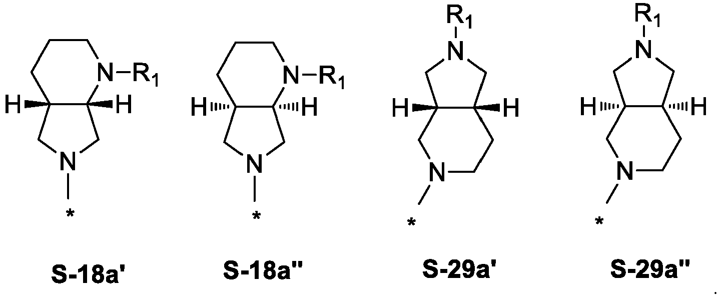

Among the fused ring compound represented by the formula (I), a stereoisomer or pharmaceutically acceptable salt thereof, the following compounds or pharmaceutically acceptable salts thereof are most preferred:

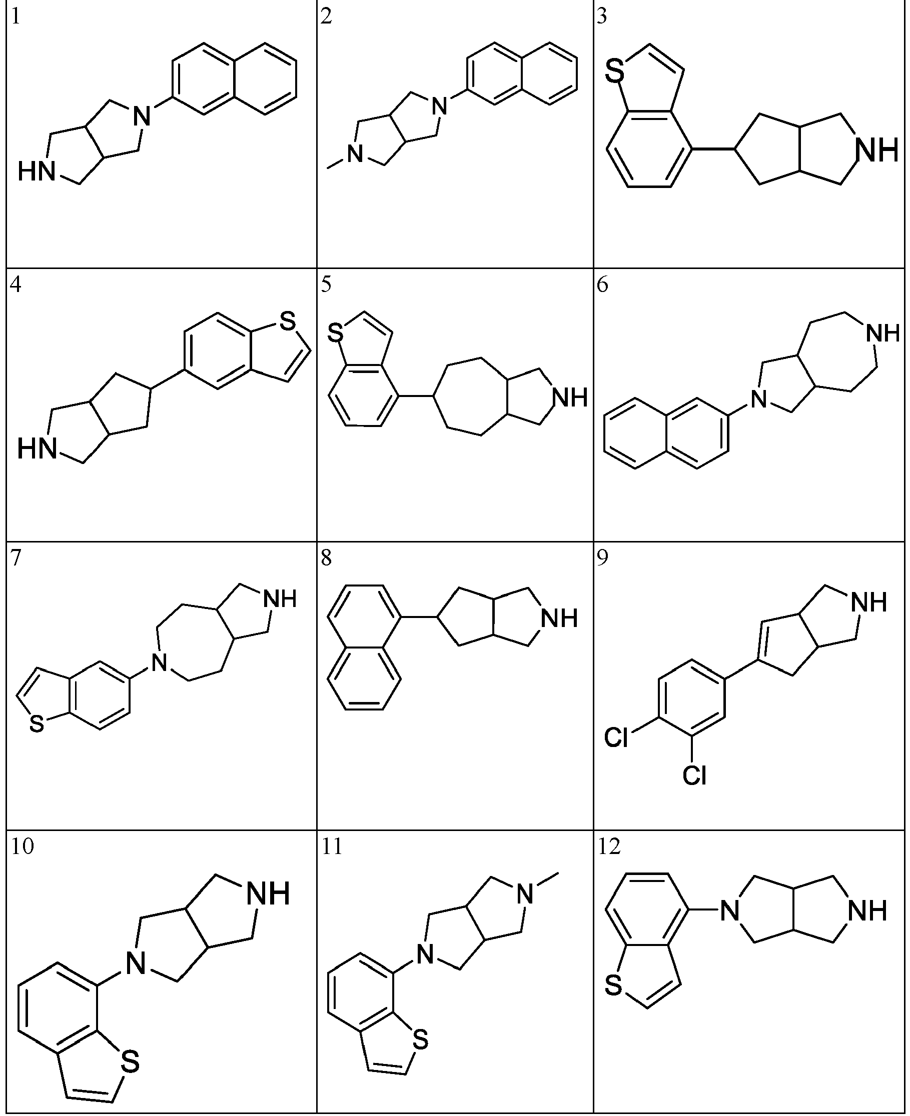

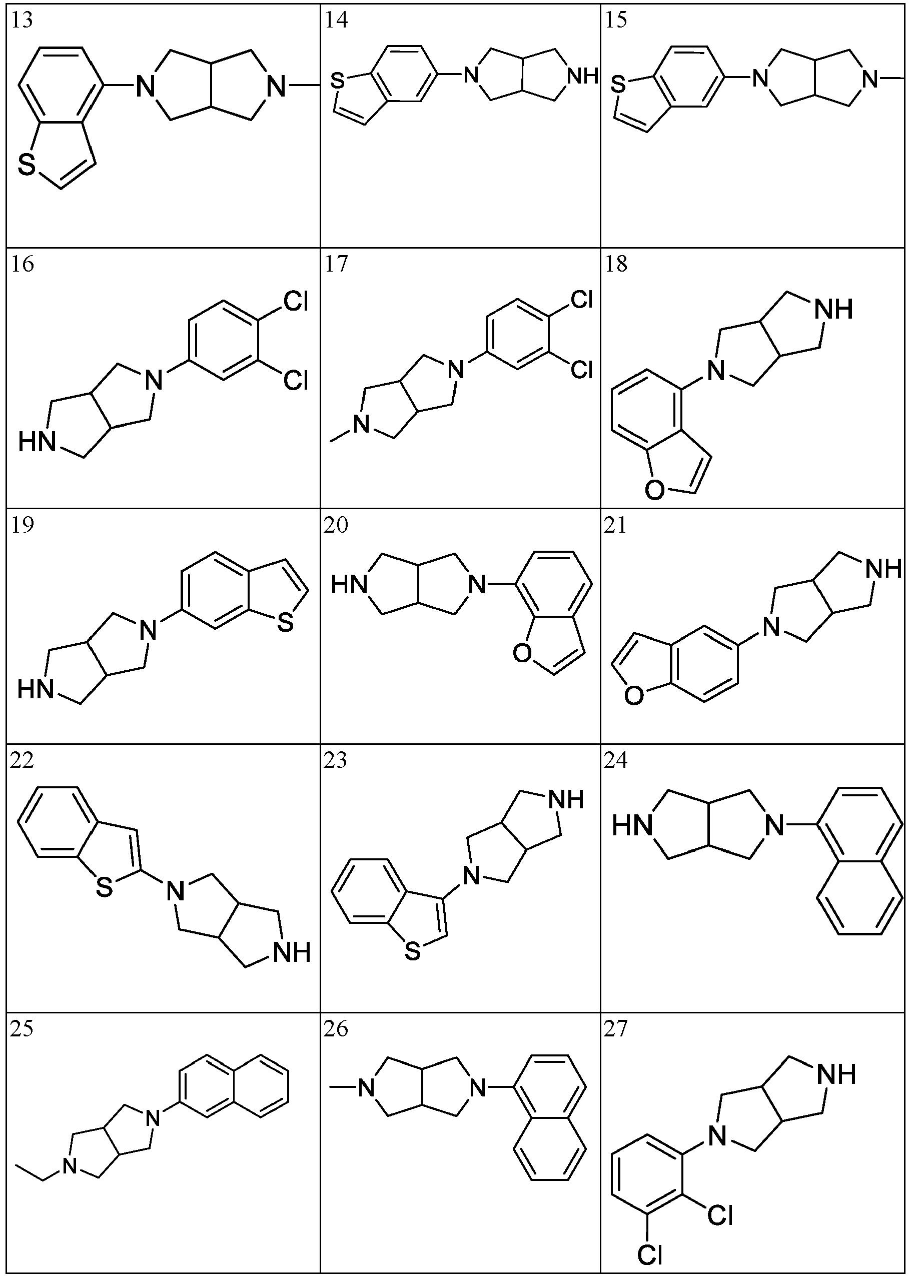

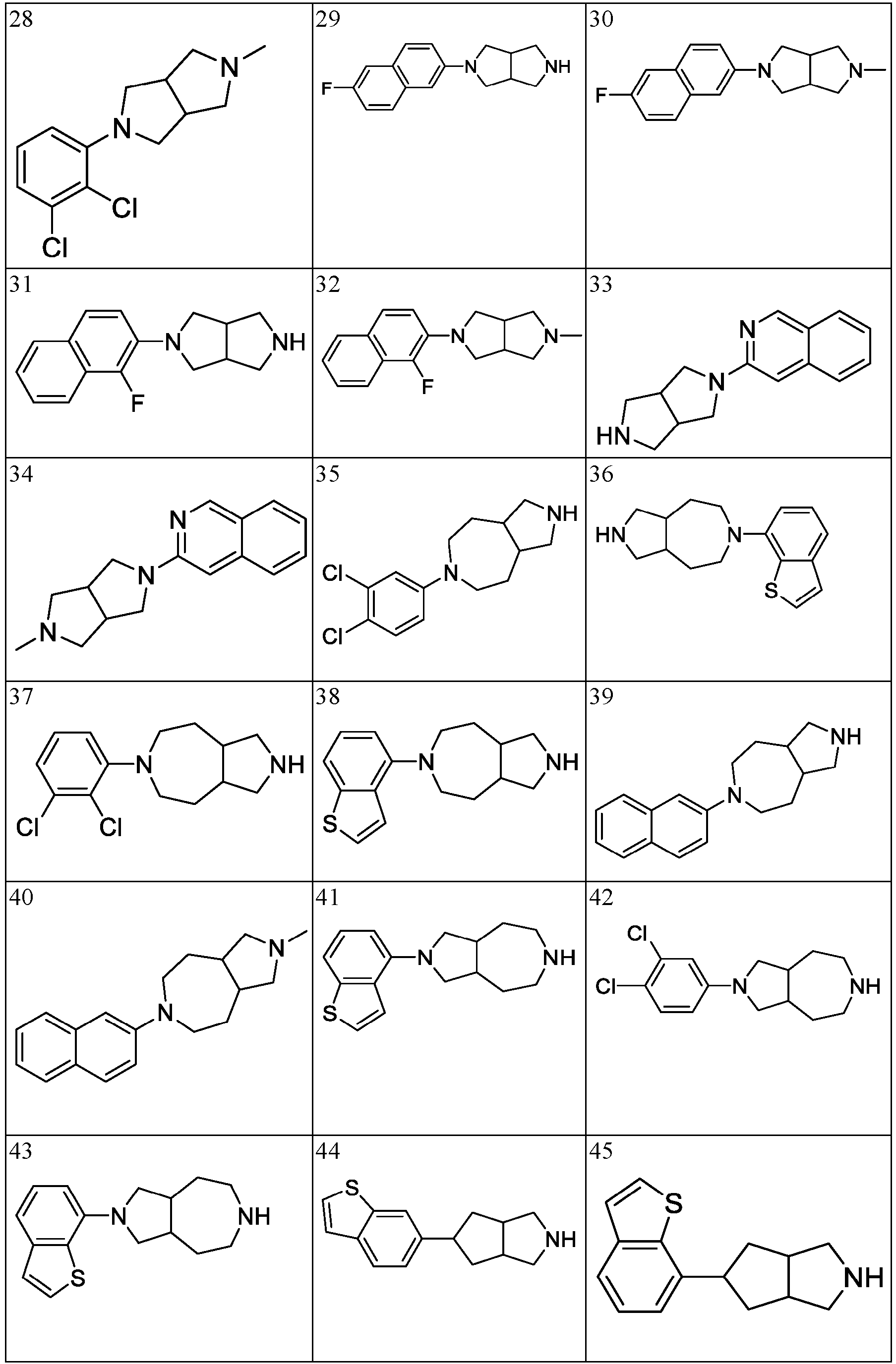

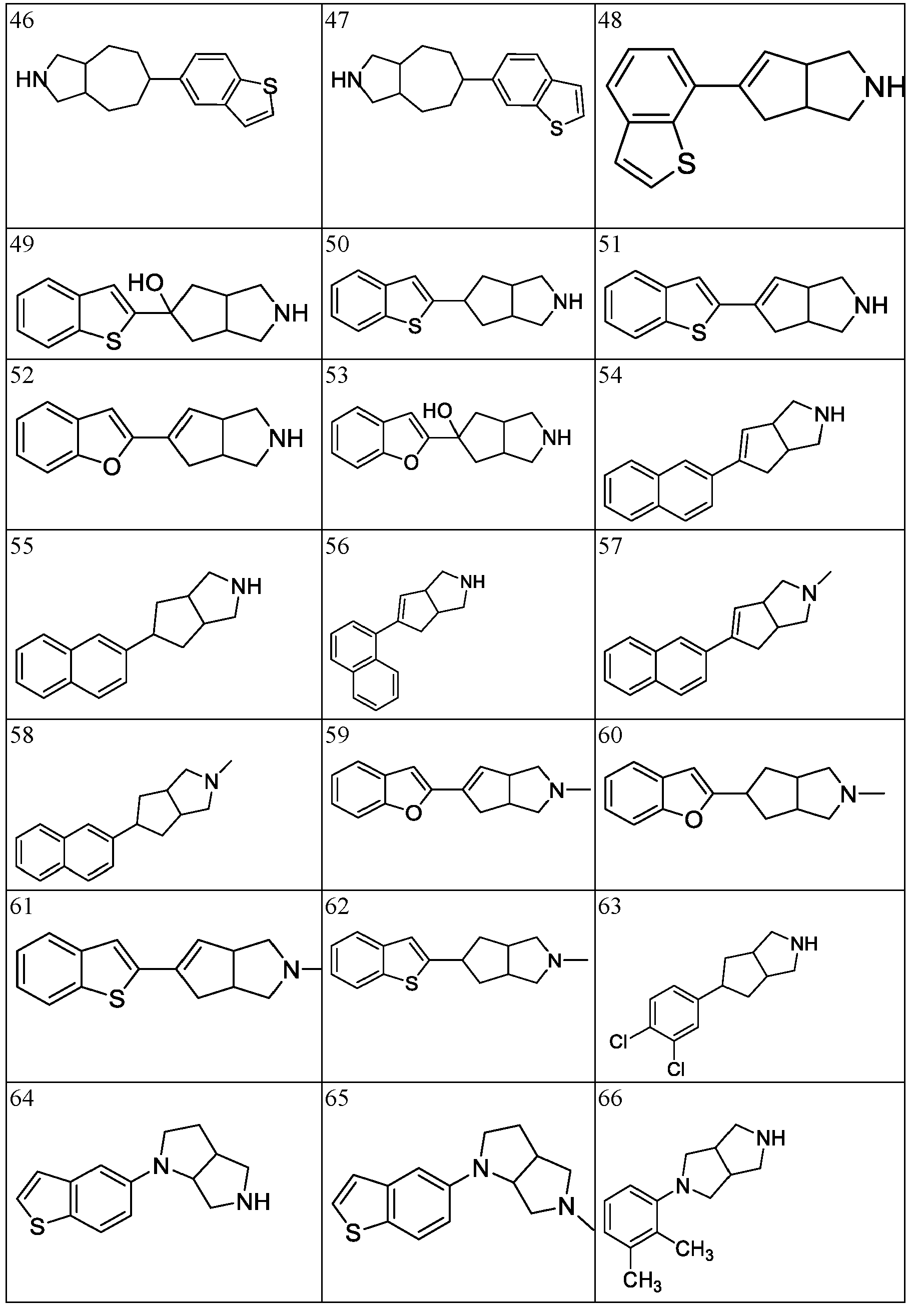

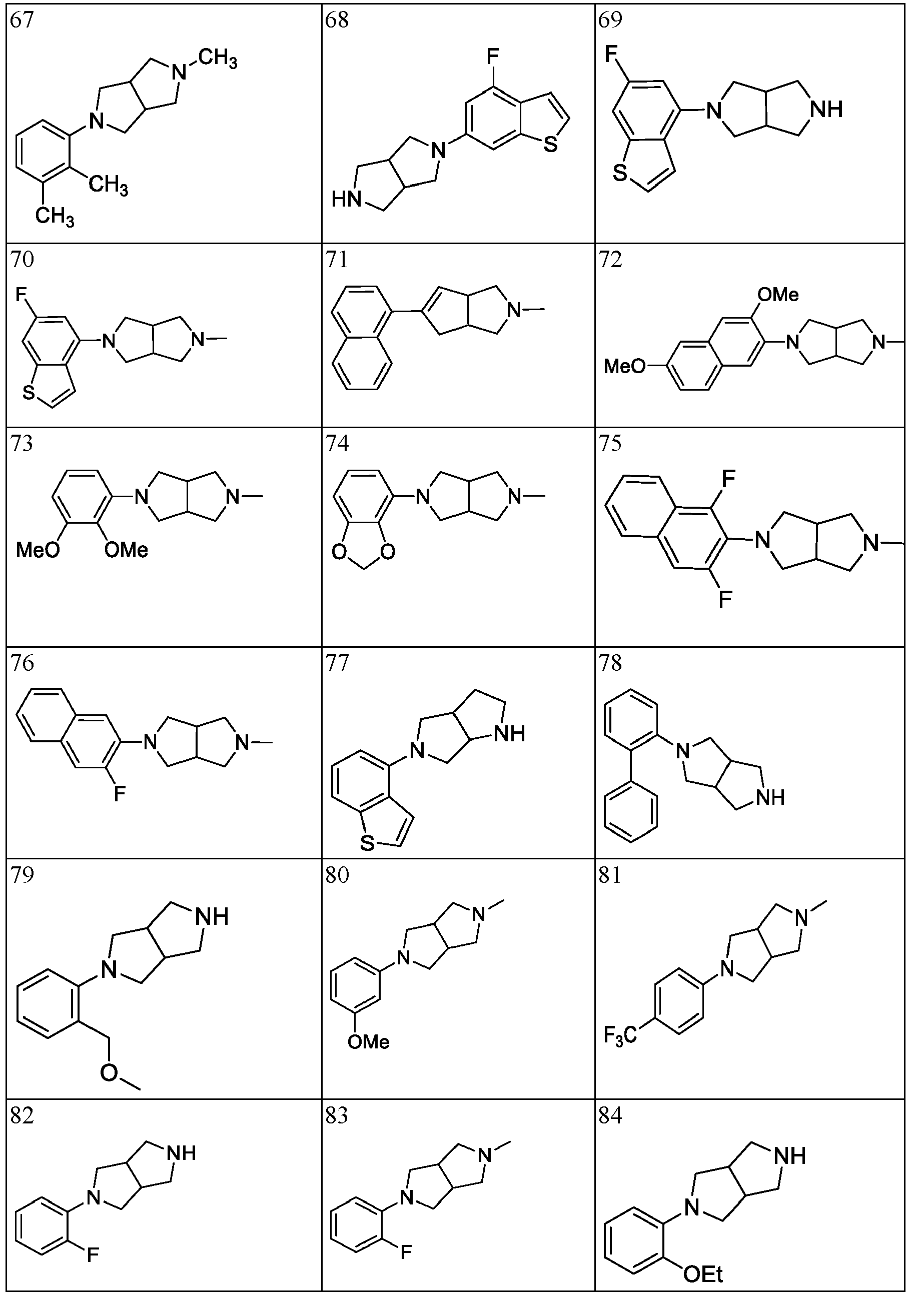

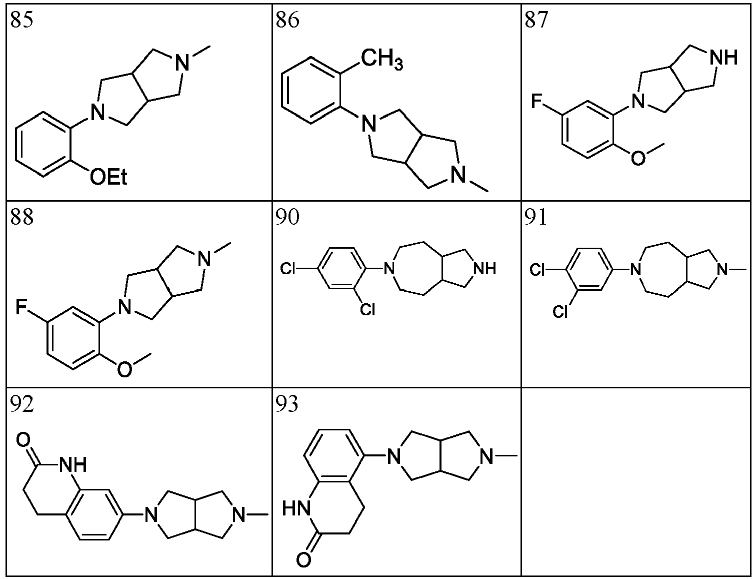

According to another embodiment of the present invention, provided is a method for preparing the fused ring compound represented by the formula (I), and the method is carried out by one of the following methods 1-7.

### Method 1:

A compound represented by formula (II-a) is coupled with a compound represented by formula (III) to obtain a compound represented by formula (Ia), as shown in Scheme 1: 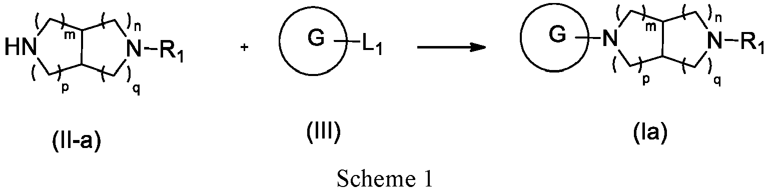 wherein, G ring, m, n, p, q, R₁ are defined the same as above; L₁ represents halogen, C1 to C6 alkylsulfonyloxy, benzenesulfonyloxy or naphthalenesulfonyloxy, the above C1 to C6 alkylsulfonyloxy, benzenesulfonyloxy and naphthalenesulfonyloxy may be optionally further substituted with one or more substituents selected from the group consisting of halogen, C1 to C6 alkyl, C1 to C6 alkoxy, nitro, hydroxy, amino and C1 to C6 alkanoyl; L₁ is preferably halogen, C1 to C4 alkylsulfonyloxy, benzenesulfonyloxy or naphthalenesulfonyloxy, the above C1 to C4 alkylsulfonyloxy, benzenesulfonyloxy and naphthalenesulfonyloxy may be optionally further substituted by one or more substituents selected from the group consisting of halogen, C1 to C4 alkyl, C1 to C4 alkoxy, nitro, hydroxyl, amino and C1 to C4 alkanoyl; L₁ is most preferably chlorine, bromine, methanesulfonyloxy, trifluoromethanesulfonyloxy, benzenesulfonyloxy, naphthalenesulfonyloxy, methylbenzenesulfonyloxy, nitrobenzenesulfonyloxy, aminobenzenesulfonyloxy, chlorobenzenesulfonyloxy, bromobenzenesulfonyloxy or methoxybenzenesulfonyloxy.

### Method 2:

A compound represented by formula (II-b) is coupled with a compound represented by formula (III) to obtain a compound represented by formula (IV), and then the amino protecting group is removed to obtain a compound of formula (Ib), which is optionally alkylated or reductive aminated to obtain a compound represented by formula (Ia), as shown in Scheme 2: 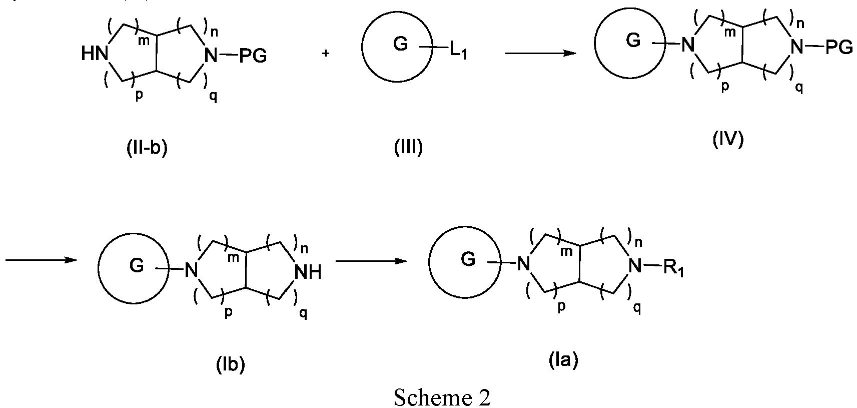 wherein, G ring, m, n, p, q are defined the same as above; R₁ is C1 to C20 alkyl, L₁ represents halogen, C1 to C6 alkylsulfonyloxy, benzenesulfonyloxy or naphthalenesulfonyloxy, the above C1 to C6 alkylsulfonyloxy, benzenesulfonyloxy and naphthalenesulfonyloxy may be optionally further substituted with one or more substituents selected from the group consisting of halogen, C1 to C6 alkyl, C1 to C6 alkoxy, nitro, hydroxy, amino and C1 to C6 alkanoyl; L₁ is preferably halogen, C1 to C4 alkylsulfonyloxy, benzenesulfonyloxy, or naphthalenesulfonyloxy, the above C1 to C4 alkylsulfonyloxy, benzenesulfonyloxy and naphthalenesulfonyloxy may be optionally further substituted by one or more substituents selected from the group consisting of halogen, C1 to C4 alkyl, C1 to C4 alkoxy, nitro, hydroxyl, amino and C1 to C4 alkanoyl; L₁ is most preferably chlorine, bromine, methanesulfonyloxy, trifluoromethanesulfonyloxy, benzenesulfonyloxy, naphthalenesulfonyloxy, methylbenzenesulfonyloxy, nitrobenzenesulfonyloxy, aminobenzenesulfonyloxy, chlorobenzenesulfonyloxy, bromobenzenesulfonyloxy or methoxybenzenesulfonyloxy;

PG is a substituted or unsubstituted benzyl group, acyl type amino protecting group or alkoxycarbonyl type amino protecting group, and the substituent on the benzyl group is one or more independently selected from the group consisting of halogen, trifluoromethyl, C1 to C6 alkyl, C1 to C6 alkoxy and nitro, preferably the acyl type amino protecting group is formyl, acetyl, propionyl, benzoyl, haloacetyl or phthaloyl, and the alkoxycarbonyl type amino protecting group is tert-butoxycarbonyl, benzyloxycarbonyl or 9-fluorenylmethyloxycarbonyl.

### Method 3:

A compound represented by formula (V) is coupled with a compound represented by formula (III) to obtain a compound of formula (VI), and then the amino protecting group is removed to obtain a compound of formula (Ic), which is optionally alkylated or reductive aminated to obtain a compound represented by formula (If), as shown in Scheme 3: 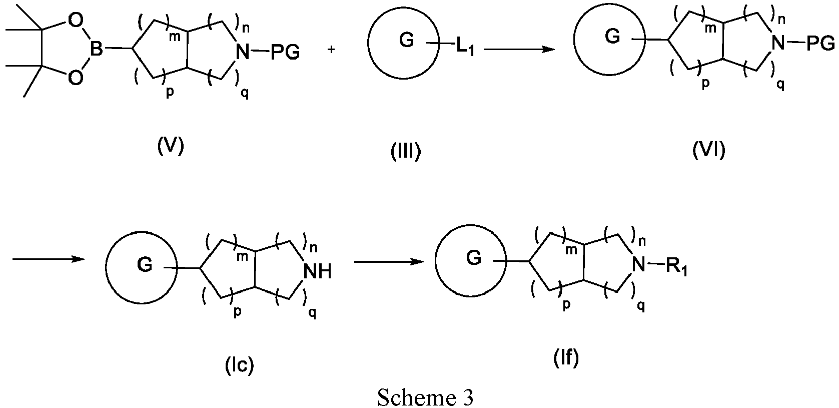 wherein, G ring, m, n, p, q are defined the same as above; R₁ is C1 to C20 alkyl, L₁ represents halogen, C1 to C6 alkylsulfonyloxy, benzenesulfonyloxy or naphthalenesulfonyloxy, the above C1 to C6 alkylsulfonyloxy, benzenesulfonyloxy and naphthalenesulfonyloxy may be optionally further substituted with one or more substituents selected from the group consisting of halogen, C1 to C6 alkyl, C1 to C6 alkoxy, nitro, hydroxy, amino and C1 to C6 alkanoyl; L₁ is preferably halogen, C1 to C4 alkylsulfonyloxy, benzenesulfonyloxy or naphthalenesulfonyloxy, the above C1 to C4 alkylsulfonyloxy, benzenesulfonyloxy and naphthalenesulfonyloxy may be optionally further substituted by one or more selected from the group consisting of halogen, C1 to C4 alkyl, C1 to C4 alkoxy, nitro, hydroxyl, amino and C1 to C4 alkanoyl; L₁ is most preferably chlorine, bromine, methanesulfonyloxy, trifluoromethanesulfonyloxy, benzenesulfonyloxy, naphthalenesulfonyloxy, methylbenzenesulfonyloxy, nitrobenzenesulfonyloxy, aminobenzenesulfonyloxy, chlorobenzenesulfonyloxy, bromobenzenesulfonyloxy or methoxybenzenesulfonyloxy;

PG is a substituted or unsubstituted benzyl group, acyl type amino protecting group or alkoxycarbonyl type amino protecting group, and the substituent on the benzyl group is one or more independently selected from the group consisting of halogen, trifluoromethyl, C1 to C6 alkyl, C1 to C6 alkoxy and nitro, preferably the acyl type amino protecting group is formyl, acetyl, propionyl, benzoyl, haloacetyl or phthaloyl, and the alkoxycarbonyl type amino protecting group is tert-butoxycarbonyl, benzyloxycarbonyl, or 9-fluorenylmethyloxycarbonyl.

In the above method 2 and method 3,

The reaction for removing the amino protecting group is conducted in the presence of an acid or an acidic silica gel.

The acid is one or more selected from the group consisting of hydrochloric acid, hydrogen chloride gas, sulfuric acid, phosphoric acid, nitric acid, acetic acid, hydrobromic acid, hydroiodic acid, perchloric acid, trichloroacetic acid and trifluoroacetic acid.

The reaction for removing the amino protecting group is conducted in the presence or absence of a solvent, and the solvent is one or more selected from the group consisting of water, dioxane, methanol, ethanol, n-propanol, isopropanol, tert-butanol, diethyl ether, N-methyl pyrrolidone, tetrahydrofuran, acetonitrile, dichloromethane, chloroform, N,N -dimethylformamide, toluene, ethyl acetate, propyl acetate and butyl acetate.

In particular, when PG is a substituted or unsubstituted benzyl group, the reaction for removing the amino protecting group is: 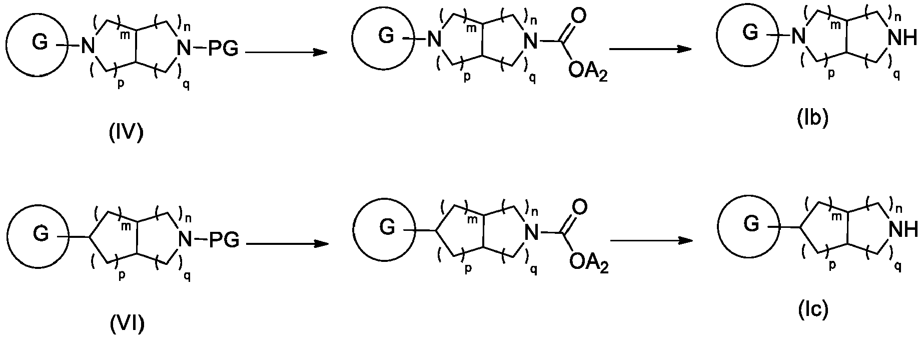
the compound (IV) or (VI) or its salt is dissolved in a solvent and reacted with the chloroformate A₂OOCCl to remove the benzyl group, and the resultant product is hydrolyzed in the solvent to remove the acyl group to obtain the formula (Ib) or (Ic),
wherein A₂ is a halogenated or unsubstituted C₁-C₆ alkyl, a substituted or unsubstituted phenyl, or a substituted or unsubstituted benzyl, and the substituent on the substituted phenyl or benzyl is one or more selected from the group consisting of fluorine, chlorine, bromine, iodine, nitro, C₁-C₄ alkyl and C₁-C₄ alkoxy; preferably, A₂ is C₁-C₄ alkyl, substituted or unsubstituted phenyl, or substituted or unsubstituted benzyl, the substituent on the substituted phenyl or benzyl is one or more selected from the group consisting of fluorine, chlorine, bromine, iodine and nitro; more preferably, A₂ is methyl, ethyl, phenyl or benzyl.

The temperature of the reaction for removing the amino protecting group is 0°C to 150°C, preferably 30°C to 100°C; the reaction time is 0.5 to 24h, preferably 1 to 12h.

### Method 4:

A compound represented by formula (II-a) is reacted with a compound represented by formula (X) through carbonyl addition to obtain a compound represented by formula (VIII), and then the amino protecting group is removed to obtain a compound of formula (Id), as shown in Scheme 4: 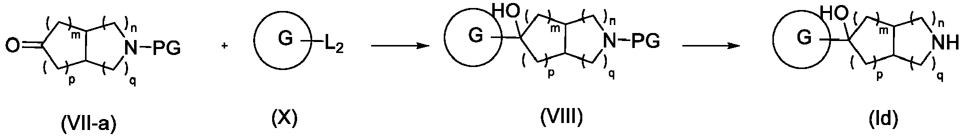 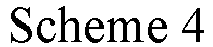 wherein, G ring, m, n, p, q are defined the same as above; L₂ represents Li, MgBr, MgCl, MgI, ZnBr, ZnCl or Znl;

PG is an acyl type amino protecting group or alkoxycarbonyl type amino protecting group, preferably the acyl type amino protecting group is formyl, acetyl, propionyl, benzoyl, haloacetyl or phthaloyl, and the alkoxycarbonyl type amino protecting group is tert-butoxycarbonyl, benzyloxycarbonyl or 9-fluorenylmethyloxycarbonyl.

The reaction for removing the amino protecting group is conducted in the presence of an acid or an acidic silica gel;

The acid is one or more selected from the group consisting of hydrochloric acid, hydrogen chloride gas, sulfuric acid, phosphoric acid, nitric acid, acetic acid, hydrobromic acid, hydroiodic acid, perchloric acid, trichloroacetic acid and trifluoroacetic acid.

The reaction for removing the amino protecting group is conducted in the presence or absence of a solvent, and the solvent is one or more selected from the group consisting of water, dioxane, methanol, ethanol, n-propanol, isopropanol, tert-butanol, diethyl ether, N-methyl pyrrolidone, tetrahydrofuran, acetonitrile, dichloromethane, chloroform, N,N -dimethylformamide, toluene, ethyl acetate, propyl acetate and butyl acetate.

The temperature of the reaction for removing the amino protecting group is 0°C to 150°C, preferably 30°C to 100°C; the reaction time is 0.5 to 24h, preferably 1 to 12h.

### Method 5:

A compound represented by formula (VII-a) is reacted with a compound represented by formula (X) through carbonyl addition to obtain a compound of formula (VIII), and then dehydration and amino deprotection are simultaneously performed to obtain a compound of formula (Ie), which is optionally alkylated or reductive aminated to obtain a compound represented by formula (Ig), as shown in Scheme 5: 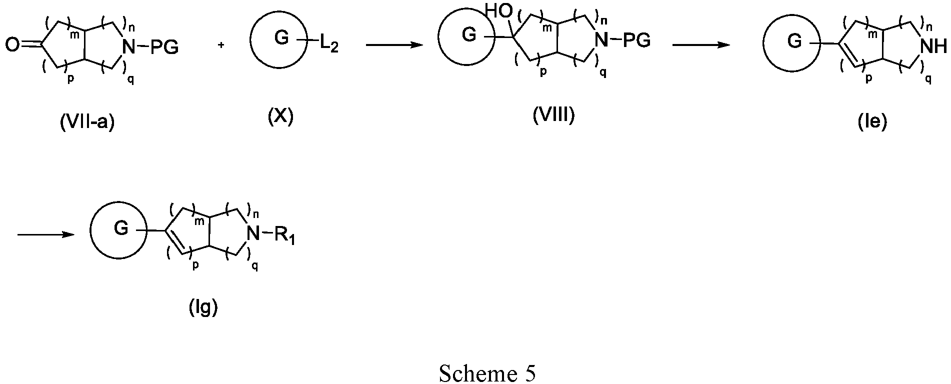
wherein, G ring, m, n, p, q are defined the same as above; R₁ is C₁-C₂₀ alkyl; L₂ represents Li, MgBr, MgCl, MgI, ZnBr, ZnCl or Znl;
PG is an acyl type amino protecting group or alkoxycarbonyl type amino protecting group, preferably the acyl type amino protecting group is formyl, acetyl, propionyl, benzoyl, haloacetyl or phthaloyl, and the alkoxycarbonyl type amino protecting group is tert-butoxycarbonyl, benzyloxycarbonyl or 9-fluorenylmethyloxycarbonyl.

The simultaneous dehydration and amino deprotection are conducted in the presence of an acid; preferably, the acid is one or more selected from the group consisting of hydrochloric acid, hydrogen chloride gas, sulfuric acid, phosphoric acid, nitric acid, acetic acid, hydrobromic acid, hydroiodic acid, perchloric acid, trichloroacetic acid and trifluoroacetic acid. The simultaneous dehydration and amino deprotection are conducted in the presence or absence of a solvent, and the solvent is one or more selected from the group consisting of water, dioxane, methanol, ethanol, n-propanol, isopropanol, tert-butanol, diethyl ether, N-methyl pyrrolidone, tetrahydrofuran, acetonitrile, dichloromethane, chloroform, N,N -dimethylformamide, toluene, ethyl acetate, propyl acetate and butyl acetate.

### Method 6:

A compound represented by formula (VII-b) is reacted a the compound represented by formula (X) through carbonyl addition to obtain a compound represented by formula (Ih), which is dehydrated to obtain a compound of formula (Ig), as shown in Scheme 6: 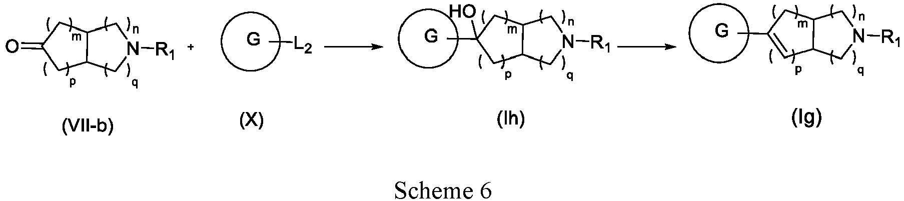 wherein, G ring, m, n, p, q are defined the same as above; R₁ is C1-C20 alkyl; L₂ represents Li, MgBr, MgCl, MgI, ZnBr, ZnCl or ZnI.

The dehydration is conducted in the presence of an acid; preferably, the acid is one or more selected from the group consisting of hydrochloric acid, hydrogen chloride gas, sulfuric acid, phosphoric acid, nitric acid, acetic acid, hydrobromic acid, hydroiodic acid, perchloric acid, trichloroacetic acid and trifluoroacetic acid. The dehydration reaction is conducted in the presence or absence of a solvent, and the solvent is one or more selected from the group consisting of water, dioxane, methanol, ethanol, n-propanol, isopropanol, tert-butanol, diethyl ether, N-methyl pyrrolidone, tetrahydrofuran, acetonitrile, dichloromethane, chloroform, N,N -dimethylformamide, toluene, ethyl acetate, propyl acetate and butyl acetate.

### Method 7:

A compound represented by formula (Ie) or (Ig) is subjected to hydrogenation reduction to obtain a compound represented by formula (Ic) or (If) respectively: 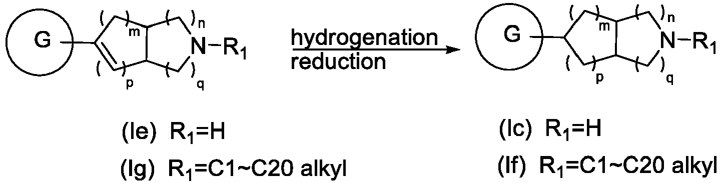 wherein, G ring, m, n, p, q are defined the same as above;

The hydrogenation reduction is carried out in the presence of a reducing agent, and the reducing agent includes, but is not limited to, hydrogen/palladium on carbon, hydrogen/Raney nickel, hydrogen/palladium hydroxide, and the like.

In the above methods 1 to 3, the coupling reaction is carried out in the presence of a palladium catalyst and an alkali.

The palladium catalyst is palladium acetate (Pd(OAc)₂), bis(triphenylphosphine) palladium dichloride ((Ph₃P)₂PdCl₂), bis(benzonitrile) palladium chloride ((PhCN)₂PdCl₂), tetrakis(triphenylphosphine) palladium (Pd(PPh₃)₄), bis(triphenylphosphine) palladium acetate ((Ph₃P)₂Pd(OAc)₂), 1,2-bis(diphenylphosphinyl)ethane palladium dichloride((PdCl₂(dppe)₂)), bis(1,2-bis(diphenylphosphine)ethane) palladium (Pd(dppe)₂), bis(dibenzylideneacetone) palladium (Pd(dba)₂), tri(dibenzylideneacetone) dipalladium (Pd₂(dba)₃), [1,3-bis(diphenylphosphino)propane] palladium dichloride (PdCl₂(dippp)) and [1,1'-bis(diphenylphosphine)ferrocene] palladium dichloride (Pd(dppf)Cl₂).

The base is one or more selected from the group consisting of sodium bis(trimethylsilyl)amide, potassium tert-butoxide, sodium tert-butoxide, cesium carbonate, potassium phosphate, sodium phosphate, sodium methoxide, sodium ethoxide, potassium hydroxide, sodium hydroxide, potassium fluoride, sodium fluoride, tetrabutylammonium fluoride (TBAF) , sodium acetate, potassium acetate, cesium carbonate, potassium carbonate and sodium carbonate.

Preferably, the solvent used in the reaction includes water, dioxane, tetrahydrofuran, toluene, xylene, tert-butanol, acetone, N,N-dimethylformamide, dimethylsulfoxide, acetonitrile, or a mixture of the above solvents.

Preferably, a ligand as a reaction accelerator is added optionally in the reaction, and the ligand is 2,2'-diphenylphosphino-1,1'-binaphthyl(BINAP), tri-tert-butylphosphine(P(t-Bu)₃), 1,1'-bis-(diphenylphosphino)ferrocene (dppf), 2-dicyclohexylphosphorus-2,4,6-triisopropylbiphenyl(x-phos), 4,5-bisdiphenylphosphine-9,9-dimethylxanthene (Xantphos), tri-tert-butylphosphine tetrafluoroborate or tris(2-methylphenyl)phosphine (P(o-tolyl)₃).

In the above methods 4 to 6, the solvent for the carbonyl addition is one or more selected from the group consisting of tetrahydrofuran, diethyl ether, n-hexane, n-pentane, dioxane and toluene, and the reaction temperature of the carbonyl addition is -80°C to 100°C, preferably -80°C to 30°C.

In the above methods 2, 3 and 5:
The alkylation is carried out in the presence of an alkylating reagent, and the alkylating reagent includes, but is not limited to, methyl iodide, ethyl trifluoromethanesulfonate, ethyl iodide, ethyl bromide, and the like.

The reductive amination is carried out in the presence of a corresponding aldehyde/ketone and a reducing agent, and the reducing agent includes, but are not limited to, sodium borohydride, potassium borohydride, sodium triacetoxyborohydride (NaBH(OAc)₃), ammonium tetramethyltriacetoxyborohydride and sodium cyanoborohydride, etc.

The compounds of formula (II-a), formula (II-b), formula (III), formula (V), formula (X), formula (VII-a), and formula (VII-b) are commercially available compounds, or can be prepared according to methods known in the art or according to synthetic methods of similar compounds.

The stereoisomers of the compound of formula (I) include any of its enantiomers, diastereomers, tautomers, racemic mixtures, enantiomerically enriched mixtures and enantiomerically pure forms. The racemic form may be separated into optical enantiomers by known methods. For example, diastereomeric salts may be separated using an optically active acid and then treated with a base to release an optically active amine compound. Another method for resolving a racemate into optical enantiomers is based on chromatography on an optically active substrate. The racemic compounds of the present invention may also be resolved into their optical enantiomers by fractional crystallization of d- or 1-(tartaric acid, mandelic acid or camphorsulfonic acid) salt. The compounds of the present invention may also be resolved by forming diastereomeric derivatives.

The starting compound used in each of the above schemes may be a suitable salt, and the suitable salt includes alkali metal salts and alkaline earth metal salts, such as sodium salt, potassium salt, calcium salt, magnesium salt, etc.; organic alkali salts such as pyridine salt, triethylamine salt, etc.; inorganic acid salts such as hydrochloride, hydrobromide, hydroiodide, sulfate, nitrate, phosphate, etc.; organic acid salts such as formate, acetate, propionate, glycolate, oxalate, malonate, succinate, fumarate, maleate, lactate, malate, citrate, tartrate, picrate, glutamate, methanesulfonate and benzenesulfonate, etc.

In addition, the starting compound used in each of the above schemes may include its solvates, such as hydrates, alcoholates, and the like.

The fused ring compound represented by the formula (I) of the present invention and its stereoisomers also include their solvate forms, such as hydrates, alcoholates, etc., and the solvates are included in the scope of the present invention.

The pharmaceutically acceptable salt of the fused ring compound represented by the formula (I) and its stereoisomers of the present invention means that the fused ring compound represented by the formula (I) or its stereoisomer is treated with an appropriate acid, so as to convert them into therapeutically active non-toxic salt forms. The salt may be, for example, hydrochloride, hydrobromide, hydroiodide, sulfate or hydrogen sulfate, nitrate, phosphate or acidic phosphate, perchlorate, formate, acetate, trifluoroacetate, propionate, pyruvate, glycolate, oxalate, malonate, succinate, glutarate, maleate, fumarate, lactate, malate, citrate, tartrate, picrate, glutamate, benzoate, methanesulfonate, ethanesulfonate, benzenesulfonate, p-toluenesulfonate, salicylate, ascorbate, camphorate or camphor sulfonate, etc. Alternatively, alkali treatment may also be used to convert the salt form to the free base form.

As used above, the term "pharmaceutically acceptable salts" also includes solvates thereof, and said solvates are included within the scope of the present invention. Examples of solvates include, for example, hydrates, alcoholates, and the like.

Each target compound obtained by each scheme may be separated and purified from the reaction mixture by the following methods, for example: after the reaction mixture is cooled, the crude product is separated by methods such as filtration, extraction or concentration, and then purified by a conventional method, such as column chromatography, slurrying or recrystallization.

The present invention also provide a pharmaceutical composition, comprising a therapeutically effective amount of one or more selected from the group consisting of the above fused ring compound represented by the formula (I), the stereoisomers thereof and the pharmaceutically acceptable salts thereof, and optionally a pharmaceutically acceptable carrier. The pharmaceutical composition may be used to treat or prevent central nervous system diseases.

The present invention also provide a method for preparing the pharmaceutical composition, comprising mixing the fused ring compound represented by the above formula (I), a stereoisomer thereof or a pharmaceutically acceptable salt thereof with a pharmaceutically acceptable carrier. For example, a tablet may be prepared by mixing an active ingredient with a common adjuvant and/or diluent, and then compressing the mixture in a conventional tablet press.

In the pharmaceutical composition of the present invention, a variety of pharmaceutical preparation forms may be selected according to the purpose of treatment, and in general, they may include tablets, pills, capsules, granules, suspensions, solutions, creams, ointments, powders, suppositories, gas sprays and injections, etc. Conveniently, the compound of the present invention is administered in a unit dosage form, which contains the compound in an amount of about 0.01 to 100 mg. The total daily dose is usually about 0.05 to 500 mg, most preferably about 0.1 to 50 mg of the active compound of the present invention.

The present invention also provide the use of the above fused ring compound represented by the formula (I), a stereoisomer thereof or a pharmaceutically acceptable salt thereof, or the above-mentioned pharmaceutical composition in preparation of a drug for prevention and/or treatment of a central nervous system disease.

The present invention also provides a method for treating and/or preventing a central nervous system disorder, comprising administering to a human or animal with the fused ring compound represented by the formula (I), a stereoisomer thereof or a pharmaceutically acceptable salt thereof, or the above-mentioned pharmaceutical composition of the present invention.

The above-mentioned central nervous system disease is selected from the group consisting of affective disorder; mental disorder; mood disorder; depression; intrinsic depression; major depression; uncontrollable depression; dysthymic disorder; cyclic affective disorder; panic attack; panic disorder; social phobia; obsessive-compulsive and behavioral disorders; impulsive disorders; post-traumatic stress disorders; anxiety disorders; acute stress disorders; hysteria; anorexia nervosa; sleep disorders; adaptive disorders; autism; neuropathic headache; mania; hyperactivity; fibromyalgia; neuropathic pain; attention deficit/hyperactivity diseases and tics, etc. Preferably, the sleep disorder is sleep apnea, insomnia, narcolepsy, or cataplexy. Preferably, the central nervous system disease is selected from the group consisting of depression; anxiety; and obsessive-compulsive and behavioral disorders.

The neuropathic pain includes, but is not limited to, postherpetic neuralgia, reflex sympathetic dystrophy/causalgia or nerve trauma, prosthetic pain, and peripheral neuropathy. Preferably, the herpes is herpes zoster; the peripheral neuropathy is diabetic neuropathy or neuropathy caused by long-term drinking of alcohol.

The various groups in formula (I) are defined as follows.

The term halogen generally refers to fluorine, chlorine, bromine and iodine; preferably fluorine, chlorine or bromine; more preferably fluorine or chlorine.

The C1 to C20 alkyl refers to a linear or branched saturated hydrocarbonyl containing 1 to 20 carbon atoms, for example, methyl, ethyl, n-propyl, isopropyl, n-butyl, isobutyl, tert-butyl, sec-butyl, n-pentyl, 1-ethylpropyl, isopentyl, neopentyl, isohexyl, 3-methylpentyl or n-hexyl, etc., preferably methyl, ethyl, n-propyl, isopropyl, butyl, isobutyl or tert-butyl; and the C1 to C10 alkyl and C1 to C6 alkyl are defined similarily.

The C1 to C20 alkoxy refers to a linear or branched alkoxy containing 1 to 20 carbon atoms, for example, methoxy, ethoxy, n-propoxy, isopropoxy, n-butoxy, isobutoxy, tert-butoxy, sec-butoxy, n-pentoxy, isopentoxy, neopentoxy, isohexoxy, 3-methyl pentoxy or n-hexoxy, preferably methoxy, ethoxy, n-propoxy, isopropoxy, n-butoxy, isobutoxy or tert-butoxy, and the C1 to C10 alkoxyand C1 to C6 alkoxy are defined similarily.

The compound of the present invention has the following beneficial effects.
1) The compound of the present invention has high affinity to 5-HT₃ receptor, and are thus capable of treating a central nervous system disease associated with or affected by 5-HT₃ receptor.
2) The compound of the present invention has high affinity to 5-HT transporter, and are thus capable of treating a central nervous system disease associated with or affected by 5-HT transporter.
3) The compound of the present invention is characterized by multi-targeting action, can act on monoamine transporter/5-HT receptors, etc. at the same time, i.e., it has high activity on at least two targets selected from the group consisting of 5-HT transporter, dopamine transporter, norepinephrine transporter, 5-HT₃ receptor, etc. It can be seen from the results of pharmacological experiments that most of the compounds have simultaneous 5-HT transporter/5-HT₃ receptor effects. Such multi-targeting action property is beneficial to balance neurotransmitters in the brain, and has a better curative effect on a central nervous system disease.
4) Since the compound of the present invention has the multi-targeting action property, it can act quickly through the synergistic effect of multi-targeting action and reduce the side effects caused by the drug.
5) The compound of the present invention not only has high activity, but also is effective after oral administration and characterized by low effective dose and low toxic and side effects. It has curative effect on the central nervous system diseases, especially has a good effect for major depression disorder (MDD), anxiety, obsessive-compulsive disorder, etc.

In conclusion, compared with existing antidepressant drugs, the compound of the present invention has the advantages of multi-targeting action, lower effective dose, fewer toxic and side effects, better safety and tolerability, etc., and has good prosepect for clinical applications.

### Best mode for carrying out the invention

The present invention is further illustrated by the following preparation examples, examples and pharmacological activity test examples, but the scope of the present invention is not limited thereto.

### Example 1: 2-(naphthalen-2-yl)octahydropyrrolo[3,4-c]pyrrole

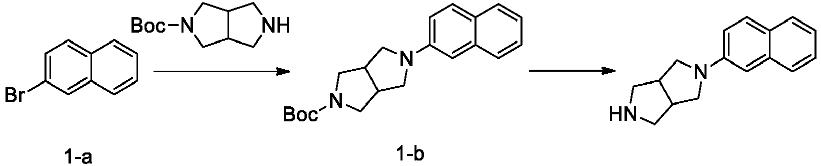

### Step 1:

2-Bromonaphthalene (1-a, 10.7 g, 52 mmol, 1.1 eq) and tert-butyl hexahydropyrrolo[3,4-c]pyrrole-2(1H)-carboxylate (10 g, 47 mmol, 1 eq) were added to 150 mL of toluene. The toluene solution was added with Pd₂(dba)₃ (2.16 g, 2.36 mmol, 0.05 eq), BINAP (2.35 g, 3.78 mmol, 0.08 eq) and potassium tert-butoxide (7.9 g, 70.8 mmol, 1.5 eq), and heated under N₂ protection at an external temperature of 105 °C for 10h. The reaction solution was diluted with 150 ml of ethyl acetate and filtered, and the filter cake was washed with a small amount of ethyl acetate. The filtrate was washed with 300 ml of a saturated sodium chloride solution. The organic phase was dried over anhydrous sodium sulfate and rotary evaporated to dryness to obtain 16g of a brown oil. The oil was slurried in 100 ml of ethanol to precipitate out a large amount of solids, stirred for 2 h at room temperature to disperse the solids evenly, and filtered. The filter cake was heated at 50 °C for 3 h to obtain 1-b as a gray solid (15.2 g, yield 95.3%).

### Step 2:

**1-b** (15.2g) was added to 100 ml of methanol, added with 80 ml of a concentrated hydrochloric acid, and stirred at room temperature for 2h. The reaction solution was diluted with 500 ml of water, and extracted with 400 ml of methyl tert-butyl ether to remove less polar impurities. The aqueous phase was adjusted pH with 200 ml of a 5M NaOH solution to 9 and extracted with 600 ml of dichloromethane. The organic phase was washed with 300 ml of a saturated sodium chloride solution, dried with anhydrous sodium sulfate and rotary evaporated to dryness to obtain 10.8 g of a yellow solid. ESI-MS (m/z): 239.17 [M+H]⁺. HPLC>95%.

### Example 2: 2-methyl-5-(naphthalen-2-yl)octahydropyrrolo[3,4-c]pyrrole

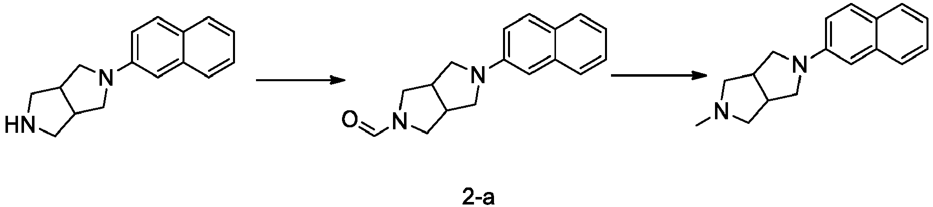

### Step 1:

The product of Example 1 (7.5 g, 31.6 mmol) was added to 120 ml of ethyl formate, and added with triethylamine (17.6 ml, 126.6 mmol, 4 eq). The system was heated at an external temperature of 60 °C for 3h, and diluted with 100 ml of ethyl acetate. The organic phase was washed with 200 ml of a saturated ammonium chloride solution and then with 200 ml of a saturated sodium chloride solution, dried over anhydrous sodium sulfate and rotary evaporated to dryness to obtain **2-a** as a yellowish solid (8.4g, yield 99.8%).

### Step 2:

**2-a** (8.4 g, 31.6 mmol) was added to 120 ml of dry tetrahydrofuran, and added with a 1M borane tetrahydrofuran solution (63.2 ml, 63.2 mmol, 2 eq). The reaction solution was gradually cleared, and kept under N₂ protection at an outer temperature of 68 °C for 2 h. The reaction solution was put in an ice bath, added dropwise with 7.9 ml of hydrochloric acid (3eq) dissolved in 100 ml of methanol to produce a large number of bubbles, heated at an external temperature of 68°C for 10h, and rotary evaporated to dryness. The residue was added with 80 ml of water. The aqueous phase was adjusted pH with 20 ml of a 5M NaOH solution to 9, and extracted with 200 ml of DCM. The organic phase was washed with 200 ml of a saturated sodium chloride solution, dried over anhydrous sodium sulfate and rotary evaporated to dryness to obtain 8g of a solid. ESI-MS (m/z): 253.35 [M+H]⁺. HPLC>95%.

### Example 3: 5-(Benzo[b]thiophen-4-yl)octahydrocyclopenta[c]pyrrole hydrochloride

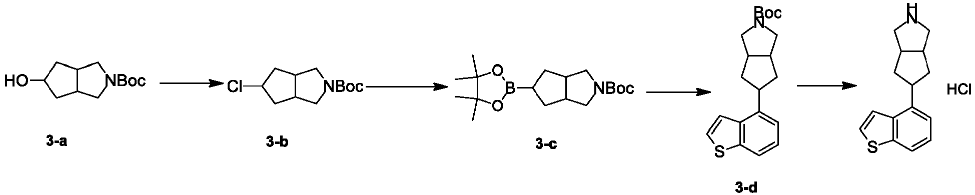

### Step 1:

Thionyl chloride (1.2 g, 10 mmol) was added dropwise to a solution of **3-a** (2.27 g, 10 mmol) and triethylamine (1.01 g, 10 mmol) in dichloromethane (30 mL) at 0°C. After the addition, the mixture was raised to 20°C to react for 5h, added with water to quench the reaction, and layered. The organic layer was concentrated and subjected to column chromatography (EA:PE=1:15 to 1:5) to obtain **3-b** (1.25 g, yield 60%).

### Step 2:

Potassium *tert*-butoxide (1.12 g, 10 mmol) was added to a solution of bis(pinacolato)diboron (2.54 g, 10 mmol), cuprous chloride (25 mg, 0.25 mmol) and the ligand xantphos (145 mg, 0.25 mmol) in THF (30 mL) at 0°C under nitrogen protection. After the addition, the mixture was raised to 20°C and reacted for 30min, added dropwise with a solution of **3-b** (1.23 g, 5 mmol) in THF (2 mL), and reacted at room temperature overnight. The reaction solution was directly mixed with silica gel and subjected to column chromatography (EA:PE=1:15 to 1:5) to obtain **3-c** (800 mg, yield 48%).

### Step 3:

A solution of **3-c** (340 mg, 1 mmol), 4-bromobenzothiophene (213 mg, 1 mmol), cesium carbonate (326 mg, 1 mmol) and dppfPdCl₂ (73.2 mg, 0.1 mmol) in dioxane (10 mL) was reacted at 80°C under nitrogen protection overnight. The reaction solution was directly mixed with silica gel and subjected to column chromatography (EA:PE=1:15 to 1:5) to obtain **3-d** (40 mg, yield 12%).

### Step 4:

**3-d** (66 mg, 0.2 mmol) was dissolved in methanol (1 mL), added with a 4N HCl methanol solution (1 mL), and reacted at room temperature for 1h. The reaction solution was concentrated to dryness, and slurried in methyl tert-butyl ether to obtain the title compound as an off-white solid (40 mg). ¹H NMR (400 MHz, CD₃OD) δ 7.88 (d, 1H), 7.69 (d, 1H), 7.54 (d, 1H), 7.46 (d, 1H), 7.23 (t, 1H), 3.28 (m, 2H), 3.20 (m, 2H), 3.08 (m, 1H), 2.90 (m, 2H), 2.39 (m, 2H), 1.37 (m, 2H). ESI-MS (m/z): 244.20 [M+H]⁺.

### Example 4: 5-(Benzothiophen-5-yl)octahydrocyclopenta[c]pyrrole hydrochloride

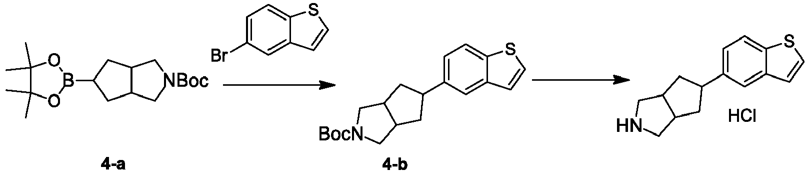

### Step 1:

**4-a** (340 mg, 1 mmol), 5-bromobenzothiophene (213 mg, 1 mmol), cesium carbonate (326 mg, 1 mmol) and dppfPdCl₂ (73.2 mg, 0.1 mmol) were added in dioxane (10 mL), and reacted at 80°C under nitrogen protection overnight. The reaction solution was directly mixed with silica gel and subjected to column chromatography (EA:PE=1:15 to 1:5) to obtain **4-b** (43 mg, yield 14%).

### Step 2:

**4-b** (66 mg, 0.2 mmol) was dissolved in methanol (1 mL), added with a 4N HCl methanol solution (1 mL), and reacted at room temperature for 1 h. The reaction solution was concentrated to dryness, and slurried in methyl tert-butyl ether to obtain the title compound as an off-white solid (38 mg). ¹H NMR (400 MHz, CD₃OD) δ 8.00 (d, 1H), 7.81 (d, 1H), 7.62 (d, 1H), 7.43 (dd, 1H), 7.33 (d, 1H), 3.28 (m, 2H), 3.20 (m, 2H), 3.09 (m, 1H), 2.90 (m, 2H), 2.40 (m, 2H), 1.37 (m, 2H). ESI-MS (m/z): 244.20 [M+H]⁺.

### Example 5: 6-(Benzo[b]thiophen-4-yl)decahydrocyclohepta[c]pyrrole hydrochloride

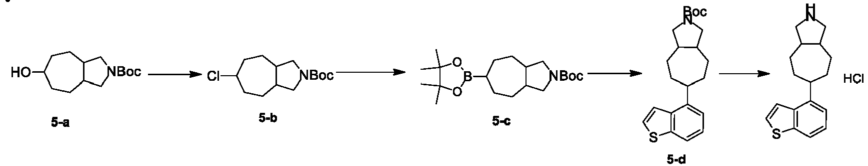

### Step 1:

Thionyl chloride (1.2 g, 10 mmol) was added dropwise to a solution of **5-a** (2.55 g, 10 mmol) and triethylamine (1.01 g, 10 mmol) in dichloromethane (30 mL) at 0°C. After the addition, the reaction mixture was raised to 20°C to react for 5h, added with water to quench the reaction, and layered. The organic layer was concentrated and subjected to column chromatography (EA:PE=1:15 to 1:5) to obtain **5-b** (1.5 g, yield 55%).

### Step 2:

Potassium *tert*-butoxide (1.12 g, 10 mmol) was added to a solution of bis(pinacolato)diboron (2.54 g, 10 mmol), cuprous chloride (25 mg, 0.25 mmol) and the ligand xantphos (145 mg, 0.25 mmol) in THF (30 mL) at 0°C under nitrogen protection. After the addition, the reaction solution was raised to 20°C and reacted for 30min, added dropwise with a solution of **5-b** (1.37 g, 5 mmol) in THF (2 mL), and reacted at room temperature overnight. The reaction solution was directly mixed with silica gel and subjected to column chromatography (EA:PE=1:15 to 1:5) to obtain **5-c** (900 mg, yield 50%).

### Step 3:

A solution of **5-c** (360 mg, 1 mmol), 4-bromobenzothiophene (213 mg, 1 mmol), cesium carbonate (326 mg, 1 mmol) and dppfPdCl₂ (73.2 mg, 0.1 mmol) in dioxane (10 mL) was reacted at 80°C under nitrogen protection overnight. The reaction solution was directly mixed with silica gel and subjected to column chromatography (EA:PE=1:15 to 1:5) to obtain **5-d** (45 mg, yield 13%).

### Step 4:

**5-d** (74mg, 0.2 mmol) was dissolved in methanol (1 mL), added with a 4N HCl methanol solution (1 mL), and reacted at room temperature for 1 h. The reaction solution was concentrated to dryness, and slurried in methyl tert-butyl ether to obtain the title compound as an off-white solid (48 mg). ¹H NMR (400 MHz, CD₃OD) δ 7.90 (d, 1H), 7.70 (d, 1H), 7.56 (d, 1H), 7.47 (d, 1H), 7.24 (t, 1H), 3.52 (m, 3H), 2.87 (dd, 2H), 2.53 (m, 2H), 1.89 (m, 6H), 1.56 (m, 2H). ESI-MS (m/z): 272.28 [M+H]⁺.

### Example 6: 2-(naphthalen-2-yl)decahydropyrrolo[3,4-d]azepine maleate

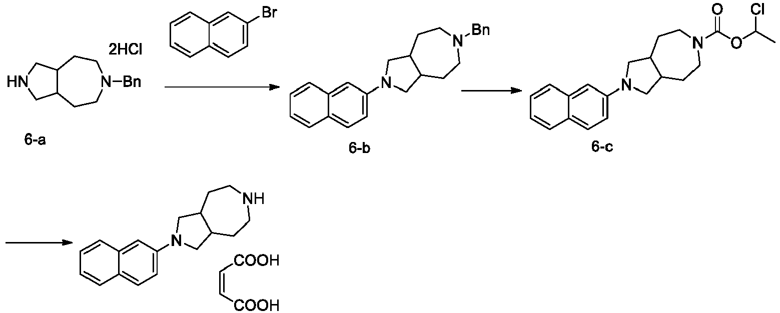

### Step 1:

**6-a** (200 mg, 0.66 mmol, 1 eq), 2-bromonaphthalene (137 mg, 0.66 mmol, 1 eq), Pd₂(dba)₃ (30 mg, 0.033 mmol, 0.05 eq), BINAP (33 mg, 0.053 mmol, 0.08 eq), potassium tert-butoxide (222 mg, 1.98 mmol, 3 eq) were added to 5 ml of dioxane under nitrogen protection, and heated at an external temperature of 105 °C for 8h. The resultant was filtered, and the filter cake was washed with ethyl acetate. The filtrate was washed with water and then with saturated sodium chloride, dried, and rotary evaporated to dryness. The residue was subjected to silica gel column chromatography to obtain crude **6-b** as an oil (153 mg).

### Step 2 and step 3:

**6-b** (153 mg, 0.43 mmol, 1 eq) was dissolved in chloroform, added with ethyl 1-chloroethylchloroformate (93 µl, 0.86 mmol, 2 eq), and heated at an external temperature of 70 °C for 4h. The reaction solution was rotary evaporated to dryness, added with methanol, and heated to reflux at 70 °C for 1h. The resultant was added with water and then with 1 ml of concentrated hydrochloric acid. Methyl *tert* ether was added to remove impurities. The aqueous phase was adjusted pH to 9, and extracted with DCM. The organic phase was washed with saturated brine, dried, rotary evaporated to dryness, and subjected to column chromatography to obtain a solid (95 mg), which formed a maleate in THF, and dried to obtain the title compound as a white solid (98 mg). ¹H NMR (400 MHz, DMSO-*d₆*) *δ* 8.46 (br, 2H), 7.73 (d, 1H), 7.70 (d, 1H), 7.63 (d, 1H), 7.34 (t, 1H), 7.15 (t, 1H), 7.06 (dd, 1H), 6.79 (d, 1H), 6.02 (s, 2H), 3.70 (dd, 2H), 3.37 (m, 2H), 3.00 (m, 4H), 2.70 (br, 2H), 2.00-1.79 (m, 4H). ESI-MS (m/z): 267.37 [M+H]⁺.

### Example 7: 6-(Benzothiophen-5-yl)decahydropyrrolo[3,4-d]azepine maleate

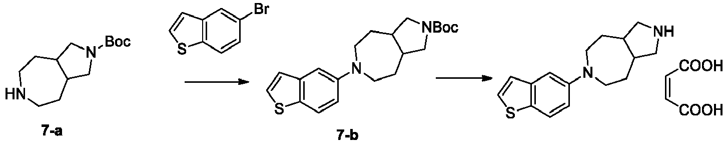

### Step 1:

820 mg of **7-a,** 1.1 eq of 5-bromobenzothiophene, 0.05 eq of Pd₂(dba)₃ and 0.08 eq of BINAP were mixed in toluene, stirred at room temperature for 5min, and added with 2 eq of potassium *tert*-butoxide. The atmosphere was replaced with nitrogen for three times and the reaction mixture was reacted at 80°C overnight. The insoluble matter was filtered off, and the filter cake was washed with ethyl acetate. The filtrate was combined, washed with brine, dried and concentrated, and then subjected to silica gel column chromatography to obtain 870 mg of a product, which was slurried in a small amount of dichloromethane to obtain **7-b** as a white solid (780 mg).

### Step 2:

**7-b** was dissolved in methanol, added with hydrochloric acid, and stirred at room temperature for 1 h. After the reaction, the reaction mixture was added with water and MTBE, and the organic phase was discarded. The aqueous phase was separated and adjusted pH to be weakly alkaline, and extracted with DCM. The organic phase was washed with brine, dried and concentrated to obtain an oil, which formed maleate in THF, and dried to obtain the title compound as a yellowish solid (920 mg). ¹H NMR (400 MHz, DMSO-*d₆*) *δ* 8.66 (d, 2H), 7.75 (d, 1H), 7.62 (d, 1H), 7.26 (d, 1H), 7.19 (d, 1H), 6.99 (dd, 1H), 6.09 (s, 2H), 3.83 (dd, 2H), 3.35 (brs, 2H), 3.18 (ddd, 2H), 2.77 (d, 2H), 2.56 (q, 2H), 1.66-1.85 (m, 4H). ESI-MS *(m*/*z):* 273.28 [M+H]⁺.

### Example 8: 5-(naphthalen-1-yl)octahydrocyclopenta[c]pyrrole hydrochloride

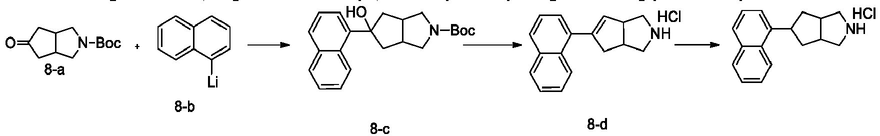

### Step 1:

500 mg of 1-bromonaphthalene was dissolved in 5 mL of THF under nitrogen protection, cooled at an extermal temperature of -80 °C to control the internal temperature to be less than -70 °C. 1.05 eq of *n*-BuLi (2.5M) was added dropwise to the THF solution of 1-bromonaphthalene, stirred at -70 °C for 1h to form **8-b.** The internal temperature was controlled to be less than -70 °C, the reaction mixture was added dropwise with 0.8 eq of **8-a** (dissolved in 3 mL THF) as a raw material, and slowly raised to -40 °C to be stirred for 12h. After quenched with an ammonium chloride aqueous solution, the reaction mixture was layered with ethyl acetate/water layers, and the ethyl acetate phase was concentrated to obtain a crude **8-c.**

### Step 2:

The crude **8-c** was dissolved in 5 mL of methanol, added with 2 mL of concentrated hydrochloric acid, and refluxed at 65°C for 1h. After cooled to room temperature, the reaction mixture was concentrated and replaced with acetone twice, slurried in acetone, filtered and dried to obtain 140 mg of **8-d.**

### Step 3:

90 mg of **8-d** was dissolved in 5 mL of acetic acid, added with 20 mg of 10% Pd/C, purged with hydrogen, and reacted at 25 °C for 24 h. The reaction solution was filtered, and the acetic acid phase was alkalized with 5 ml of a 20% NaOH and layered with 10 ml of DCM. The organic phase was concentrated. The residue was added with 3 mL of HCl-dioxane in an ice-water bath to form a salt. The solid was filtered, slurried in 5 mL of petroleum ether, filtered and dried to obtain 26 mg of the title compound as a white solid. ESI-MS m/z 238.32 [M+H]⁺.

### Example 9: 5-(3,4-chlorophenyl)-1,2,3,3a,4,6a-hexahydrocyclopenta[c]pyrrole hydrochloride

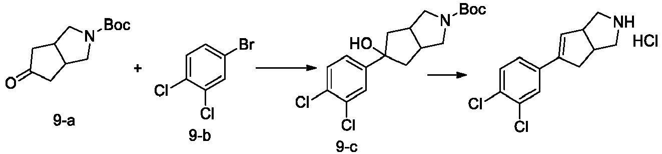

### Step 1:

500 mg of 3,4-dichlorobromobenzene 9-b (1 eq) was added to 5ml of a freshly distilled THF, decreased to -80 °C under nitrogen protection, and added dropwise with a butyl lithium solution (2.5M, 1.1eq), and the system is dissolved clearly. At this temperature, the resultant was added with 2 ml of a THF solution containing 300 mg of **9-a** (0.6 eq), and then naturally warmed to room temperature for reaction. The reaction system was quenched with ammonium chloride, extracted with ethyl acetate and water, dried, concentrated and subjected to column chromatography to obtain 90 mg of the intermediate **9-c.**

### Step 2:

The intermediate **9-c** was added to 2 ml of methanol, added with 1 ml of concentrated hydrochloric acid, and heated to reflux for 3h. The system was concentrated to dryness, and slurried in acetone to obtain 42 mg of the title compound as a white flaky crystal. ¹H NMR (400MHz, CD₃OD) *δ* 7.66 (d, 1H), 7.51 (d, 1H), 7.45 (dd, 1H), 6.17 (m, 1H), 3.81 (m, 1H), 3.57-3.26 (m, 4H), 3.15 (m, 2H), 2.74 (m, 1H).

### Example 10: 2-(Benzo[b]thiophen-7-yl)octahydropyrrolo[3,4-c]pyrrole maleate

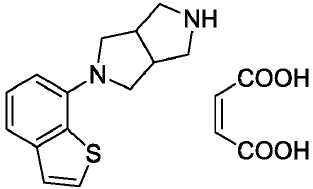

2-(Benzo[b]thiophen-7-yl)octahydropyrrolo[3,4-c]pyrrole was prepared in the same manner as that in Example 1, except using 7-bromo-benzo[b]thiophene instead of 2-bromonaphthalene.

The basic product was dissolved in methanol. 1.05 eq of maleic acid was dissolved in tetrahydrofuran, added dropwise into the methanol solution of the basic product, stirred at room temperature for 5min, concentrated, slurried in tetrahydrofuran, stirred at room temperature for 3 h and filtered. The filter cake was heated at 50 °C for 3h to obtain the title compound.

¹H NMR (400 MHz, DMSO-*d₆*) *δ* 8.77 (s, 2H), 7.72 (d, 1H), 7.46 (d, 1H), 7.43 (d, 1H), 7.29 (t, 1H), 6.77 (d, 1H), 6.02 (s, 2H), 3.47-3.60 (m, 4H), 3.27-3.37 (m, 2H), 3.03-3.15 (m, 4H). ESI-MS *(m*/*z):* 245.22 [M+H]⁺.

### Example 11: 2-(Benzo[b]thiophen-7-yl)-5-methyloctahydropyrrolo[3,4-c]pyrrole oxalate

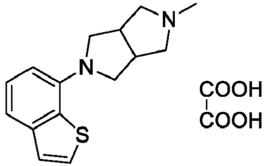

2-(Benzo[b]thiophen-7-yl)-5-methyloctahydropyrrolo[3,4-c]pyrrole was prepared in the same manner as that in Example 2, except using the basic product of Example 10 instead of the product of Example 1.

The basic product was dissolved in methanol. 1.05 eq of oxalic acid was dissolved in tetrahydrofuran, added dropwise to the methanol solution of the basic product, stirred at room temperature for 5min, concentrated, slurried in tetrahydrofuran, stirred at room temperature for 3 h and filtered. The filter cake was heated at 50 °C for 3h to obtain the title compound.

¹H NMR (400 MHz, DMSO-*d₆*) *δ* 9.59 (brs, 2H), 7.72 (d, 1H), 7.47 (d, 1H), 7.44 (d, 1H), 7.29 (t, 1H), 6.79 (d, 1H), 3.64 (dd, 2H), 3.52 (dd, 2H), 3.24 (t, 2H), 3.13 (m, 2H), 3.01 (m, 2H), 2.80 (s, 3H). ESI-MS *(m*/*z):* 259.27 [M+H]⁺.

### Example 12: 2-(Benzo[b]thiophen-4-yl)octahydropyrrolo[3,4-c]pyrrole maleate

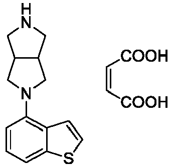

The basic product was prepared in the same manner as that in Example 1, except using 4-bromo-benzo[b]thiophene instead of 2-bromonaphthalene, and the maleate was formed in accordance with Example 10 to obtain 2-(Benzo[b]thiophen-4-yl)octahydropyrrolo[3,4-c]pyrrole maleate.

¹H NMR (400 MHz, DMSO-*d₆*) *δ* 8.78 (s, 2H), 7.67 (d, 1H), 7.64 (d, 1H), 7.55 (d, 1H), 7.24 (t, 1H), 6.76 (d, 1H), 6.02 (s, 2H), 3.51 (dd, 2H), 3.43 (dd, 2H), 3.23 (dd, 2H), 3.04-3.20 (m, 4H). ESI-MS *(m*/*z):* 245.21 [M+H]⁺.

### Example 13: 2-(Benzo[b]thiophen-4-yl)-5-methyloctahydropyrrolo[3,4-c]pyrrole hydrobromide

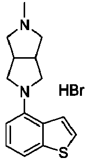

2-(Benzo[b]thiophen-4-yl)-5-methyloctahydropyrrolo[3,4-c]pyrrole was prepared in the same manner as that in Example 2, except using the basic product of Example 12 instead of the product of Example 1, and the hydrobromide was formed in accordance with Example 10 by using a 40% aqueous hydrobromic acid instead of the tetrahydrofuran solution of maleic acid in Example 10, to give the title compound.

¹H NMR (400 MHz, DMSO-*d₆*) *δ* 9.80 (d, 1H), 7.56-7.72 (m, 3H), 7.26 (t, 1H), 6.82 (dd, 1H), 3.89 (td, 1H), 3.61 (dd, 1H), 3.20-3.55 (m, 5H), 2.92-3.11 (m, 3H), 2.87 (dd, 3H). ESI-MS *(m*/*z):* 259.30 [M+H]⁺.

### Example 14: 2-(Benzo[b]thiophen-5-yl)octahydropyrrolo[3,4-c]pyrrole maleate

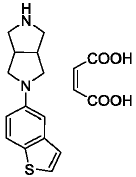

The basic product was prepared in the same manner as that in Example 1, except using 5-bromo-benzo[b]thiophene instead of 2-bromonaphthalene, and the maleate was formed in accordance with Example 10 to obtain 2-(Benzo[b]thiophen-5-yl)octahydropyrrolo[3,4-c]pyrrole maleate.

¹H NMR (400 MHz, DMSO-*d₆*) *δ* 8.78 (s, 2H), 7.79 (d, 1H), 7.65 (d, 1H), 7.29 (d, 1H), 7.08 (d, 1H), 6.88 (dd, 1H), 6.01 (s, 2H), 3.44-3.52 (m, 2H), 3.39 (dd, 2H), 3.26-3.30 (m, 2H), 3.07-3.17 (m, 4H). ESI-MS *(m*/*z):* 245.18 [M+H]⁺.

### Example 15: 2-(Benzo[b]thiophen-5-yl)-5-methyloctahydropyrrolo[3,4-c]pyrrole hydrochloride

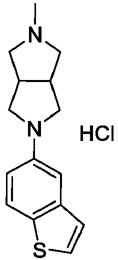

2-(Benzo[b]thiophen-5-yl)-5-methyloctahydropyrrolo[3,4-c]pyrrole was prepared in the same manner as that in Example 2, except using the basic product of Example 14 instead of the product of Example 1, and the hydrochloride was formed in accordance with Example 10 by using a methanol solution of hydrochloride instead of the tetrahydrofuran solution of maleic acid in Example 10, to give the title compound.

¹H NMR (400 MHz, CD₃OD) *δ* 8.06 (m, 3H), 7.73 (t, 1H), 7.63 (dd, 1H), 7.43 (t, 1H), 3.97 (m, 4H), 3.85 (d, 1H), 3.75 (m, 1H), 3.65-3.46 (m, 3H), 3.43 (dd, 1H), 3.01 (d, 3H). ESI-MS *(m*/*z):* 259.23 [M+H]⁺.

### Example 16: 2-(3,4-dichlorophenyl)octahydropyrrolo[3,4-c]pyrrole

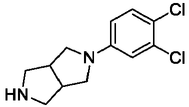

2-(3,4-dichlorophenyl)octahydropyrrolo[3,4-c]pyrrole was prepared in the same manner as that in Example 1, except using 1-bromo-3,4-dichlorobenzene instead of 2-bromonaphthalene.

¹H NMR (400 MHz, MeOH-*d*₄) δ 7.29 (d, 1H), 6.84 (d, 1H), 6.65 (dd, 1H), 3.59 (m, 2H), 3.42 (m, 2H), 3.33 (m, 2H), 3.27-3.18 (m, 4H). ESI-MS *(m*/*z):* 257.29 [M+H]⁺.

### Example 17: 2-(3,4-dichlorophenyl)-5-methyloctahydropyrrolo[3,4-c]pyrrole

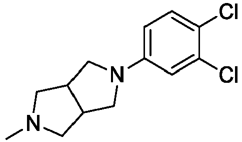

2-(3,4-dichlorophenyl)-5-methyloctahydropyrrolo[3,4-c]pyrrole was prepared in the same manner as that in Example 2, except using the product of Example 16 instead of the product of Example 1.

¹H NMR (400 MHz, CDCl₃) *δ* 7.20 (d, 1H), 6.67 (d, 1H), 6.44 (dd, 1H), 3.37 (t, 2H), 3.14 (dd, 2H), 2.97 (m, 2H), 2.70 (t, 2H), 2.46 (dd, 2H), 2.33 (s, 3H). ESI-MS *(m*/*z):* 271.24 [M+H]⁺.

### Example 18: 2-(Benzofuran-4-yl)octahydropyrrolo[3,4-c]pyrrole hydrochloride

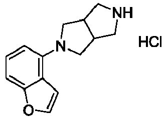

2-(Benzofuran-4-yl)octahydropyrrolo[3,4-c]pyrrole was prepared in the same manner as that in Example 1, except using 4-bromobenzofuran instead of 2-bromonaphthalene, and the hydrochloride was formed in accordance with Example 10 by using a methanol solution of hydrochloride instead of the tetrahydrofuran solution of maleic acid in Example 10, to give the title compound.

¹H NMR (400 MHz, DMSO-*d₆*) *δ* 9.46 (d, 2H), 7.84 (d, 1H), 7.16 (d, 1H), 7.13 (t, 1H), 6.98 (d, 1H), 6.41 (d, 1H), 3.40-3.60 (m, 6H), 3.06-3.15 (m, 4H). ESI-MS *(m*/*z):* 229.19 [M+H]⁺.

### Example 19: 2-(Benzo[b]thiophen-6-yl)octahydropyrrolo[3,4-c]pyrrole maleate

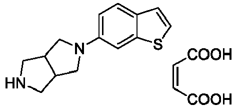

2-(Benzo[b]furan-4-yl)octahydropyrrolo[3,4-c]pyrrole was prepared in the same manner as that in Example 1, except using 6-bromo-benzo[b]thiophene instead of 2-bromonaphthalene, and the maleate was formed in accordance with Example 10 to obtain the title compound. ¹H NMR (400 MHz, DMSO-*d₆*) *δ* 8.83 (s, 2H), 7.69 (d, 1H), 7.35 (d, 1H), 7.25 (d, 1H), 7.18 (d, 1H), 6.85 (dd, 1H), 6.02 (s, 2H), 3.48 (m, 2H), 3.40 (dd, 2H), 3.33 (m, 2H), 3.05-3.17 (m, 4H). ESI-MS *(m*/*z):* 245.39 [M+H]⁺.

### Example 20: 2-(Benzofuran-7-yl)octahydropyrrolo[3,4-c]pyrrole

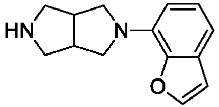

2-(Benzofuran-7-yl)octahydropyrrolo[3,4-c]pyrrole was prepared in the same manner as that in Example 1, except using 7-bromo-benzofuran instead of 2-bromonaphthalene.

¹H NMR (400 MHz, DMSO-*d₆*) *δ* 7.90 (d, 1H), 7.03 (m, 2H), 6.86 (d, 1H), 6.51 (d, 1H), 3.56 (m, 2H), 3.24 (dd, 2H), 2.94 (m, 2H), 2.78 (m, 2H), 2.62 (m, 2H). ESI-MS *(m*/*z):* 229.34 [M+H]⁺.

### Example 21: 2-(Benzofuran-5-yl)octahydropyrrolo[3,4-c]pyrrole maleate

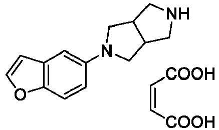

2-(Benzofuran-7-yl)octahydropyrrolo[3,4-c]pyrrole was prepared in the same manner as that in Example 1, except using 5-bromo-benzofuran instead of 2-bromonaphthalene, and the maleate was formed in accordance with Example 10 to obtain the title compound.

¹H NMR (400 MHz, DMSO-*d₆*) *δ* 8.78 (s, 2H), 7.86 (d, 1H), 7.43 (d, 1H), 6.86 (d, 1H), 6.81 (dd, 1H), 6.76 (dd, 1H), 6.02 (s, 2H), 3.47 (m, 2H), 3.37 (m, 2H), 3.22 (m, 2H), 3.05-3.15 (m, 4H). ESI-MS *(m*/*z):* 229.27 [M+H]⁺.

### Example 22: 2-(Benzo[b]thiophen-2-yl)octahydropyrrolo[3,4-c]pyrrole maleate

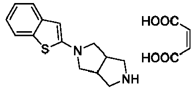

2-(Benzo[b]thiophen-2-yl)octahydropyrrolo[3,4-c]pyrrole was prepared in the same manner as that in Example 1, except using 2-bromo-benzo[b]thiophene instead of 2-bromonaphthalene, and the maleate was formed in accordance with Example 10 to obtain the title compound.

¹H NMR (400 MHz, DMSO-*d₆*) *δ* 8.81 (s, 2H), 7.68 (d, 1H), 7.45 (d, 1H), 7.21 (td, 1H), 7.03 (t, 1H), 6.09 (s, 1H), 6.02 (s, 2H), 3.37-3.49 (m, 4H), 3.33 (m, 2H), 3.09-3.20 (m, 4H). ESI-MS *(m*/*z):* 245.18 [M+H]⁺.

### Example 23: 2-(Benzo[b]thiophen-3-yl)octahydropyrrolo[3,4-c]pyrrole maleate

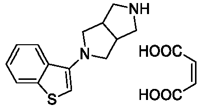

2-(Benzo[b]thiophen-3-yl)octahydropyrrolo[3,4-c]pyrrole was prepared in the same manner as that in Example 1, except using 3-bromo-benzo[b]thiophene instead of 2-bromonaphthalene, and the maleate was formed in accordance with Example 10 to obtain the title compound.

¹H NMR (400 MHz, DMSO-*d₆*) *δ* 8.73 (s, 2H), 7.96 (dd, 1H), 7.91 (dd, 1H), 7.38 (m, 2H), 6.78 (s, 1H), 6.02 (s, 2H), 3.50 (m, 2H), 3.26-3.46 (m, 2H), 3.17 (d, 2H), 3.01-3.11 (m, 4H). ESI-MS *(m*/*z):* 245.18 [M+H]⁺.

### Example 24: 2-(naphthalen-1-yl)octahydropyrrolo[3,4-c]pyrrole maleate

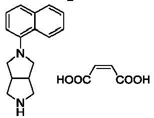

2-(naphthalen-1-yl)octahydropyrrolo[3,4-c]pyrrole was prepared in the same manner as that in Example 1, except using 1-bromonaphthalene instead of 2-bromonaphthalene, and the maleate was formed in accordance with Example 10 to obtain the title compound.

¹H NMR (400MHz, DMSO-*d*₆) *δ* 8.73 (s, 2H), 8.15 (m, 1H), 7.89 (m, 1H), 7.59 (d, 1H), 7.52 (m, 2H), 7.42 (t, 1H), 7.11 (d, 1H), 6.01 (s, 2H), 3.57 (m, 2H), 3.28 (d, 2H), 3.19 (dd, 2H), 3.09 (d, 4H). ESI-MS m/z 239.27 [M+H]⁺.

### Example 25: 2-ethyl-5-(naphthalen-2-yl)octahydropyrrolo[3,4-c]pyrrole oxalate

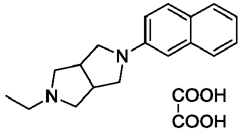

2-ethyl-5-(naphthalen-2-yl)octahydropyrrolo[3,4-c]pyrrole was prepared in the same manner as that in Example 2, except using ethyl acetate instead of ethyl formate, and the oxalate was formed in accordance with Example 11 to obtain the title compound.

¹H NMR (400MHz, DMSO-*d₆*) *δ* 7.75 (d, 1H), 7.72 (d, 1H), 7.68 (d, 1H), 7.36 (m, 1H), 7.18 (m, 2H), 6.92 (d, 1H), 3.59 (br, 2H), 3.51 (m, 2H), 3.32 (m, 2H), 3.13 (m, 6H), 1.2 (t, 3H). ESI-MS m/z 267.40[M+H]⁺.

### Example 26: 2-methyl-5-(naphthalen-1-yl)octahydropyrrolo[3,4-c]pyrrole oxalate

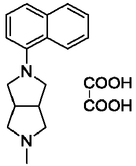

2-methyl-5-(naphthalen-1-yl)octahydropyrrolo[3,4-c]pyrrole was prepared in the same manner as that in Example 2, except using the basic product of Example 24 instead of the product of Example 1, and the oxalate was formed in accordance with Example 11 to obtain the title compound.

¹H NMR (400MHz, DMSO-*d₆*) *δ* 8.18 (m, 1H), 7.88 (m, 1H), 7.59 (d, 1H), 7.52 (m, 2H), 7.41 (t, 1H), 7.11 (d, 1H), 3.62 (br, 2H), 3.28 (d, 2H), 3.08 (m, 6H), 2.86 (s, 3H). ESI-MS m/z 253.33 [M+H]⁺.

### Example 27: 2-(2,3-dichlorophenyl)octahydropyrrolo[3,4-c]pyrrole maleate

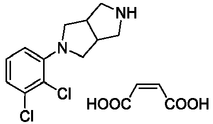

2-(2,3-dichlorophenyl)octahydropyrrolo[3,4-c]pyrrole was prepared in the same manner as that in Example 1, except using 1-bromo-2,3-dichlorobenzene instead of 2-bromonaphthalene, and the maleate was formed in accordance with Example 10 to obtain the title compound.

¹H NMR (400MHz, DMSO-*d₆*) *δ* 8.67 (s, 2H), 7.29 (m, 2H), 7.10 (dd, 1H), 6.01 (s, 2H), 3.53 (m, 2H), 3.34 (d, 2H), 3.00 (m, 6H). ESI-MS m/z 257.23 [M+H]⁺.

### Example 28: 2-(2,3-dichlorophenyl)-5-methyloctahydropyrrolo[3,4-c]pyrrole oxalate

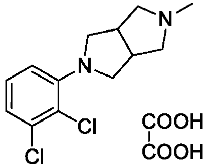

2-(2,3-dichlorophenyl)-5-methyloctahydropyrrolo[3,4-c]pyrrole was prepared in the same manner as that in Example 2, except using the basic product of Example 27 instead of the product of Example 1, and the oxalate was formed in accordance with Example 11 to obtain the title compound.

¹H NMR (400MHz, DMSO-*d₆*) *δ* 7.29 (m, 2H), 7.10 (dd, 1H), 3.58 (m, 2H), 3.32 (d, 2H), 3.00 (m, 6H), 2.77 (s, 3H). ESI-MS m/z 271.23 [M+H]⁺.

### Example 29: 2-(6-fluoronaphthalen-2-yl)octahydropyrrolo[3,4-c]pyrrole maleate

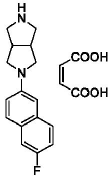

2-(6-fluoronaphthalen-2-yl)octahydropyrrolo[3,4-c]pyrrole was prepared in the same manner as that in Example 1, except using 2-bromo-6-fluoronaphthalene instead of 2-bromonaphthalene, and the maleate was formed in accordance with Example 10 to obtain the title compound.

¹H NMR (400MHz, DMSO-*d₆*) *δ* 8.88 (s, 2H), 7.76 (d, 1H), 7.74 (d, 1H), 7.52 (dd, 1H), 7.28 (td, 1H), 7.19 (dd, 1H), 6.95 (d, 1H), 6.02 (s, 2H), 3.43 (m, 6H), 3.12 (m, 4H). ESI-MS m/z 257.31 [M+H]⁺.

### Example 30: 2-(6-fluoronaphthalen-2-yl)-5-methyloctahydropyrrolo[3,4-c]pyrrole maleate

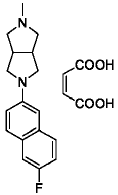

2-(6-fluoronaphthalen-2-yl)-5-methyloctahydropyrrolo[3,4-c]pyrrole was prepared in the same manner as that in Example 2, except using the basic product of Example 29 instead of the product of Example 1, and the maleate was formed in accordance with

Example 10 to obtain the title compound.

¹H NMR (400MHz, DMSO-*d₆*) *δ* 7.76 (dt, 2H), 7.53 (dd, 1H), 7.29 (td, 1H), 7.24 (dd, 1H), 7.01 (d, 1H), 6.02 (s, 2H), 3.54 (m, 4H), 3.24 (m, 6H), 2.84 (s, 3H). ESI-MS m/z 271.29 [M+H]⁺.

### Example 31: 2-(1-fluoronaphthalen-2-yl)octahydropyrrolo[3,4-c]pyrrole maleate

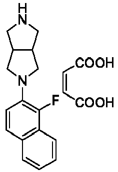

2-(l-fluoronaphthalen-2-yl)octahydropyrrolo[3,4-c]pyrrole was prepared in the same manner as that in Example 1, except using 2-bromo-1-fluoronaphthalene instead of 2-bromonaphthalene, and the maleate was formed in accordance with Example 10 to obtain the title compound.

¹H NMR (400MHz, DMSO-*d₆*) *δ* 8.83 (s, 2H), 7.86 (m, 2H), 7.67 (d, 1H), 7.51 (m, 1H), 7.35 (m, 1H), 7.25 (t, 1H), 6.02 (s, 2H), 3.49 (m, 6H), 3.10 (m, 4H). ESI-MS m/z 257.25 [M+H]⁺.

### Example 32: 2-(1-fluoronaphthalen-2-yl)-5-methyloctahydropyrrolo[3,4-c]pyrrole maleate

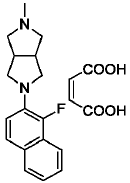

2-(1-fluoronaphthalen-2-yl)-5-methyloctahydropyrrolo[3,4-c]pyrrole was prepared in the same manner as that in Example 2, except using the basic product of Example 31 instead of the product of Example 1, and the maleate was formed in accordance with Example 10 to obtain the title compound.

¹H NMR (400MHz, DMSO-*d₆*) *δ* 7.89 (d, 1H), 7.86 (d, 1H), 7.68 (d, 1H), 7.52 (m, 1H), 7.37 (m, 1H), 7.28 (t, 1H), 6.02 (s, 2H), 3.60 (m, 2H), 3.31 (br, 6H), 3.13 (br, 2H), 2.85 (s, 3H). ESI-MS m/z 271.33 [M+H]⁺.

### Example 33: 3-(hexahydropyrrolo[3,4-c] pyrrole-2(1H)-yl)isoquinoline maleate

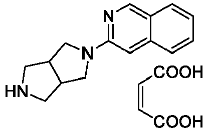

3-(hexahydropyrrolo[3,4-c]pyrrole-2(1H)-yl)isoquinoline was prepared in the same manner as that in Example 1, except using 3-bromoisoquinoline instead of 2-bromonaphthalene, and the maleate was formed in accordance with Example 10 to obtain the title compound.

¹H NMR (400MHz, DMSO-*d₆*) *δ* 8.96 (s, 1H), 8.85 (s, 2H), 7.86 (d, 1H), 7.66 (d, 1H), 7.53 (m, 1H), 7.24 (m, 1H), 6.69 (s, 1H), 6.07 (s, 2H), 3.52 (m, 6H), 3.14 (m, 4H). ESI-MS m/z 240.32 [M+H]⁺.

### Example 34: 3-(5-methylhexahydropyrrolo[3,4-c]pyrrole-2(1H)-yl)isoquinoline maleate

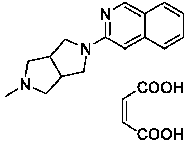

3-(5-methylhexahydropyrrolo[3,4-c]pyrrole-2(1H)-yl)isoquinoline was prepared in the same manner as that in Example 2, except using the basic product of Example 33 instead of the product of Example 1, and the maleate was formed in accordance with Example 10 to obtain the title compound.

¹H NMR (400MHz, DMSO-*d₆*) *δ* 8.98 (s, 1H), 7.87 (d, 1H), 7.67 (d, 1H), 7.54 (t, 1H), 7.26 (t, 1H), 6.75 (s, 1H), 6.06 (s, 2H), 3.48 (m, 6H), 2.85 (s, 3H), 2.77 (m, 2H), 2.50 (m, 2H). ESI-MS m/z 254.41 [M+H]⁺.

### Example 35: 6-(3,4-dichlorophenyl)decahydropyrrolo[3,4-d]azepine

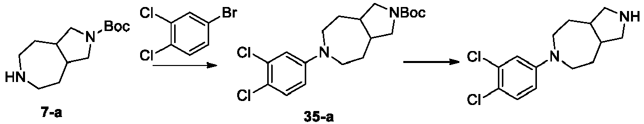

### Step 1:

800 mg of **7-a** as a raw material, 1.1 eq of 3,4-dichlrobromobenzene as a raw material, 0.05 eq of Pd₂(dba)₃, 0.08 eq of BINAP were mixed in toluene, stirred at room temperature for 5 min, and added with 2 eq of potassium *tert*-butoxide. The atmosphere was replaced with nitrogen for three times and the reaction mixture was reacted at 80°C overnight. The insoluble matter was filtered off, and the filter cake was washed with ethyl acetate. The filtrate was combined, washed with brine, dried and concentrated, and then subjected to silica gel column chromatography to obtain 850 mg of **35-a.**

### Step 2:

The **35-a** was dissolved in methanol, added with hydrochloric acid, and stirred at room temperature for 1 h. After the reaction, the reaction mixture was added with water and MTBE, and the organic phase was discarded. The aqueous phase was separated and adjusted pH to be weakly alkaline, added with DCM to extract. The organic phase was washed with brine, dried and concentrated, and then subjected to silica gel column chromatography to obtain the title compound as a white solid. ESI-MS *(m*/*z):* 285.22 [M+H]⁺.

### Example 36: 6-(Benzo[b]thiophen-7-yl)decahydropyrrolo[3,4-d]azepine maleate

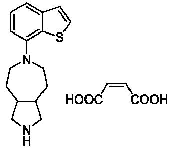

6-(Benzo[b]thiophen-7-yl)decahydropyrrolo[3,4-d]azepine maleate was prepared in the same manner as that in Example 7, except using 7-bromo-benzo[b]thiophene instead of 5-bromobenzothiophene.

¹H NMR (400MHz, DMSO-*d*₆) *δ* 8.69 (s, 2H), 7.71 (d, 1H), 7.50 (d, 1H), 7.43 (d, 1H), 7.30 (t, 1H), 6.94 (d, 1H), 6.02 (s, 2H), 3.49 (m, 4H), 3.08 (m, 2H), 2.85 (m, 2H), 2.66 (m, 2H), 1.90 (m, 4H). ESI-MS m/z 273.29 [M+H]⁺.

### Example 37: 6-(2,3-dichlorophenyl)decahydropyrrolo[3,4-d]azepine maleate

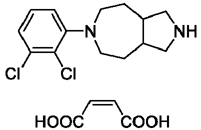

6-(2,3-dichlorophenyl)decahydropyrrolo[3,4-d]azepine maleate was prepared in the same manner as that in Example 7, except using 1-bromo-2,3-dichlorobenzene instead of 5-bromobenzothiophene.

¹H NMR (400MHz, DMSO-*d*₆) *δ* 8.66 (s, 2H), 7.26 (m, 2H), 7.13 (dd, 1H), 6.02 (s, 2H), 3.43 (m, 2H), 3.23 (m, 2H), 2.91 (m, 2H), 2.81 (m, 2H), 2.63 (m, 2H), 1.86 (m, 4H). ESI-MS m/z 285.38 [M+H]⁺.

### Example 38: 6-(Benzo[b]thiophen-4-yl)decahydropyrrolo[3,4-d]azepine maleate

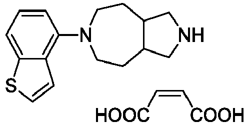

6-(Benzo[b]thiophen-4-yl)decahydropyrrolo[3,4-d]azepine maleate was prepared in the same manner as that in Example 7, except using 4-bromo-benzo[b]thiophene instead of 5-bromobenzothiophene.

¹H NMR (400MHz, DMSO-*d*₆) *δ* 8.69 (s, 2H), 7.69 (d, 1H), 7.55 (d, 1H), 7.37 (d, 1H), 7.24 (t, 1H), 6.87 (d, 1H), 6.02 (s, 2H), 3.45 (m, 4H), 3.05 (m, 2H), 2.85 (m, 2H), 2.66 (m, 2H), 1.93 (m, 4H). ESI-MS m/z 273.47 [M+H]⁺.

### Example 39: 6-(naphthalen-2-yl)decahydropyrrolo[3,4-d]azepine maleate

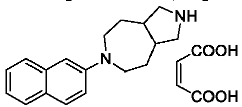

6-(naphthalen-2-yl)decahydropyrrolo[3,4-d]azepine maleate was prepared in the same manner as that in Example 7, except using 2-bromonaphthalene instead of 5-bromobenzothiophene.

¹H NMR (400 MHz, DMSO-*d₆*) *δ* 8.75 (s, 2H), 7.72 (d, 1H), 7.68 (d, 1H), 7.63 (d, 1H), 7.33 (td, 1H), 7.26 (dd, 1H), 7.16 (td, 1H), 7.00 (d, 1H), 6.01 (s, 2H), 3.90 (m, 2H), 3.30 (m, 4H), 2.78 (m, 2H), 2.56 (m, 2H), 1.76 (m, 4H). ESI-MS *(m*/*z):* 267.27 [M+H]⁺.

### Example 40: 2-methyl-6-(naphthalen-2-yl)decahydropyrrolo[3,4-d]azepine oxalate

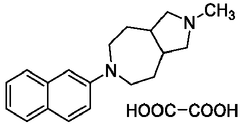

2-methyl-6-(naphthalen-2-yl)decahydropyrrolo[3,4-d]azepine was prepared in the same manner as that in Example 2, except using the basic product of Example 39 instead of the product of Example 1, and the oxalate was formed in accordance with Example 11 to obtain the title compound.

^{l}H NMR (400 MHz, DMSO-*d₆*) *δ* 7.69 (m, 3H), 7.31 (m, 2H), 7.17 (t, 1H), 7.04 (d, 1H), 4.01 (m, 2H), 3.52 (m, 2H), 3.17 (m, 2H), 2.72(m, 7H), 1.78 (m, 4H). ESI-MS *(m*/*z):* 281.36 [M+H]⁺.

### Example 41: 2-(Benzo[b]thiophen-4-yl)decahydropyrrolo[3,4-d]azepine maleate

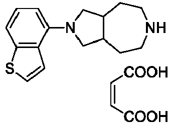

2-(Benzo[b]thiophen-4-yl)decahydropyrrolo[3,4-d]azepine maleate was prepared in the same manner as that in Example 6, except using 4-bromo-benzo[b]thiophene instead of 2-bromonaphthalene.

¹H NMR (400MHz, DMSO-*d₆*) *δ* 8.73 (s, 1H), 8.37 (s, 1H), 7.62 (d, 1H), 7.58 (d, 1H), 7.40 (d, 1H), 7.19 (t, 1H), 6.60 (d, 1H), 6.04 (s, 2H), 3.72 (m, 2H), 3.38 (m, 2H), 3.01 (m, 4H), 2.65 (m, 2H), 1.90 (m, 4H). ESI-MS m/z 273.39 [M+H]⁺.

### Example 42: 2-(3,4-dichlorophenyl)decahydropyrrolo[3,4-d]azepine maleate

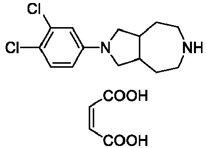

2-(3,4-dichlorophenyl)decahydropyrrolo[3,4-d]azepine maleate was prepared in the same manner as that in Example 6, except using 1-bromo-3,4-dichlorobenzene instead of 2-bromonaphthalene.

¹H NMR (400MHz, DMSO-*d₆*) *δ* 8.31 (br, 1H), 7.34 (d, 1H), 6.70 (d, 1H), 6.52 (dd, 1H), 6.02 (s, 2H), 3.53 (m, 2H), 3.33 (m, 2H), 2.94 (m, 4H), 2.65 (m, 2H), 1.85 (m, 4H). ESI-MS m/z 285.38 [M+H]⁺.

### Example 43: 2-(Benzo[b]thiophen-7-yl)decahydropyrrolo[3,4-d]azepine maleate

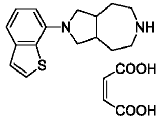

2-(Benzo[b]thiophen-7-yl)decahydropyrrolo[3,4-d]azepine maleate was prepared in the same manner as that in Example 6, except using 7-bromo-benzo[b]thiophene instead of 2-bromonaphthalene.

¹H NMR (400MHz, DMSO-*d₆*) *δ* 8.73 (s, 1H), 8.39 (s, 1H), 7.68 (d, 1H), 7.40 (d, 1H), 7.36 (d, 1H), 7.24 (t, 1H), 6.64 (d, 1H), 6.03 (s, 2H), 3.79 (m, 2H), 3.38 (m, 2H), 3.00 (m, 4H), 2.67 (m, 2H), 1.89 (m, 4H). ESI-MS m/z 273.40 [M+H]⁺.

### Example 44: 5-(Benzo[b]thiophen-6-yl)octahydrocyclopenta[c]pyrrole hydrochloride

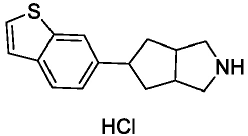

5-(Benzo[b]thiophen-6-yl)octahydrocyclopenta[c]pyrrole hydrochloride was prepared in the same manner as that in Example 3, except using 6-bromo-benzothiophene instead of 4-bromo-benzothiophene.

¹H NMR (400MHz, CD₃OD) *δ* 8.07 (d, 1H), 7.73 (d, 1H), 7.57 (d, 1H), 7.47 (dd, 1H), 7.35 (d, 1H), 3.28 (m, 2H), 3.20 (m, 2H), 3.09 (m, 1H), 2.90 (m, 2H), 2.40 (m, 2H), 1.37 (m, 2H). ESI-MS m/z 244.20 [M+H]⁺.

### Example 45: 5-(Benzo[b]thiophen-7-yl)octahydrocyclopenta[c]pyrrole hydrochloride

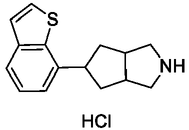

5-(Benzo[b]thiophen-7-yl)octahydrocyclopenta[c]pyrrole hydrochloride was prepared in the same manner as that in Example 3, except using 7-bromo-benzothiophene instead of 4-bromo-benzothiophene.

¹H NMR (400MHz, CD₃OD) *δ* 7.83 (d, 1H), 7.65 (d, 1H), 7.50 (m, 2H), 7.28 (t, 1H), 3.28 (m, 2H), 3.20 (m, 2H), 3.09 (m, 1H), 2.90 (m, 2H), 2.40 (m, 2H), 1.37 (m, 2H). ESI-MS m/z 244.22 [M+H]⁺.

### Example 46: 6-(Benzo[b]thiophen-5-yl)decahydrocyclohepta[c]pyrrole hydrochloride

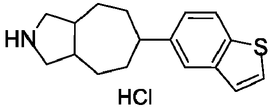

6-(Benzo[b]thiophen-5-yl)decahydrocyclohepta[c]pyrrole hydrochloride was prepared in the same manner as that in Example 5, except using 5-bromo-benzothiophene instead of 4-bromo-benzothiophene.

¹H NMR (400MHz, CD₃OD) *δ* 8.02 (d, 1H), 7.82 (d, 1H), 7.64 (d, 1H), 7.45 (dd, 1H), 7.35 (d, 1H), 3.52 (m, 3H), 2.87 (dd, 2H), 2.53 (m, 2H), 1.89 (m, 6H), 1.56 (m, 2H). ESI-MS m/z 272.27 [M+H]⁺.

### Example 47: 6-(Benzo[b]thiophen-6-yl)decahydrocyclohepta[c]pyrrole hydrochloride

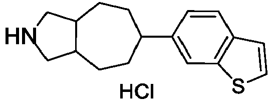

6-(Benzo[b]thiophen-6-yl)decahydrocyclohepta[c]pyrrole hydrochloride was prepared in the same manner as that in Example 5, except using 6-bromo-benzothiophene instead of 4-bromo-benzothiophene.

¹H NMR (400MHz, CD₃OD) *δ* 8.08 (d, 1H), 7.75 (d, 1H), 7.58 (d, 1H), 7.48 (dd, 1H), 7.36 (d, 1H), 3.53 (m, 3H), 2.87 (dd, 2H), 2.52 (m, 2H), 1.89 (m, 6H), 1.56 (m, 2H). ESI-MS m/z 272.29 [M+H]⁺.

### Example 48: 5-(Benzothiophen-7 -yl)-1,2,3,3a,4,6a-hexahydrocyclopenta[c]pyrrole hydrochloride

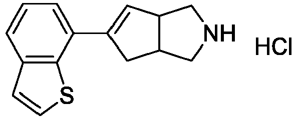

5-(Benzothiophen-7-yl)-1,2,3,3a,4,6a-hexahydrocyclopenta[c]pyrrole hydrochloride was prepared in the same manner as that in Example 9, except using 7-bromo-benzothiophene instead of 3,4-dichlorobromobenzene.

ESI-MS m/z 242.27 [M+H]⁺.

### Example 49: 5-(benzo[b]thiophen-2-yl)octahydrocyclopenta[c]pyrrol-5-ol

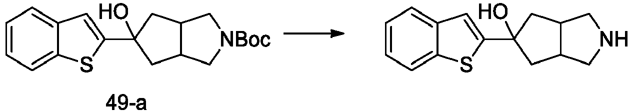

The intermediate **49-a** was prepared in the same preparation method as the **9-c** in Example 9, except using 2-bromobenzothiophene instead of 3, 4-dichlorobromobenzene.

110 mg of the intermediate was added to 3 ml of toluene, added with 550 mg of an acidic silica gel, and heated to reflux overnight. The reaction mixture was concentrated to remove the solvent and subjected to column chromatography to obtain 50 mg of the product, which was slurried in petroleum ether to obtain 26 mg of the title compound as an off-white solid.

¹H NMR (400MHz, CDCl₃) *δ* 7.81 (d, 1H), 7.69 (d, 1H), 7.28 (m, 2H), 7.17 (s, 1H), 3.06 (d, 2H), 2.97-2.82 (m, 4H), 2.49 (dd, 2H), 2.07 (d, 2H). ESI-MS m/z 260.14 [M+H]⁺.

### Example 50: 5-(benzothiophen-2-yl)octahydrocyclopenta[c]pyrrole hydrochloride

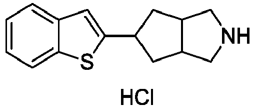

5-(benzothiophen-2-yl)octahydrocyclopenta[c]pyrrole hydrochloride was prepared in the same manner as that in Example 3, except using 2-bromo-benzothiophene instead of 4-bromo-benzothiophene.

¹H NMR (400MHz, CD₃OD) *δ* 7.78 (d, 1H), 7.70 (d, 1H), 7.28 (m, 2H), 7.15 (s, 1H), 3.46 (m, 1H), 3.40-3.24 (m, 4H), 3.08 (m, 2H), 2.55 (m, 2H), 1.69 (m, 2H). ESI-MS m/z 244.24 [M+H]⁺.

### Example 51: 5-(Benzothiophen-2-yl)-1,2,3,3a,4,6a-hexahydrocyclopenta[c]pyrrole hydrochloride

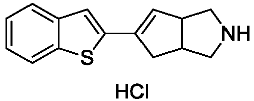

5-(Benzothiophen-2-yl)-1,2,3,3a,4,6a-hexahydrocyclopenta[c]pyrrole hydrochloride was prepared in the same manner as that in Example 9, except using 2-bromo-benzothiophene instead of 3,4-dichlorobromobenzene.

¹H NMR (400MHz, DMSO-*d₆*) *δ* 9.62 (br, 1H), 9.15 (br, 1H), 7.91 (m, 1H), 7.81 (m, 1H), 7.35 (m, 3H), 5.99 (m, 1H), 3.67 (m, 1H), 3.43-3.24 (m, 2H), 3.17 (m, 2H), 3.10-2.93 (m, 2H), 2.77 (m, 1H). ESI-MS m/z 242.25 [M+H]⁺.

### Example 52: 5-(Benzofuran-2-yl)-1,2,3,3a,4,6a-hexahydrocyclopenta[c]pyrrole hydrochloride

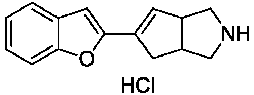

5-(Benzofuran-2-yl)-1,2,3,3a,4,6a-hexahydrocyclopenta[c]pyrrole hydrochloride was prepared in the same manner as that in Example 9, except using 2-bromo-benzofuran instead of 3,4-dichlorobromobenzene.

^{l}H NMR (400MHz, DMSO-*d₆*) *δ* 9.30 (br, 2H), 7.63 (d, 1H), 7.56 (d, 1H), 7.31 (t, 1H), 7.24 (t, 1H), 6.88 (s, 1H), 6.18 (m, 1H), 3.71 (m, 1H), 3.34 (m, 2H), 3.18 (m, 2H), 2.99 (m, 2H), 2.69 (m, 1H). ESI-MS m/z 226.21 [M+H]⁺.

### Example 53: 5-(benzofuran-2-yl)octahydrocyclopenta[c]pyrrol-5-ol

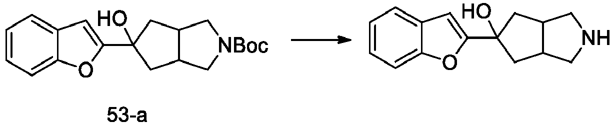

The intermediate **53-a** was prepared in the same preparation method as the **9-c** in Example 9, except using 2-bromobenzofuran instead of 3,4-dichlorobromobenzene.

120 mg of the intermediate and 600 mg of an acidic silica gel was dissolved in 3.5 mL of toluene, heated to 90 °C and reacted for 1h, and subjected to column chromatography to obtain a pure product, which was slurried in petroleum ether at 50 °C, filtered and dried to obtain 15 mg of the title compound as a yellowish solid.

¹H NMR (400MHz, CDCl₃) *δ* 7.53 (d, 1H), 7.43 (d, 1H), 7.21 (m, 2H), 6.69 (s, 1H), 3.23 (d, 2H), 3.01 (d, 4H), 2.57 (dd, 2H), 2.03 (d, 2H). ESI-MS m/z 244.14 [M+H]⁺.

### Example 54: 5-(naphthalen-2-yl)-1,2,3,3a,4,6a-hexahydrocyclopenta[c]pyrrole hydrochloride

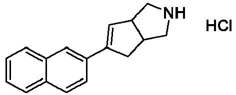

5-(naphthalen-2-yl)-1,2,3,3a,4,6a-hexahydrocyclopenta[c]pyrrole hydrochloride was prepared in the same manner as that in Example 9, except using 2-bromo-naphthalene instead of 3,4-dichlorobromobenzene.

¹H NMR (400MHz, CD₃OD) *δ* 7.85 (m, 4H), 7.76 (dd, 1H), 7.48 (m, 2H), 6.24 (m, 1H), 3.86 (m, 1H), 3.60-3.24 (m, 5H), 3.20 (dd, 1H), 2.91 (d, 1H).

### Example 55: 5-(naphthalen-2-yl)octahydrocyclopenta[c]pyrrole hydrochloride

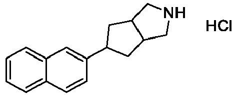

5-(naphthalen-2-yl)octahydrocyclopenta[c]pyrrole hydrochloride was prepared in the same manner as that in Example 3, except using 2-bromo-naphthalene instead of 4-bromo-benzothiophene.

¹H NMR (400MHz, DMSO-*d₆*) *δ* 9.33 (br, 1H), 7.82 (m, 4H), 7.59 (dd, 1H), 7.47 (m, 2H), 3.32 (m, 1H), 3.15 (m, 4H), 2.92 (m, 2H), 2.29 (m, 2H), 1.72 (m, 2H). ESI-MS m/z 238.23 [M+H]⁺.

### Example 56: 5-(naphthalen-1-yl)-1,2,3,3a,4,6a-hexahydrocyclopenta[c]pyrrole dihydrochloride

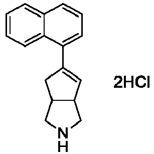

5-(naphthalen-1-yl)-1,2,3,3a,4,6a-hexahydrocyclopenta[c]pyrrole dihydrochloride was prepared in the same manner as that in Example 9, except using 1-bromo-naphthalene instead of 3,4-dichlorobromobenzene.

^{l}H NMR (400MHz, DMSO-*d₆*) *δ* 9.31 (br, 2H), 7.95-7.83 (m, 4H), 7.77 (dd, 1H), 7.50 (m, 2H), 6.28 (d, 1H), 3.70 (br, 1H), 3.40 (dd, 1H), 3.33 (dd, 1H), 3.23-3.05 (m, 3H), 2.99 (dd, 1H), 2.81 (d, 1H). ESI-MS m/z 236.32 [M+H]⁺.

### Example 57: 2-methyl-5-(naphthalen-2-yl)-1,2,3,3a,4,6a-hexahydrocyclopenta[c]pyrrole

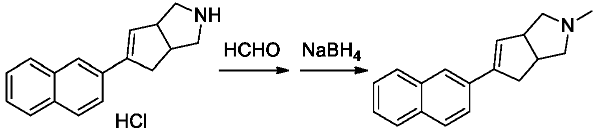

The example 54 (60 mg) was dissolved in 1 mL of a formaldehyde solution (40%), stirred at 75 °C for 4h, added with 2 eq of NaBH₄ under ice-water bath, restored to room temperature to react for 10h, and layered with dichloromethane/water layers. The organic phase was concentrated, and slurried in petroleum ether. After dried, 45 mg of the title compound as a white solid was obtained.

ESI-MS m/z 250.39 [M+H]⁺.

### Example 58: 2-methyl-5-(naphthalen-2-yl)octahydrocyclopenta[c]pyrrole hydrochloride

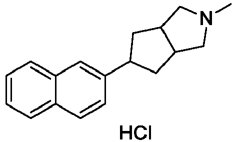

The example 57 (30 mg) was dissolved in 5mL of acetic acid, added with 5mg of 10% Pd/C, purged with hydrogen, and reacted at 25 °C for 24 h. Mass spectrometry showed that the raw material was reacted completely. The reaction solution was filtered, and the acetic acid phase was alkalized with 5 ml of 20% NaOH, and layered with 10 ml of DCM. The organic layer was concentrated, and added with 3 mL of HCl-dioxane in an ice-water bath to form a salt. The solid was filtered, slurried in 5 mL of petroleum ether, filtered and dried to obtain 8mg of the title compound as a white solid.

ESI-MS m/z 252.43 [M+H]⁺.

### Example 59: 5-(Benzofuran-2-yl)-2-methyl-1,2,3,3a,4,6a-hexahydrocyclopenta[c[pyrrole hydrochloride

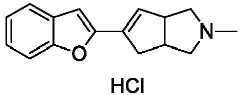

5-(Benzofuran-2-yl)-2-methyl- 1,2,3,3a,4,6a-hexahydrocyclopenta[c]pyrrole was prepared in the same manner as that in Example 57, except using the product of Example 52 instead of the product of Example 54, and the hydrochloride was formed in accordance with Example 10 by using a methanol solution of hydrochloride instead of the tetrahydrofuran solution of maleic acid in Example 10, to give the title compound.

ESI-MS m/z 240.39 [M+H]⁺.

### Example 60: 5-(Benzofuran-2-yl)-2-methyloctahydrocyclopenta[c]pyrrole hydrochloride

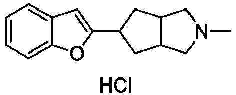

The example 59 (50mg) was dissolved in 5mL of acetic acid, added with 5mg of 10% Pd/C, purged with hydrogen, and reacted at 25 °C for 24 h. Mass spectrometry showed that the raw material was reacted completely. The reaction solution was filtered, and the acetic acid phase was alkalized with 5 ml of 20% NaOH, and layered with 10 ml of DCM. The organic layer was concentrated, and added with 3 mL of HCl-dioxane in an ice-water bath to form a salt. The solid was filtered, slurried in 5 mL of petroleum ether, filtered and dried to obtain 10mg of the title compound as a white solid.

ESI-MS m/z 242.36 [M+H]⁺.

### Example 61: 5-(Benzothiophen-2-yl)-2-methyl-1,2,3,3a,4,6a-hexahydrocyclopenta[c]pyrrole hydrochloride

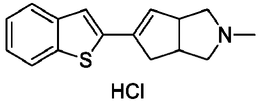

5-(Benzothiophen-2-yl)-2-methyl-1,2,3,3a,4,6a-hexahydrocyclopenta[c]pyrrole was prepared in the same manner as that in Example 57, except using the product of Example 51 instead of the product of Example 54, and the hydrochloride was formed in accordance with Example 10 by using a methanol solution of hydrochloride instead of the tetrahydrofuran solution of maleic acid in Example 10, to give the title compound.

ESI-MS m/z 256.23 [M+H]⁺.

### Example 62: 5-(benzothiophen-2-yl)-2-methyl-octahydrocyclopenta[c]pyrrole hydrochloride

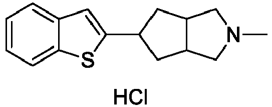

The example 61 (45mg) was dissolved in 5mL of acetic acid, added with 20 mg of 10% Pd/C, purged with hydrogen, and reacted at 25 °C for 24 h. Mass spectrometry showed that the raw material was reacted completely. The reaction solution was filtered, and the acetic acid phase was alkalized with 5 ml of 20% NaOH, and layered with 10 ml of DCM. The organic layer was concentrated, added with 3 mL of HCl-dioxane in an ice-water bath to form a salt. The solid was filtered, slurried in 5 mL of petroleum ether, filtered and dried to obtain 5mg of the title compound as a white solid.

ESI-MS m/z 258.31 [M+H]⁺.

### Example 63: 5-(3,4-dichlorophenyl)octahydrocyclopenta[c]pyrrole hydrochloride

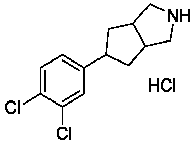

5-(3,4-dichlorophenyl)octahydrocyclopenta[c]pyrrole hydrochloride was prepared in the same manner as that in Example 3, except using 1-bromo-3,4-dichlorobenzene instead of 4-bromo-benzothiophene.

¹H NMR (400MHz, CD₃OD) *δ* 7.73 (d, 1H), 7.45 (m, 2H), 3.47 (m, 2H), 3.37 (m, 1H), 3.05 (m, 2H), 2.93 (m, 2H), 1.96 (m, 2H), 1.82 (m, 2H). ESI-MS m/z 256.27 [M+H]⁺.

### Example 64: 1-(Benzothiophen-5-yl)octahydropyrrolo[3,4-b]pyrrole maleate

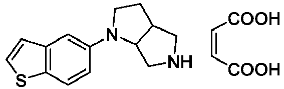

1-(Benzothiophen-5-yl)octahydropyrrolo[3,4-b]pyrrole was prepared in the same manner as that in Example 1, except using 5-bromo-benzothiophene instead of 2-bromonaphthalene and using *t*-butyl hexahydropyrrolo[3,4-b]pyrrole-5(1H)-carboxylate instead of *t*-butyl hexahydropyrrolo[3,4-c]pyrrole-2(1H)-carboxylate, and the maleate was formed in accordance with Example 10 to obtain the title compound.

¹H NMR (400 MHz, DMSO-*d₆*) *δ* 8.88 (s, 2H), 7.80 (d, 1H), 7.66 (d, 1H), 7.29 (d, 1H), 7.03 (d, 1H), 6.81 (d, 1H), 6.01 (s, 2H), 4.29 (t, 1H), 3.62 (m, 1H), 3.48 (m, 1H), 3.33 (m, 1H), 3.19 (m, 4H), 2.17 (m, 1H), 1.94 (m, 1H). ESI-MS *(m*/*z):* 245.21 [M+H]⁺.

### Example 65: 1-(Benzothiophen-5-yl)-5-methyloctahydropyrrolo[3,4-b]pyrrole oxalate

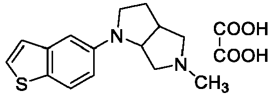

1-(Benzothiophen-5-yl)-5-methyloctahydropyrrolo[3,4-b]pyrrole was prepared in the same manner as that in Example 2, except using the basic product of Example 64 instead of the product of Example 1, and the oxalate was formed in accordance with Example 11 to obtain the title compound.

¹H NMR (400 MHz, DMSO-*d₆*) *δ* 9.50 (brs, 2H), 7.79 (d, 1H), 7.65 (d, 1H), 7.29 (d, 1H), 7.02 (d, 1H), 6.80 (d, 1H), 4.34 (m, 1H), 3.59 (m, 1H), 3.47 (m, 1H), 3.24 (m, 5H), 2.72 (s, 3H), 2.15 (m, 1H), 1.95 (m, 1H). ESI-MS *(m*/*z):* 259.22 [M+H]⁺.

### Example 66: 2-(2,3-dimethylphenyl)octahydropyrrolo[3,4-c]pyrrole maleate

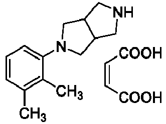

2-(2,3-dimethylphenyl)octahydropyrrolo[3,4-c]pyrrole was prepared in the same manner as that in Example 1, except using 1-bromo-2,3-dimethylbenzene instead of 2-bromonaphthalene, and the maleate was formed in accordance with Example 10 to obtain the title compound.

¹H NMR (400 MHz, DMSO-*d₆*) *δ* 8.68 (s, 2H), 7.02 (t, 1H), 6.87 (m, 2H), 6.02 (s, 2H), 3.52 (m, 2H), 2.98 (m, 6H), 2.86 (m, 2H), 2.21 (s, 3H), 2.16 (s, 3H). ESI-MS *(m*/*z):* 217.23 [M+H]⁺.

### Example 67: 2-(2,3-dimethylphenyl)-5-methyloctahydropyrrolo[3,4-c]pyrrole oxalate

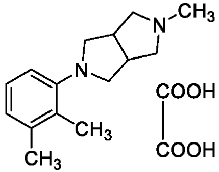

2-(2,3-dimethylphenyl)-5-methyloctahydropyrrolo [3,4-c]pyrrole was prepared in the same manner as that in Example 2, except using the basic product of Example 66 instead of the product of Example 1, and the oxalate was formed in accordance with Example 11 to obtain the title compound.

¹H NMR (400 MHz, DMSO-*d₆*) *δ* 7.02 (t, 1H), 6.87 (m, 2H), 3.59 (m, 2H), 2.82-3.06 (m, 8H), 2.80 (s, 3H), 2.21 (s, 3H), 2.17 (s, 3H). ESI-MS *(m*/*z):* 231.30 [M+H]⁺.

### Example 68: 2- 4-fluorobenzo[b]thiophen-6-yl)octahydropyrrolo[3,4-c]pyrrole dihydrochloride

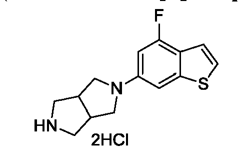

2-(4-fluorobenzo[b]thiophen-6-yl)octahydropyrrolo[3,4-c]pyrrole was prepared in the same manner as that in Example 1, except using 6-bromo-4-fluorobenzo[b]thiophene instead of 2-bromonaphthalene, and the hydrochloride was formed in accordance with Example 10 by using a methanol solution of hydrochloride instead of the tetrahydrofuran solution of maleic acid in Example 10, to give the title compound.

¹H NMR (400 MHz, DMSO-*d₆*) *δ* 9.51 (brs, 2H), 7.40 (d, 1H), 7.27 (d, 1H), 6.99 (s, 1H), 6.65 (dd, 1H), 3.34-3.50 (m, 6H), 2.98-3.18 (m, 4H). ESI-MS *(m*/*z):* 263.18 [M+H]⁺.

### Example 69: 2-(6-fluorobenzothiophen-4-yl)octahydropyrrolo[3,4-c]pyrrole dihydrochloride

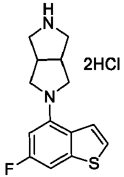

2-(6-fluorobenzothiophen-4-yl)octahydropyrrolo[3,4-c]pyrrole was prepared in the same manner as that in Example 1, except using 4-bromo-6-fluorobenzothiophene instead of 2-bromonaphthalene, and the hydrochloride was formed in accordance with Example 10 by using a methanol solution of hydrochloride instead of the tetrahydrofuran solution of maleic acid in Example 10, to give the title compound.

¹H NMR (400 MHz, CD₃OD) *δ* 7.57 (d, 1H), 7.47 (d, 1H), 7.25 (dd, 1H), 6.63 (dd, 1H), 3.65 (m, 2H), 3.55 (m, 2H), 3.18-3.36 (m, 6H). ESI-MS *(m*/*z):* 263.13 [M+H]⁺.

### Example 70: 2-6-fluorobenzothiohen-4-l-5-methloctahdrorrolo[3,4-c] pyrrole oxalate

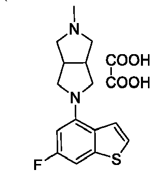

2-(6-fluorobenzothiophen-4-yl)-5-methyloctahydropyrrolo[3,4-c]pyrrole was prepared in the same manner as that in Example 2, except using the basic product of Example 69 instead of the product of Example 1, and the oxalate was formed in accordance with Example 11 to obtain the title compound.

¹H NMR (400 MHz, DMSO-*d₆*) *δ* 7.63 (m, 2H), 7.41 (dd, 1H), 6.59 (dd, 1H), 3.57 (m, 2H), 3.49 (m, 2H), 3.24 (m, 2H), 3.12 (m, 4H), 2.81 (s, 3H). ESI-MS *(m*/*z):* 277.24 [M+H]⁺.

### Example 71: 2-methyl-5-(naphthalen-1-yl)-1,2,3,3a,4,6a-hexahydrocyclopenta[c]pyrrole

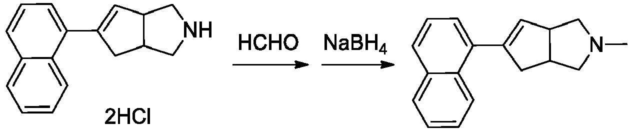

The example 56 (50 mg) was dissolved in 1 mL of a formaldehyde solution (40%), stirred at 75 °C for 4h, added with 2 eq of NaBH₄ under ice-water bath, restored to room temperature to react for 10h, and layered with dichloromethane/water layers. The organic phase was concentrated, slurried in petroleum ether. After dried, 40mg of the title compound as a white solid was obtained.

ESI-MS m/z 250.44 [M+H]⁺.

### Example 72: 2-(3,6-dimethoxynaphthalen-2-yl)-5-methyloctahydropyrrolo[3,4-c]pyrrole dihydrochloride

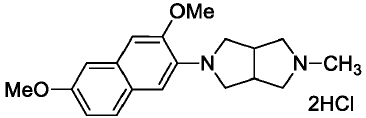

The intermediate 2-(3,6-dimethoxynaphthalen-2-yl)octahydropyrrolo[3,4-c]pyrrole was prepared in the same manner as that in Example 1, except using 2-bromo-3,6-dimethoxynaphthalene instead of 2-bromonaphthalene. Then, 2-(3,6-dimethoxynaphthalen-2-yl)-5-methyloctahydropyrrolo[3,4-c]pyrrole was prepared in the same manner as that in Example 2 by using the intermediate instead of the product of Example 1, and the hydrochloride was formed in accordance with Example 10 by using a methanol solution of hydrochloride instead of the tetrahydrofuran solution of maleic acid in Example 10, to give the title compound.

¹H NMR (400 MHz, CD₃OD) *δ* 8.13 (m, 1H), 7.77 (m, 1H), 7.45 (m, 1H), 7.25 (br, 1H), 7.08 (m, 1H), 4.10 (s, 3H), 4.15-3.96 (m, 4H), 3.91 (s, 3H), 3.84 (m, 2H), 3.65-3.38 (m, 4H), 3.02 (m, 3H). ESI-MS *(m*/*z):* 313.43 [M+H]⁺.

### Example 73: 2-(2,3-dimethoxyphenyl)-5-methyloctahydropyrrolo[3,4-c]pyrrole dihydrochloride

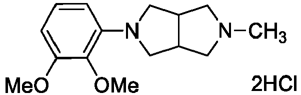

The intermediate 2-(2,3-dimethoxyphenyl)octahydropyrrolo[3,4-c]pyrrole was prepared in the same manner as that in Example 1, except using 1-bromo-2,3-dimethoxybenzene instead of 2-bromonaphthalene. Then, 2-(2,3-dimethoxyphenyl)-5-methyloctahydropyrrolo[3,4-c]pyrrole was prepared in the same manner as that in Example 2 by using the intermediate instead of the product of Example 1, and the hydrochloride was formed in accordance with Example 10 by using a methanol solution of hydrochloride instead of the tetrahydrofuran solution of maleic acid in Example 10, to give the title compound.

¹H NMR (400 MHz, CD₃OD) *δ* 7.40 (d, 1H), 7.19 (t, 1H), 7.11 (t, 1H), 6.96 (d, 1H), 4.05-3.81 (m, 10H), 3.76 (m, 2H), 3.55 (m, 1H), 3.40 (m, 2H), 3.19 (m, 1H), 2.99 (m, 3H),. ESI-MS *(m*/*z):* 263.38 [M+H]⁺.

### Example 74: 2-(benzo[d][1,3]dioxol-4-yl)-5-methyloctahydropyrrolo[3,4-c]pyrrole dihydrochloride

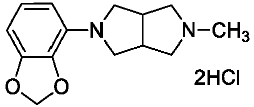

The intermediate 2-(benzo[d][1,3]dioxol-4-yl)octahydropyrrolo[3,4-c]pyrrole was prepared in the same manner as that in Example 1, except using 4-bromobenzo[d][1,3]dioxolane instead of 2-bromonaphthalene. Then, 2-(benzo[d][1,3]dioxol-4-yl)-5-methyloctahydropyrrolo[3,4-c]pyrrole was prepared in the same manner as that in Example 2 by using the intermediate instead of the product of Example 1, and the hydrochloride was formed in accordance with Example 10 by using a methanol solution of hydrochloride instead of the tetrahydrofuran solution of maleic acid in Example 10, to give the title compound.

¹H NMR (400 MHz, CD₃OD) *δ* 6.83 (m, 1H), 6.70 (t, 1H), 6.63 (d, 1H), 5.97 (d, 2H), 3.95 (dd, 1H), 3.77 (t, 2H), 3.69 (d, 1H), 3.50-3.35 (m, 4H), 3.28 (m, 1H), 3.14 (m, 1H), 2.95 (m, 3H). ESI-MS *(m*/*z):* 247.31 [M+H]⁺.

### Example 75: 2-(1,3-difluoronaphthalen-2-yl)-5-methyloctahydropyrrolo[3,4-c]pyrrole dihydrochloride

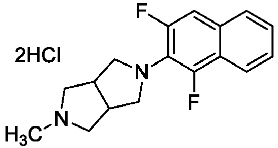

The intermediate 2-(1,3-difluoronaphthalen-2-yl)octahydropyrrolo[3,4-c]pyrrole was prepared in the same manner as that in Example 1, except using 2-bromo-1,3-difluoronaphthalene instead of 2-bromonaphthalene. Then, 2-(1,3-difluoronaphthalen-2-yl)-5-methyloctahydropyrrolo[3,4-c]pyrrole was prepared in the same manner as that in Example 2 by using this intermediate instead of the product of Example 1, and the hydrochloride was formed in accordance with Example 10 by using a methanol solution of hydrochloride instead of the tetrahydrofuran solution of maleic acid in Example 10, to give the title compound.

¹H NMR (400 MHz, CD₃OD) *δ* 7.93 (d, 1H), 7.78 (d, 1H), 7.48 (m, 2H), 7.41 (d, 1H), 3.99 (m, 1H), 3.63-3.39 (m, 6H), 3.30 (m, 1H), 3.12 (m, 1H), 2.95 (m, 4H). ESI-MS *(m*/*z):* 289.32 [M+H]⁺.

### Example 76: 2-(3-fluoronaphthalen-2-yl)-5-methyloctahydropyrrolo [3,4-c]pyrrole dihydrochloride

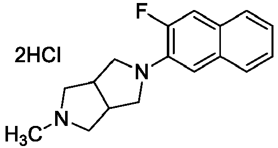

The intermediate 2-(3-fluoronaphthalen-2-yl)octahydropyrrolo[3,4-c]pyrrole was prepared in the same manner as that in Example 1, except using 2-bromo-3-fluoronaphthalene instead of 2-bromonaphthalene. Then, 2-(3-fluoronaphthalen-2-yl)-5-methyloctahydropyrrolo[3,4-c]pyrrole was prepared in the same manner as that in Example 2 by using the intermediate instead of the product of Example 1 and using hydrochloride instead of maleic acid, and the hydrochloride was formed in accordance with Example 10 by using a methanol solution of hydrochloride instead of the tetrahydrofuran solution of maleic acid in Example 10, to give the title compound.

¹H NMR (400 MHz, CD₃OD) *δ* 7.75 (d, 1H), 7.71 (d, 1H), 7.49 (dd, 1H), 7.39 (t, 1H), 7.33 (t, 1H), 7.28 (dd, 1H), 4.00 (dd, 1H), 3.64 (m, 3H), 3.44 (dd, 1H), 3.34 (m, 1H), 3.22 (m, 2H), 3.06 (dd, 1H), 3.00-2.90 (m, 4H). ESI-MS *(m*/*z):* 271.32 [M+H]⁺.

### Example 77: 5-(Benzo[b]thiophen-4-yl)octahydropyrrolo[3,4-b]pyrrole hydrochloride

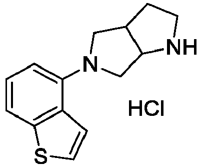

5-(Benzo[b]thiophen-4-yl)octahydropyrrolo[3,4-b]pyrrole was prepared in the same manner as that in Example 1, except using 4-bromo-benzothiophene instead of 2-bromonaphthalene and using t-butyl hexahydropyrrolo[3,4-b]pyrrole-1(2H)-carboxylate instead of t-butyl hexahydropyrrolo[3,4-c]pyrrole-2(1H)-carboxylate, and the hydrochloride was formed in accordance with Example 10 by using a methanol solution of hydrochloride instead of the tetrahydrofuran solution of maleic acid in Example 10, to give the title compound.

¹H NMR (400 MHz, DMSO-*d₆*) *δ* 9.79 (brs, 1H), 9.39 (brs, 1H),7.62 (m, 2H), 7.46 (d, 1H), 7.21 (t, 1H), 6.70 (d, 1H), 4.62 (m, 1H), 3.97 (m, 1H), 3.36 (m, 3H), 3.11 (m, 2H), 2.97 (m, 1H), 2.03 (m, 2H). ESI-MS *(m*/*z):* 245.17 [M+H]⁺.

### Example 78: 2-([1,1'-biphenyl]-2-yl)octahydropyrrolo[3,4-c]pyrrole hydrochloride

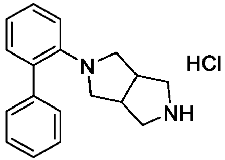

2-([1,1'-biphenyl]-2-yl)octahydropyrrolo[3,4-c]pyrrole was prepared in the same manner as that in Example 1, except using 2-bromo-1,1'-biphenyl instead of 2-bromonaphthalene, and the hydrochloride was formed in accordance with Example 10 by using a methanol solution of hydrochloride instead of the tetrahydrofuran solution of maleic acid in Example 10, to give the title compound.

¹H NMR (400 MHz, DMSO-*d₆*) *δ* 9.21 (brs, 1H), 9.09 (brs, 1H),7.49 (m, 4H), 7.35 (t, 1H), 7.28 (td, 1H), 7.15 (dd, 1H), 7.04 (m, 2H), 3.32 (m, 2H), 2.78 (m, 4H), 2.69 (m, 2H), 2.61 (m, 2H). ESI-MS *(m*/*z):* 265.26 [M+H]⁺.

### Example 79: 2-(2-(methoxymethyl)phenyl)octahydropyrrolo[3,4-c]pyrrole dihydrochloride

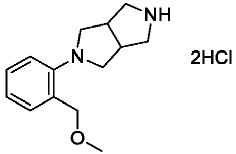

2-(2-(methoxymethyl)phenyl)octahydropyrrolo[3,4-c]pyrrole was prepared in the same manner as that in Example 1, except using 1-bromo-2-(methoxymethyl)benzene instead of 2-bromonaphthalene, and the hydrochloride was formed in accordance with Example 10 by using a methanol solution of hydrochloride instead of the tetrahydrofuran solution of maleic acid in Example 10, to give the title compound.

¹H NMR (400 MHz, DMSO-*d₆*) *δ* 9.23 (brs, 2H), 7.33 (m, 1H), 7.24 (m, 1H), 7.02 (m, 2H), 4.44 (s, 2H), 3.47 (m, 2H), 3.32 (s, 3H), 3.06 (m, 2H), 2.96 (m, 6H). ESI-MS *(m*/*z):* 233.29 [M+H]⁺.

### Example 80: 2-(3-methoxyphenyl)-5-methyloctahydropyrrolo[3,4-c]pyrrole oxalate

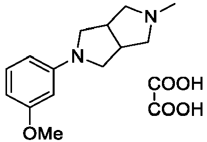

The intermediate 2-(3-methoxyphenyl)octahydropyrrolo[3,4-c]pyrrole was prepared in the same manner as that in Example 1, except using 1-bromo-3-methoxybenzene instead of 2-bromonaphthalene. 2-(3-methoxyphenyl)-5-methyloctahydropyrrolo[3,4-c]pyrrole was prepared in the same manner as that in Example 2, except using the intermediate instead of the product of Example 1, and the oxalate was formed in accordance with Example 11 to obtain the title compound.

¹H NMR (400 MHz, DMSO-*d₆*) *δ* 7.08 (t, 1H), 6.29 (td, 2H), 6.20 (t, 1H), 3.71 (s, 3H), 3.53 (m, 2H), 3.35 (m, 2H), 3.14 (m, 4H), 3.02 (m, 2H), 2.76 (s, 3H). ESI-MS *(m*/*z):* 233.30 [M+H]⁺.

### Example 81: 2-methyl-5-(4-(trifluoromethyl)phenyl)octahydropyrrolo[3,4-c]pyrrole oxalate

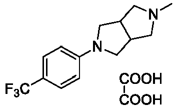

The intermediate 2-(4-(trifluoromethyl)phenyl)octahydropyrrolo[3,4-c]pyrrole was prepared in the same manner as that in Example 1, except using 1-bromo-4-(trifluoromethyl)benzene instead of 2-bromonaphthalene. (ESI-MS (*m*/*z*): 257.32 [M+H]⁺). 2-methyl-5-(4-(trifluoromethyl)phenyl)octahydropyrrolo[3,4-c]pyrrole was prepared in the same manner as that in Example 2, except using the intermediate instead of the product of Example 1, and the oxalate was formed in accordance with Example 11 to obtain the title compound.

¹H NMR (400 MHz, DMSO-*d₆*) *δ* 7.50 (d, 2H), 6.75 (d, 2H), 3.50 (m, 2H), 3.42 (m, 2H), 3.36 (m, 2H), 3.17 (m, 2H), 3.08 (m, 2H), 2.76 (s, 3H). ESI-MS *(m*/*z):* 271.21 [M+H]⁺.

### Example 82: 2-(2-fluorophenyl)octahydropyrrolo[3,4-c]pyrrole maleate

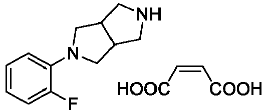

2-(2-fluorophenyl)octahydropyrrolo[3,4-c]pyrrole was prepared in the same manner as that in Example 1, except using 1-bromo-2-fluorobenzene instead of 2-bromonaphthalene, and the maleate was formed in accordance with Example 10 to obtain the title compound.

¹H NMR (400 MHz, DMSO-*d₆*) *δ* 8.72 (s, 2H), 7.11 (m, 1H), 7.06 (m, 1H), 6.85 (m, 2H), 6.01 (s, 2H), 3.48 (m, 2H), 3.35 (m, 2H), 3.20 (m, 2H), 3.02 (m, 4H). ESI-MS *(m*/*z):* 207.18 [M+H]⁺.

### Example 83: 2-(2-fluorophenyl)-5-methyloctahydropyrrolo[3,4-c]pyrrole oxalate

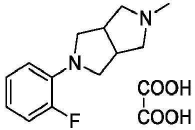

2-(2-fluorophenyl)-5-methyloctahydropyrrolo[3,4-c]pyrrole was prepared in the same manner as that in Example 2, except using the basic product of Example 82 instead of the product of Example 1, and the oxalate was formed in accordance with Example 11 to obtain the title compound.

¹H NMR (400 MHz, DMSO-*d₆*) *δ* 7.09 (m, 2H), 6.88 (m, 2H), 3.55 (m, 2H), 3.36 (d, 2H), 3.08 (m, 4H), 2.97 (m, 2H), 2.77 (s, 3H). ESI-MS *(m*/*z):* 221.28 [M+H]⁺.

### Example 84: 2-(2-ethoxyphenyl)octahydropyrrolo[3,4-c]pyrrole maleate

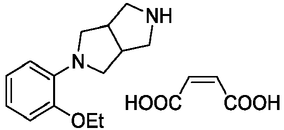

2-(2-ethoxyphenyl)octahydropyrrolo[3,4-c]pyrrole was prepared in the same manner as that in Example 1, except using 1-bromo-2-ethoxybenzene instead of 2-bromonaphthalene, and the maleate was formed in accordance with Example 10 to obtain the title compound.

¹H NMR (400 MHz, DMSO-*d₆*) *δ* 8.66 (s, 2H), 6.88 (m, 3H), 6.77 (dd, 1H), 6.02 (s, 2H), 4.01 (m, 2H), 3.53 (m, 2H), 3.36 (d, 2H), 2.94 (m, 6H), 1.37 (t, 3H). ESI-MS *(m*/*z):* 233.26 [M+H]⁺.

### Example 85: 2-(2-ethoxyphenyl)-5-methyloctahydropyrrolo[3,4-c]pyrrole oxalate

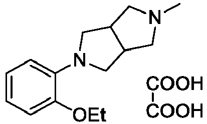

2-(2-ethoxyphenyl)-5-methyloctahydropyrrolo[3,4-c]pyrrole was prepared in the same manner as that in Example 2, except using the basic product of Example 84 instead of the product of Example 1, and the oxalate was formed in accordance with Example 11 to obtain the title compound.

¹H NMR (400 MHz, DMSO-*d₆*) *δ* 6.89 (m, 2H), 6.85 (m, 1H), 6.78 (dd, 1H), 4.01 (m, 2H), 3.73 (m, 2H), 3.41 (m, 2H), 3.02 (m, 2H), 2.82 (s, 3H), 2.50 (m, 4H), 1.38 (t, 3H). ESI-MS *(m*/*z):* 247.30 [M+H]⁺.

### Example 86: 2-methyl-5-(o-tolyl)octahydropyrrolo[3,4-c]pyrrole oxalate

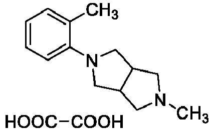

The intermediate 2-methyl-5-(o-methylphenyl)octahydropyrrolo[3,4-c]pyrrole was prepared in the same manner as that in Example 1, except using o-bromo-toluene instead of 2-bromonaphthalene. (ESI-MS *(m*/*z):* 203.34 [M+H]⁺). 2-methyl-5-(o-tolyl)octahydropyrrolo[3,4-c]pyrrole was prepared in the same manner as that in Example 2, except using the intermediate instead of the product of Example 1, and the oxalate was formed in accordance with Example 11 to obtain the title compound.

¹H NMR (400 MHz, DMSO-*d₆*) *δ* 8.23 (brs, 2H), 7.12 (m, 2H), 6.95 (m, 2H), 3.62 (m, 2H), 3.08 (d, 2H), 2.97 (m, 4H), 2.85 (m, 2H), 2.79 (s, 3H), 2.28 (s, 3H). ESI-MS *(m*/*z):* 217.31 [M+H]⁺.

### Example 87: 2-(5-fluoro-2-methoxyphenyl)octahydropyrrolo[3,4-c]pyrrole maleate

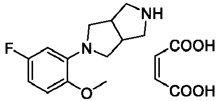

2-(5-fluoro-2-methoxyphenyl)octahydropyrrolo[3,4-c]pyrrole was prepared in the same manner as that in Example 1, except using 1-bromo-2-methoxy-5-fluorobenzene instead of 2-bromonaphthalene, and the maleate was formed in accordance with Example 10 to obtain the title compound.

ESI-MS *(m*/*z):* 237.20 [M+H]⁺.

### Example 88: 2-(5-fluoro-2-methoxyphenyl)-5-methyloctahydropyrrolo[3,4-c]pyrrole oxalate

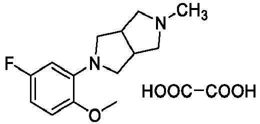

2-(5-fluoro-2-methoxyphenyl)-5-methyloctahydropyrrolo[3,4-c]pyrrole was prepared in the same manner as that in Example 2, except using the basic product of Example 87 instead of the product of Example 1, and the oxalate was formed in accordance with Example 11 to obtain the title compound.

¹H NMR (400 MHz, DMSO-*d₆*) *δ* 9.71 (brs, 2H), 6.90 (dd, 1H), 6.66 (ddd, 1H), 6.58 (dd, 1H), 3.76 (s, 3H), 3.59 (m, 2H), 3.38 (d, 2H), 2.97 (m, 6H), 2.77 (s, 3H). ESI-MS *(m*/*z):* 251.31 [M+H]⁺.

### Example 89: 6-(2,3-dichlorophenyl)decahydropyrrolo[3,4-d]azepine hydrochloride

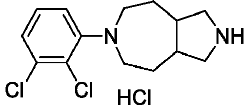

6-(2,3-dichlorophenyl)decahydropyrrolo[3,4-d]azepine was prepared in the same manner as that in Example 6, except using 2,3-dichloro-bromobenzene instead of 2-bromonaphthalene, and the hydrochloride was formed in accordance with Example 3 to obtain the title compound.

¹H NMR (400 MHz, Methanol-*d₄*): *δ* 7.66 (dd, J = 8.1, 1.3 Hz, 1H), 7.54 (dd, J = 8.1, 1.3 Hz, 1H), 7.22 (t, J = 8.1 Hz, 1H), 3.65 (d, J = 7.7 Hz, 1H), 3.62 (d, J = 7.7 Hz, 1H), 3.55 (d, J = 6.6 Hz, 1H), 3.52 (d, J = 6.6 Hz, 1H), 3.11 - 2.95 (m, 4H), 2.79 (m, 2H), 2.11 - 2.02 (m, 2H), 1.95 (m, 2H). ESI-MS(m/z): 285.35 [M+H]⁺

### Example 90: 6-(2,4-dichlorophenyl)decahydropyrrolo[3,4-d]azepine hydrochloride

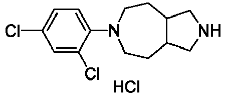

6-(2,4-dichlorophenyl)decahydropyrrolo[3,4-d]azepine was prepared in the same manner as that in Example 6, except using 2,4-dichloro-bromobenzene instead of 2-bromonaphthalene, and the hydrochloride was formed in accordance with Example 3 to obtain the title compound.

¹H NMR (400 MHz, Methanol-*d*₄): δ 7.68 (d, J = 8.6 Hz, 1H), 7.59 (d, J = 2.4 Hz, 1H), 7.27 (dd, J = 8.6, 2.4 Hz, 1H), 3.65 (d, J = 7.7 Hz, 1H), 3.62 (d, J = 7.7 Hz, 1H), 3.56 (d, J = 6.5 Hz, 1H), 3.52 (d, J = 6.5 Hz, 1H), 3.13 - 2.95 (m, 4H), 2.81 (m, 2H), 2.12 - 1.91 (m, 4H). ESI-MS (m/z): 285.35 [M+H]⁺.

### Example 91: 6-(3,4-dichlorophenyl)-2-methyldecahydropyrrolo[3,4-d]azepine hydrochloride

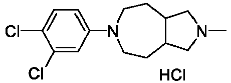

6-(3,4-dichlorophenyl)-2-methyldecahydropyrrolo[3,4-d]azepine was prepared in the same manner as that in Example 2, except using the product of Example 35 instead of the product of Example 1, and the hydrochloride was formed in accordance with Example 3 to obtain the title compound.

ESI-MS *(m*/*z):* 299.30 [M+H]⁺. HPLC: 95.99%.

### Example 92: 7-(5-methylhexahydropyrrolo[3,4-c]pyrrol-2(1H)-yl)-3,4-dihydroquinolin-2(1H)-one dihydrochloride

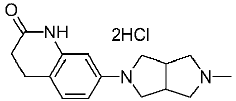

7-(5-methylhexahydropyrrolo[3,4-c]pyrrol-2(1H)-yl)-3,4-dihydroquinolin-2(1H)-o ne was prepared in accordance with the steps in the method of Example 1 by using 5-bromo-3,4-dihydroquinolin-2(1H)-one and 2-methyloctahydropyrrolo[3,4-c]pyrrole as the raw materials, and the hydrochloride was formed in accordance with Example 3 to obtain the title compound. ¹H NMR (400 MHz, Methanol-*d*₄): δ 7.06 (m, 3H), 3.94 (m, 1H), 3.73 (d, J= 11.8 Hz, 1H), 3.70-3.30 (m, 8H), 3.03-2.89 (m, 5H), 2.56 (m, 2H). ESI-MS (m/z): 272.34 [M+H]⁺.

### Example 93: 5-(5-methylhexahydropyrrolo[3,4-c]pyrrol-2(1H)-yl)-3,4-dihydroquinolin-2(1H)-o ne dihydrochloride

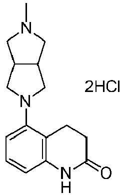

5-(5-methylhexahydropyrrolo[3,4-c]pyrrol-2(1H)-yl)-3,4-dihydroquinolin-2(1H)-o ne was prepared in accordance with the steps in the method of Example 1 by using 2-oxo-1,2,3,4-tetrahydroquinolin-5-yl trifluoromethanesulfonate and 2-methyloctahydropyrrolo[3,4-c]pyrrole as the raw materials, and the hydrochloride was formed in accordance with Example 3 to obtain the title compound. ¹H NMR (400 MHz, Methanol-d₄): δ 6.95 (t, J = 8.0 Hz, 1H), 6.49 (dd, J = 8.2, 1.0 Hz, 1H), 6.37 (dd, J = 7.9, 1.0 Hz, 1H), 3.93 (m, 1H), 3.73 (d, J = 11.9 Hz, 1H), 3.70-3.34 (m, 8H), 2.97 (d, 3H), 2.89 (t, J= 7.7 Hz, 2H), 2.52 (t, J= 7.7 Hz, 2H). ESI-MS (m/z): 272.33 [M+H]⁺.

### Pharmacological experiment

### 1. 5-HTT reuptake activity assay

5-HTT reuptake activity of the compound was conducted using a non-radioactive high-throughput screening system based on fluorescence according to the Reference (Journal of Neuroscience Methods, 2008, 169(1): 168-176). The test was performed in accordance with the instructions of Molecular Devices on their Neurotransmitter transporter uptake assay kit.

The specific operations are as follows:
1) On Day 1, HEK293-hSERT cells were seeded into a 384-well plate at 20000 cells/20 µ1/well, and incubated at 37°C overnight.
2) On Day 2, the positive control compound and the test compound were diluted with 0.1% BSA buffer.
3) The cell plate was taken out from the incubator, and the cell culture medium was drawn from the wells. Each well was added with 25 µl of the test solution. The negative control wells were added with 25 µl of BSA-containing buffer solution, and the positive control wells were added with 25 µl of the positive solution.
4) After incubated at 37°C for 30min, each well was added with 25 µl of a dye solution, and incubated at 37 °C for 30min.
5) After the plate was read on a Flexstation 3 microplate reader, the data were processed by using Graphpad Prism software. The results are shown in Table 1.

**Table 1:**

| Compound | IC₅₀ |
|---|---|
| 1 | < 10nM |
| 2 | < lOnM |
| 6 | < 200nM |
| 7 | < 10nM |
| 8 | < 200nM |
| 9 | < 200nM |
| 10 | < 50nM |
| 11 | < 50nM |
| 12 | < 50nM |
| 13 | < 50nM |
| 14 | < 10nM |
| 15 | < 10nM |
| 16 | < 10nM |
| 17 | < 10nM |
| 19 | < 10nM |
| 21 | < 50nM |
| 22 | < 50nM |
| 23 | < 200nM |
| 26 | < 200nM |
| 27 | < 50nM |
| 28 | < 200nM |
| 29 | < 10nM |
| 30 | < 10nM |
| 31 | < 10nM |
| 32 | < 10nM |
| 35 | < 10nM |
| 36 | < 10nM |
| 37 | < 10nM |
| 38 | < 10nM |
| 39 | < 10nM |
| 41 | < 10nM |
| 42 | < 10nM |
| 43 | < 50nM |
| 48 | < 50nM |
| 50 | < 200nM |
| 51 | < 50nM |
| 52 | < 200nM |
| 54 | < 50nM |
| 55 | < 10nM |
| 56 | < 50nM |
| 57 | < 50nM |
| 59 | < 50nM |
| 61 | < 50nM |
| 62 | < 50nM |
| 64 | < 50nM |
| 65 | < 50nM |
| 66 | < 50nM |
| 67 | < 200nM |
| 68 | < 200nM |
| 69 | < 200nM |
| 70 | < 50nM |
| 71 | < 50nM |
| 75 | < 100nM |
| 76 | < 50nM |
| 81 | < 50nM |
| 89 | < 10nM |
| 90 | < 50nM |
| 93 | < 200nM |
| Positive control Citalopram | 6.45nM |

In the above assay, preferred compounds of the present invention showed serotonin reuptake inhibitory concentrations less than 200 nM (IC₅₀), more preferred compounds exhibited inhibitory concentrations below 100 nM, most preferred compounds exhibit inhibitory concentrations below 50 nM, and particularly interesting compounds exhibit serotonin reuptake inhibitory concentrations below 10 nM.

### 2. 5-HT₃ receptor affinity activity assay

The cell membrane was prepared from HEK293 cells expressing human recombinant 5-HT₃ receptor, the membrane concentration was 2.2 µg per well, the concentration of the isotope ligand 3H-BRL 43694 was 0.5 nM, and the concentration of the non-specific binding compound MDL 72222 was 10 µM.
1) 1 µl of the test compound as well as 1 µl of a serially diluted positive control MDL 72222 and 1 µl of DMSO negative control were transferred to the analysis plate.
2) For cell plating, 100 µl of the membrane stock solution was added to the test plate.
3) 100 µl of isotope-labeled ligand 3H-BRL 43694 was added.
4) The test plate was sealed and incubated at room temperature for 1h.
5) 50 µl of a detergent, i.e. 0.3% polyethyleneimine was added to each well, and the Unifilter-96 GF/C filter plate was soaked at room temperature for at least 30min.
6) When the binding experiment was finished, the reaction mixture was filtered with the GF/C filter plate, and the GF/C filter plate and assay plate were then washed with a cold buffer 4 times.
7) The filter plate was dried at 50°C.
8) After dried, the filter plate was sealed at the bottom with a sealing tape, and added with 50 µl of scintillation fluid (Perkin Elmer Microscint 20 cocktail). The filter plate was sealed with a sealing film at the top.
9) The 3H captured on the filter plate was counted on a Perkin Elmer MicroBeta2 reader, and the data was analyzed with GraphPad Prism 5 software. The results are shown in Table 2.

**Table 2:**

| Compound | inhibition (%) at 1 µM |
|---|---|
| 1 | 106% |
| 2 | 97% |
| 7 | 101% |
| 10 | 86% |
| 11 | 86% |
| 12 | 101% |
| 13 | 70% |
| 14 | 99% |
| 15 | 70% |
| 16 | 96% |
| 17 | 93% |
| 26 | 76% |
| 27 | 104% |
| 28 | 82% |
| 31 | 101% |
| 32 | 103% |
| 35 | 98% |
| 36 | 89% |
| 37 | 80% |
| 38 | 103% |
| 39 | 92% |
| 41 | 86% |
| 42 | 103% |
| 50 | 83% |
| 55 | 75% |
| Positive control MDL72222 | 93% (IC₅₀=9.28 nM) |

It can be seen from table 2 that, the compounds of the present invention have high affinity to 5-HT₃ receptor, and are capable of treating central nervous system diseases associated with 5-HT₃ receptor. 5-HT₃ receptor is located in the postsynaptic membrane, and regulates inhibitory GABA interneurons in different brain regions to regulate the release of various neurotransmitters. Serotonin acts on 5-HT₃ receptor to reduce the release of various neurotransmitters. Therefore, antagonizing 5-HT₃ receptors may cause de-inhibition, thereby increasing the release of neurotransmitters. 5-HT₃ antagonists can enhance the effect of SSRIs antidepressants. Animal experiments showed that the combination of 5-HT₃ antagonist ondansetron and paroxetine can enhance the antidepressant effect of paroxetine. Ondansetron can partially prevent the suppressant effect of paroxetine on 5-HT neuronal firing in the dorsal raphe nucleus, and enhance the paroxetine-induced increase of hippocampal extracellular 5-HT release *(*Pharmacology, Biochemistry and Behavior 2015, 131, 136-142).

Patients with depression often have chronic pain. Chronic physical pain is closely related to depression, and its severity is positively correlated with the severity of depression. Fibromyalgia is a chronic pain disease characterized by extensive pain. Clinical evidences showed that 5-HT₃R antagonists granisetron, ondansetron and tropisetron can significantly reduce fibromyalgia (Arthritis Res Ther, 2006, 8(4), 212.), and therefore it is expected that antidepressant drugs with 5-HT₃R antagonism can alleviate the pain condition of patients with depression.

A large amount of research evidences showed that the 5-HTergic system and other neurotransmitter systems such as cholinergic, dopaminergic, and glutamatergic systems interact in the control of learning and memory. Both the cerebral cortex and the dorsal hippocampus are important structures related to memory. The excitement of 5-HTergic neurons can stimulate cholinergic neurons to release acetylcholine. 5-HT₃R antagonists can inhibit the regulated acetylcholine release of 5-HT without affecting the steady-state release of acetylcholine and prevent cognitive impairment. Overexpression of 5-HT₃R in mice has been shown to enhance learning, memory and concentration. It has been reported in the literature that ondansetron can improve patients' memory and reduce cognitive impairment (Pharmacology & therapeutics, 2010, 128(1): 146-169.).

It can be seen according to the above examples that, some compounds of the present invention have 5-HTT/5-HT₃ receptor multi-target effects, which are beneficial to balance neurotransmitters in the brain, and has good curative effect on the central nervous system diseases. Thus, it can act quickly through the synergistic action of multi-targeting and reduce the side effects caused by drugs.

### 3. In vivo drug efficacy test (mouse forced swimming model)

Experimental animals: C57BL/6 mice (Shanghai Xipuer-Bikai Experimental Animal Co., Ltd.), male, 6 weeks old, weighing 15-20g. ICR mice (Shanghai Xipuer-Bikai Experimental Animal Co., Ltd.), male, 6 weeks old, weighing 20-25g. All the ordered mice were fed in a standard environment for 2-3 weeks before test.

Forced Swimming Test (FST): in the forced swimming apparatus, the water level is 45 cm, and the water temperature is 25°C. Before the experiment, the mice were placed in the experimental room for 1 h for adapting to the environment. At the beginning of the experiment, the mice were placed in the apparatus for 6min, and the whole process was recorded with a camera. When analyzing the data, only the immobility time of the mice in the last 4min was counted, and the result was expressed by mean±SD.

Tail suspension test (TST): the mice were tail fixed to the apparatus with a medical tape for 6 min, the whole process was recorded by the camera. When analyzing the data, only the immobility time of the mice in the last 4min was counted.

The results were analyzed by one-way analysis of variance. The test compounds at the following doses (Table 3) can significantly reduce the immobility time of mice, which has a significant difference compared with the blank control group. po: oral administration; ip: intraperitoneal administration. FST: Forced swimming test; TST: Tail suspension test.

**Table 3:**

| Test Compound | Effective dose (mg/kg) |
|---|---|
| Compound of Example 1 | 5 (po); FST |
| Compound of Example 2 | 5 (po); FST |
| Compound of Example 7 | 10 (ip); TST |
| Compound of Example 10 | 10 (ip); FST |
| Compound of Example 11 | 10 (ip); FST |
| Compound of Example 12 | 3 (ip); FST |
| Compound of Example 13 | 5 (ip); FST |
| Compound of Example 14 | 3 (ip); FST |
| Compound of Example 15 | 2.5 (po); FST |
| Compound of Example 16 | 3 (ip); FST |
| Compound of Example 17 | 5 (po); FST |
| Compound of Example 19 | 10 (ip); FST |
| Compound of Example 21 | 3 (ip); FST |
| Compound of Example 22 | 10 (ip); TST |
| Compound of Example 25 | 10 (ip); TST |
| Compound of Example 28 | 10 (ip); TST |
| Compound of Example 29 | 5 (po); FST |
| Compound of Example 30 | 20 (po); FST |
| Compound of Example 31 | 20 (po); FST |
| Compound of Example 32 | 20 (po); FST |
| Compound of Example 35 | 20 (po); FST |
| Compound of Example 36 | 10 (ip); TST |
| Compound of Example 37 | 10 (ip); FST |
| Compound of Example 38 | 10 (ip); TST |
| Compound of Example 39 | 10 (ip); TST |
| Compound of Example 41 | 10 (ip); TST |
| Compound of Example 50 | 10 (ip); FST |
| Compound of Example 55 | 5 (po); FST |
| Compound of Example 69 | 10 (ip); FST |
| Compound of Example 81 | 10 (ip); FST |
| Duloxetine | 20 (po); FST |
| Citalopram | 20 (po); FST |

The compounds of the present invention are not only strong in activity, but also effective after oral administration. They are characterized in low effective dose and low toxic and side effects, and have curative effect on the central nervous system diseases, especially have a good effect for major depression disorder (MDD), anxiety, obsessive-compulsive disorder, etc.

## Claims

1. A compound represented by formula (I), or a stereoisomer or pharmaceutically acceptable salt thereof: 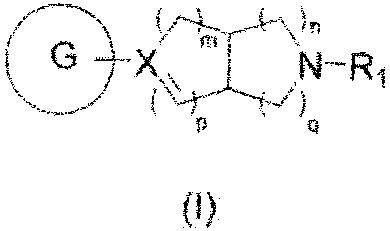 wherein
R₁ is hydrogen or C1 to C20 alkyl; preferably hydrogen or C1 to C10 alkyl; more preferably hydrogen or C1 to C6 alkyl;
m, n, p, q are each independently 0 or 1 or 2 or 3; preferably, m, n, p, q are each independently 1 or 2; provided that m+p is 1, 2, 3 or 4, N+q is 1, 2, 3 or 4, and m+p+n+q is 3, 4, 5, 6 or 7;
X is CR₂, N or C; when X is CR₂ or N, " 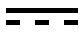 " connected with X represents a single bond, R₂ is hydrogen, hydroxyl or C1 to C6 alkoxy; when X is C, " 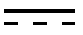 " connected with X represents a double bond;
G ring is phenyl, biphenyl group, naphthyl, tetrahydronaphthyl, dihydroindenyl, monocyclic heterocyclyl or benzoheterocyclyl;
further, G ring is optionally substituted by one or more identical or different substituents; the substituent on G ring is halogen, oxo (=O), hydroxy, halo-Cl to C6 alkyl, C1 to C6 alkanoyl, C1 to C6 alkyl substituted by C1 to C6 alkoxy, C1 to C6 alkyl or C1 to C6 alkoxy, preferably, the substituent on the G ring is halogen, oxo (=O), hydroxy, halo-Cl to C4 alkyl, C1 to C4 alkanoyl, C1 to C4 alkyl substituted by C1 to C6 alkoxy, C1 to C4 alkyl or C1 to C4 alkoxy, more preferably, the substituent on the G ring is halogen, oxo (=O), hydroxy, acetyl, trifluoromethyl, methoxymethyl, methyl, ethyl, methoxy or ethoxy.

2. The compound according to claim 1, or a stereoisomer or pharmaceutically acceptable salt thereof, wherein the G ring is 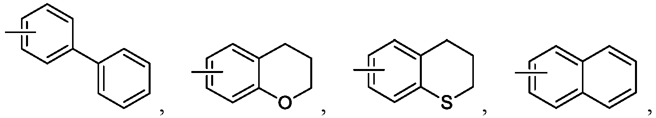 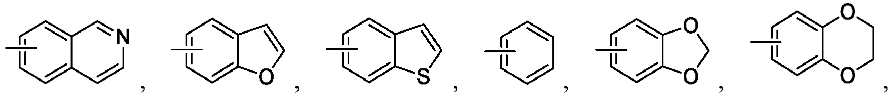 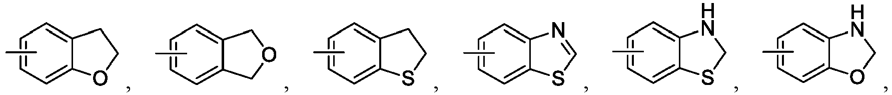 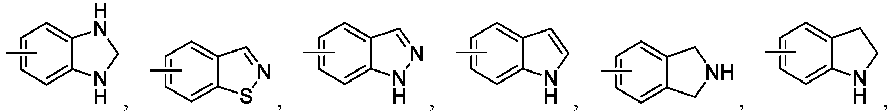 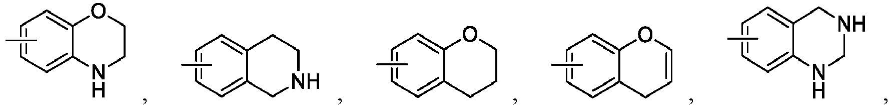 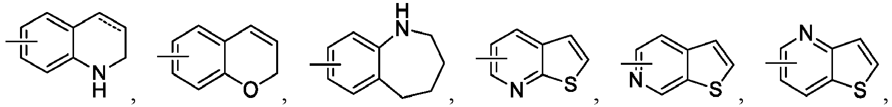 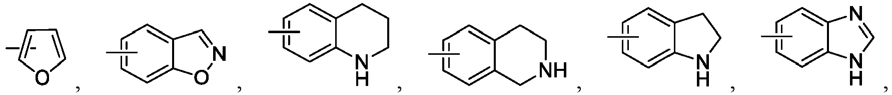 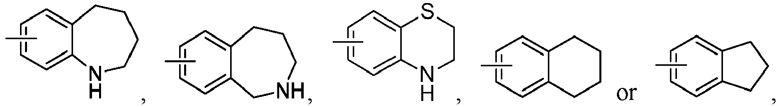 and the G ring is optionally substituted by one or more identical or different substituents, and the substituent on the G ring is defined the same as that defined in claim 1.

3. The compound according to claim 1, or a stereoisomer or pharmaceutically acceptable salt thereof, wherein the G ring is 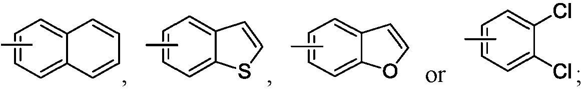 and the G ring is optionally substituted by one or more identical or different substituents, the substituent on the G ring is defined the same as that defined in claim 1.

4. The compound according to claim 1, or a stereoisomer or pharmaceutically acceptable salt thereof, wherein 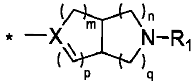 is a group selected from the group consisting of formulae S-1 to S-41: 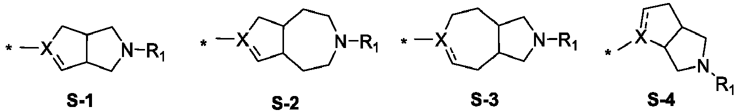 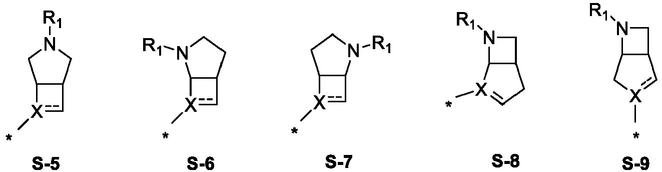 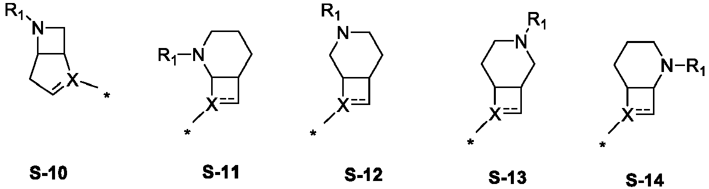 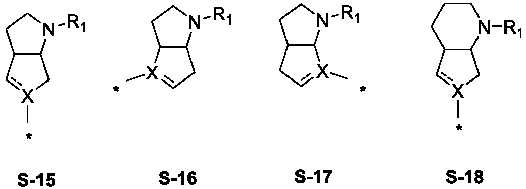 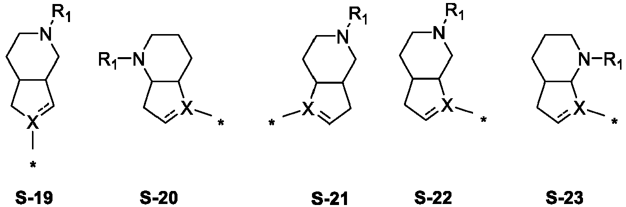 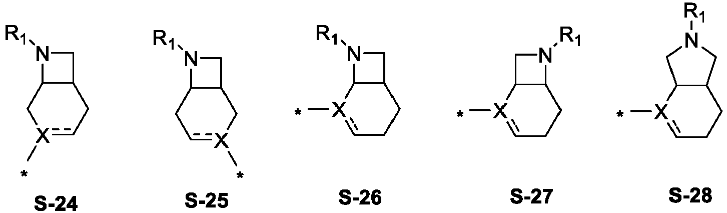 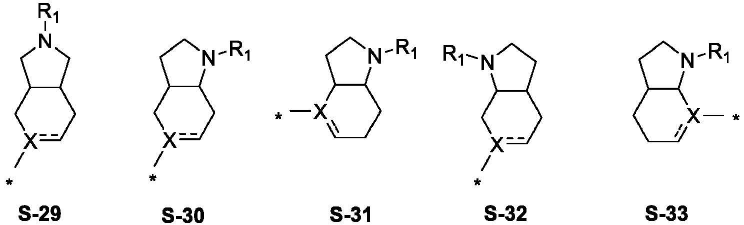 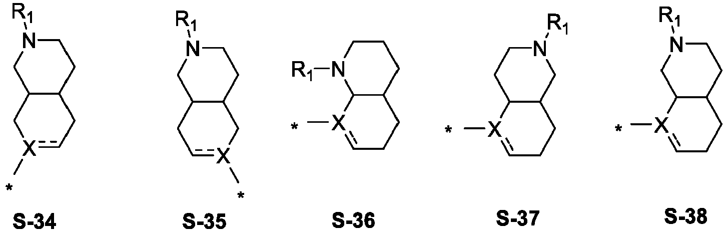 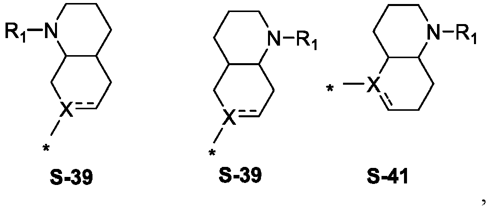 wherein, X and R₁ are defined the same as those defined in claim 1.

5. The compound according to claim 1, or a stereoisomer or pharmaceutically acceptable salt thereof, wherein 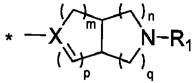 is a group selected from the group consisting of formulae S-1a to S-41a: 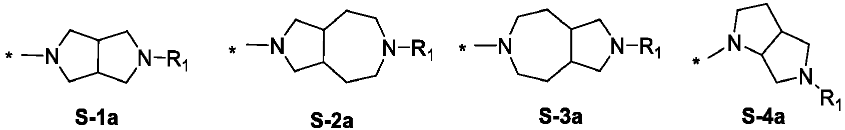 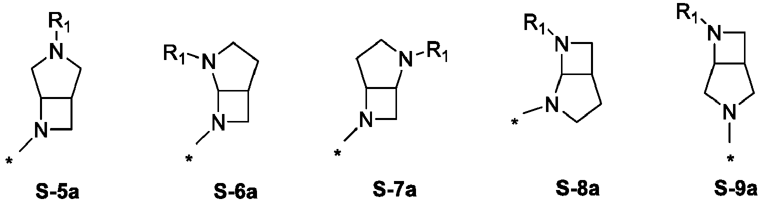 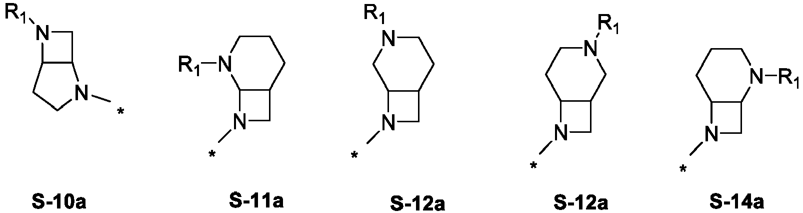 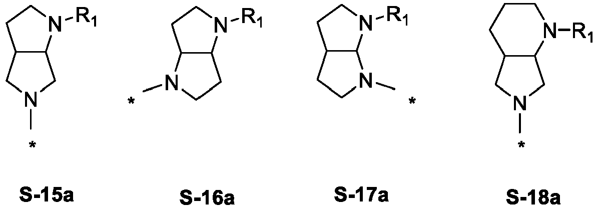 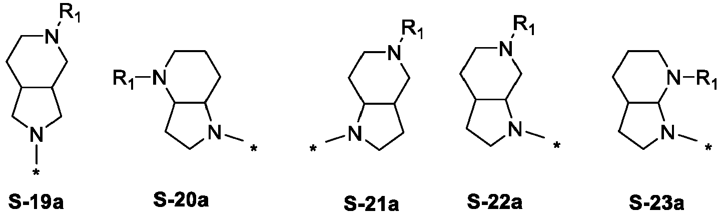 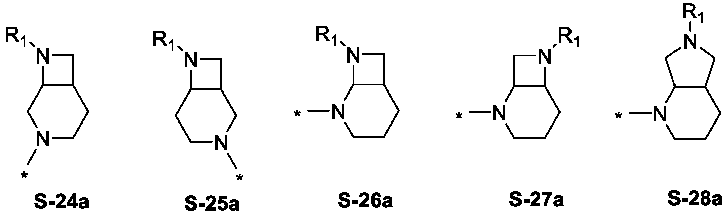 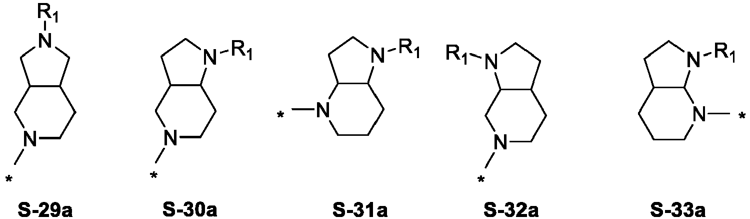 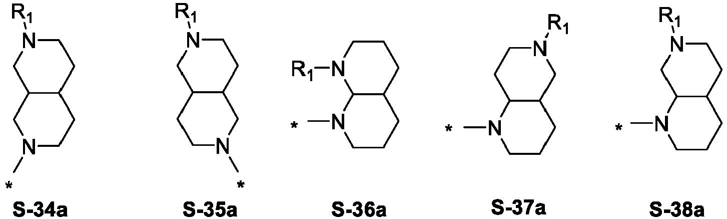 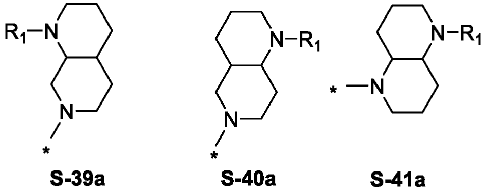 wherein, R₁ is defined the same as that defined in claim 1.

6. The compound according to claim 1, or a stereoisomer or pharmaceutically acceptable salt thereof, wherein the compound is selected from the following compounds:

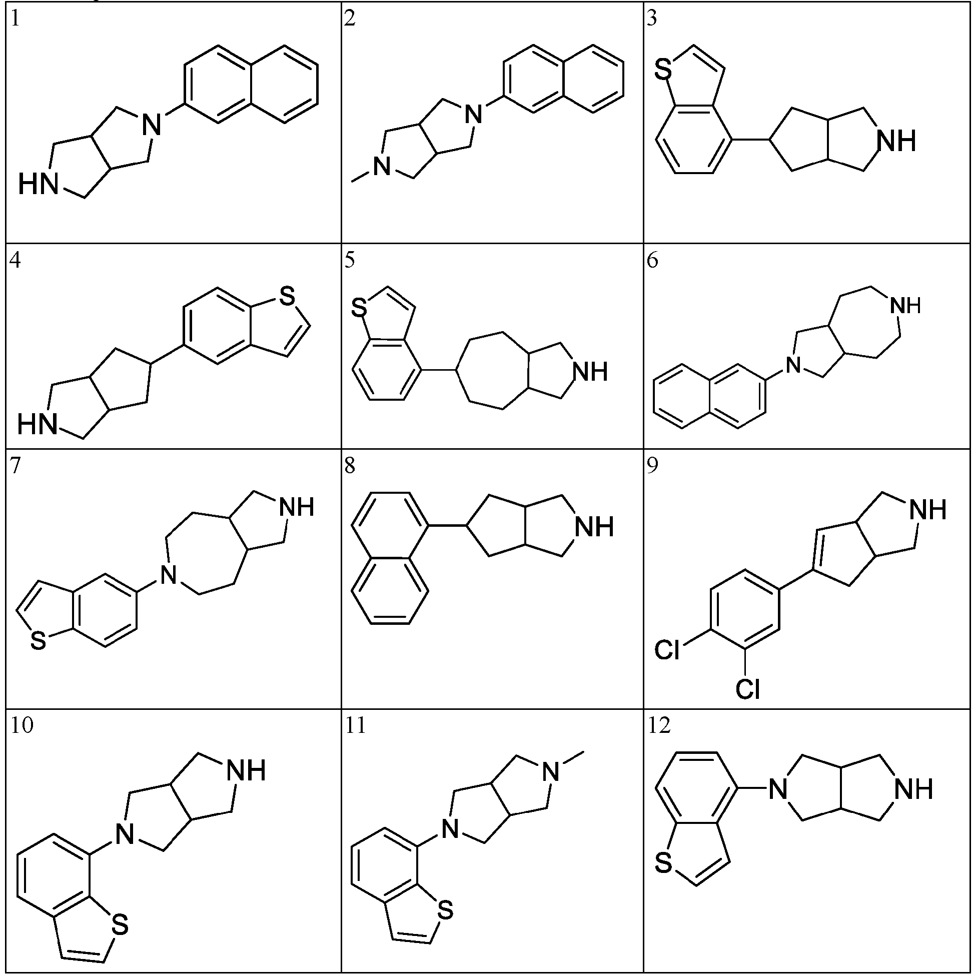

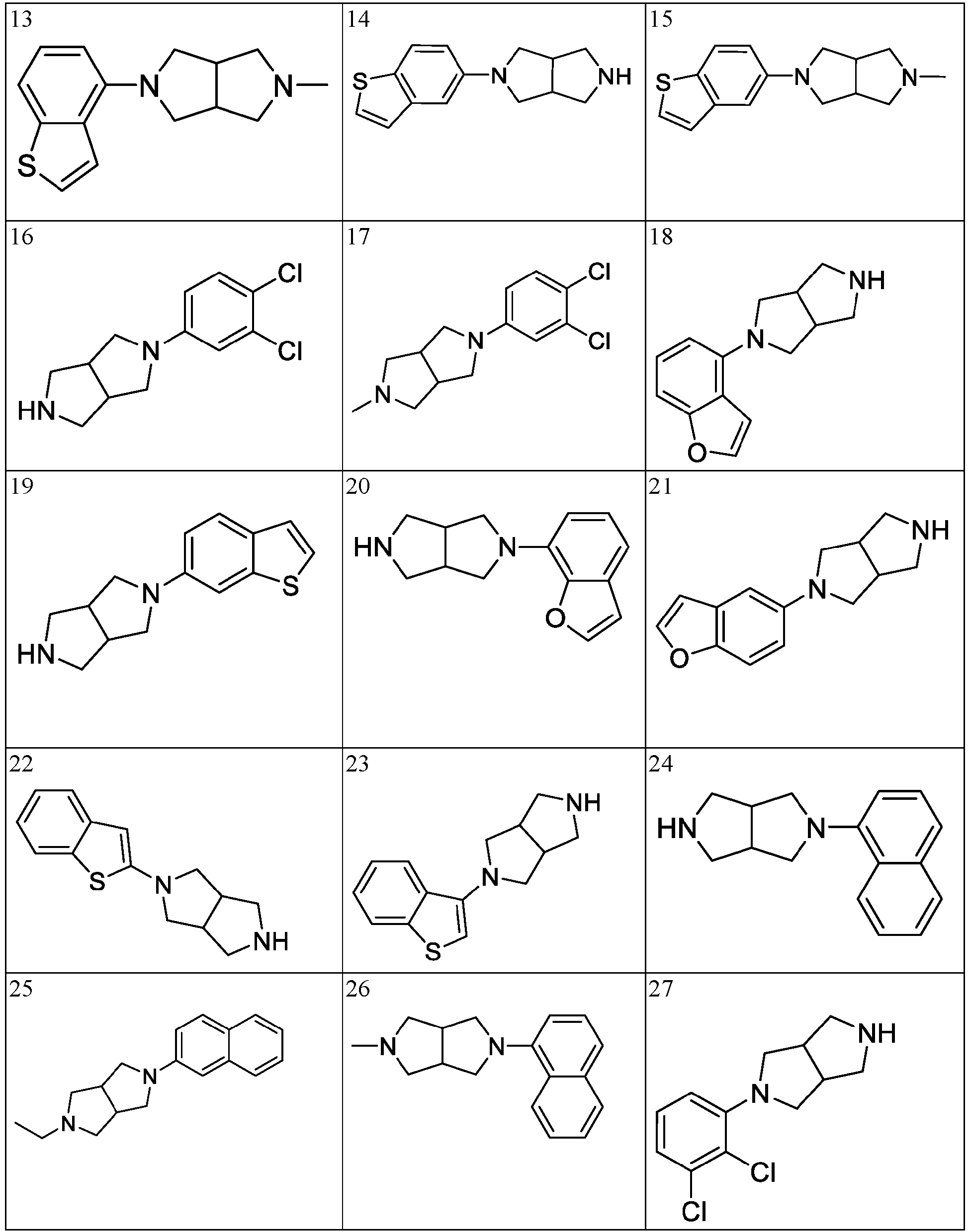

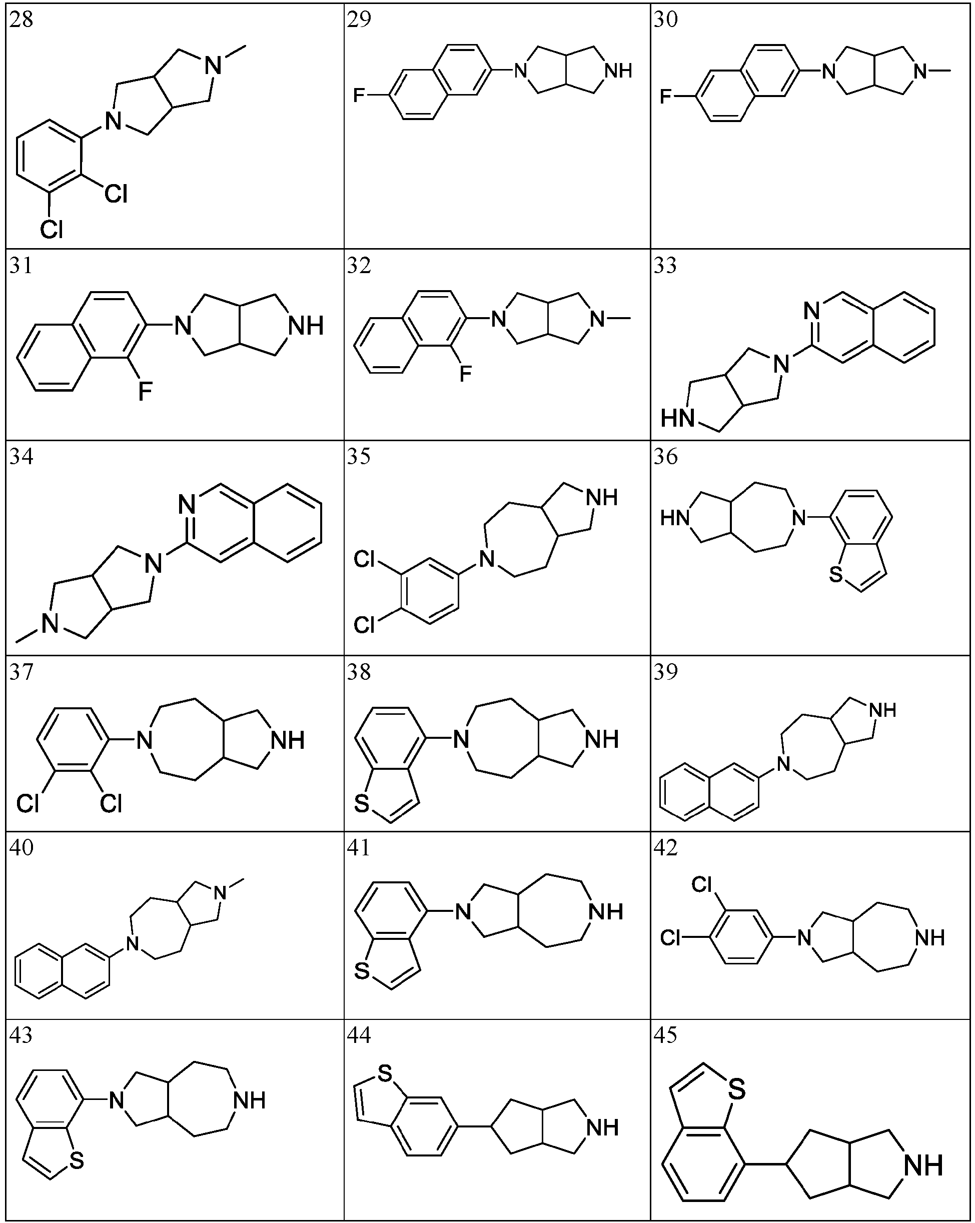

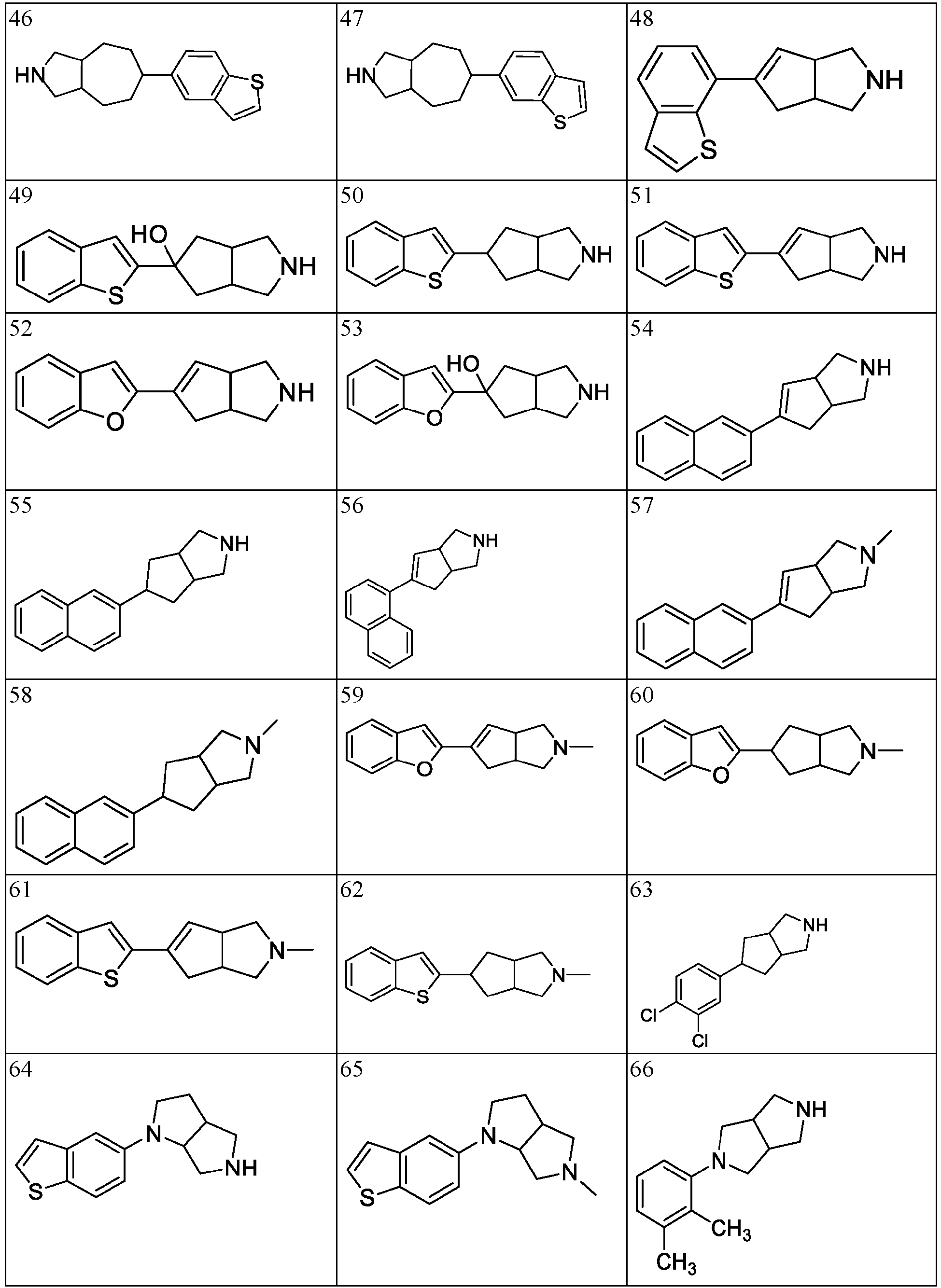

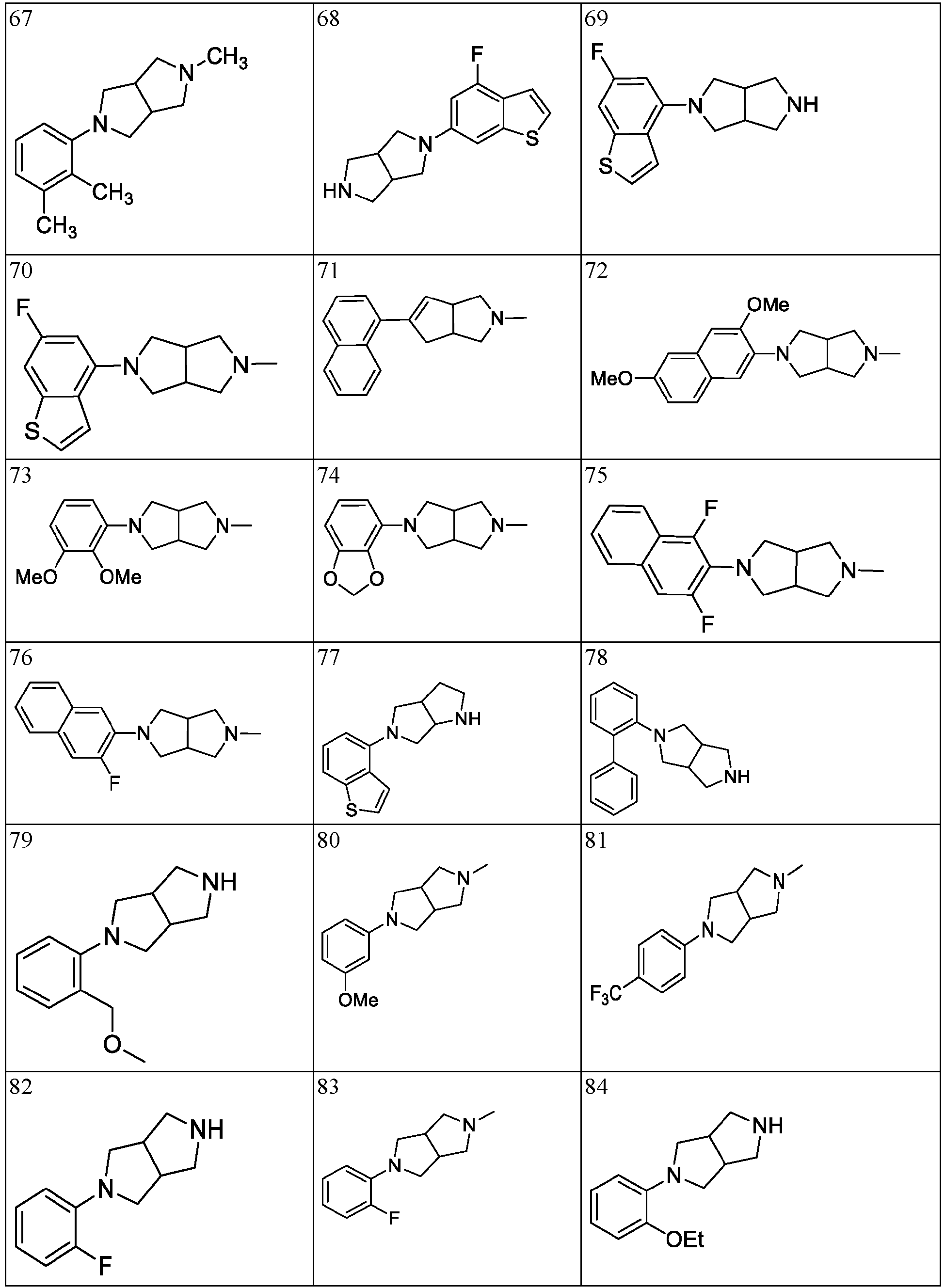

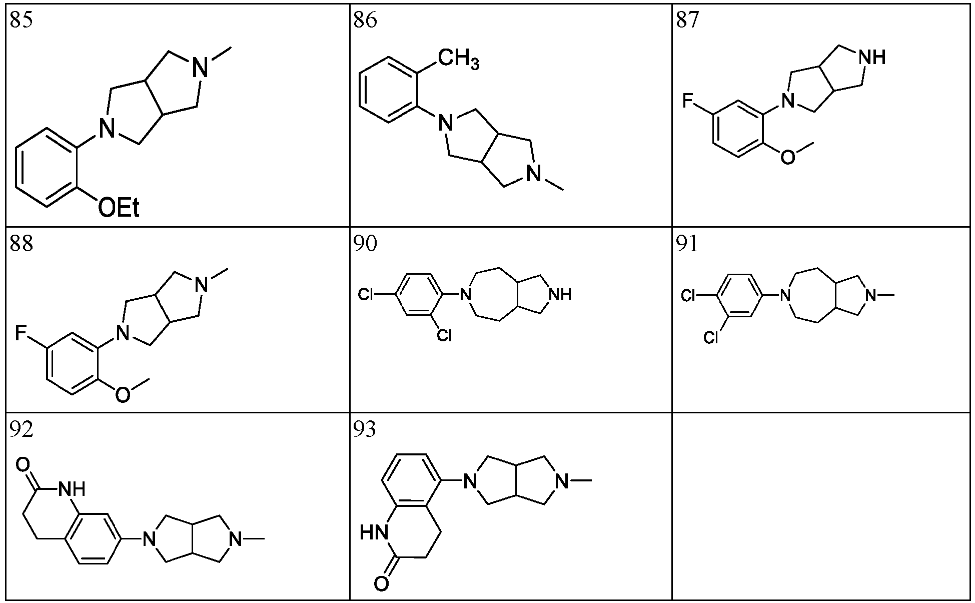

7. A method for preparing a fused ring compound, wherein the method is carried out by one of the following methods 1-7,
Method 1:
a compound represented by formula (II-a) is coupled with a compound represented by formula (III) to obtain a compound represented by formula (Ia), as shown in Scheme 1: 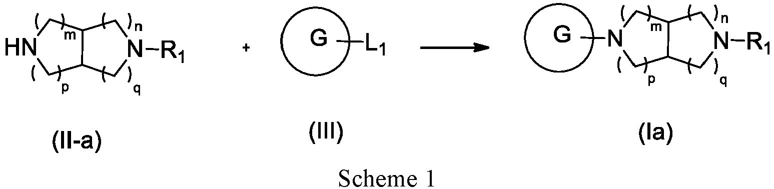
wherein, G ring, m, n, p, q, R₁ are defined the same as those defined in claim 1; L₁ represents halogen, C1 to C6 alkylsulfonyloxy, benzenesulfonyloxy or naphthalenesulfonyloxy, the above C1 to C6 alkylsulfonyloxy, benzenesulfonyloxy and naphthalenesulfonyloxy may be optionally further substituted with one or more substituents selected from the group consisting of halogen, C1 to C6 alkyl, C1 to C6 alkoxy, nitro, hydroxy, amino and C1 to C6 alkanoyl; L₁ is preferably halogen, C1 to C4 alkylsulfonyloxy, benzenesulfonyloxy or naphthalenesulfonyloxy, the above C1 to C4 alkylsulfonyloxy, benzenesulfonyloxy and naphthalenesulfonyloxy may be optionally further substituted by one or more substituents selected from the group consisting of halogen, C1 to C4 alkyl, C1 to C4 alkoxy, nitro, hydroxyl, amino and C1 to C4 alkanoyl; L₁is most preferably chlorine, bromine, methanesulfonyloxy, trifluoromethanesulfonyloxy, benzenesulfonyloxy, naphthalenesulfonyloxy, methylbenzenesulfonyloxy, nitrobenzenesulfonyloxy, aminobenzenesulfonyloxy, chlorobenzenesulfonyloxy, bromobenzenesulfonyloxy or methoxybenzenesulfonyloxy;
Method 2:
a compound represented by formula (II-b) is coupled with a compound represented by formula (III) to obtain a compound represented by formula (IV), and then the amino protecting group is removed to obtain a compound of formula (Ib), which is optionally alkylated or reductive aminated to obtain a compound represented by formula (Ia), as shown in Scheme 2: 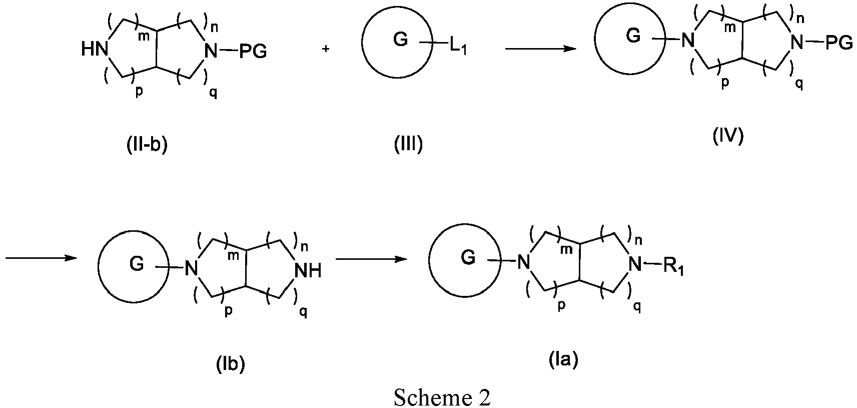
wherein, G ring, m, n, p, q are defined the same as those defined in claim 1; R₁ is C1 to C20 alkyl, L₁ represents halogen, C1 to C6 alkylsulfonyloxy, benzenesulfonyloxy or naphthalenesulfonyloxy, the above C1 to C6 alkylsulfonyloxy, benzenesulfonyloxy and naphthalenesulfonyloxy may be optionally further substituted with one or more substituents selected from the group consisting of halogen, C1 to C6 alkyl, C1 to C6 alkoxy, nitro, hydroxy, amino and C1 to C6 alkanoyl; L₁ is preferably halogen, C1 to C4 alkylsulfonyloxy, benzenesulfonyloxy or naphthalenesulfonyloxy, the above C1 to C4 alkylsulfonyloxy, benzenesulfonyloxy and naphthalenesulfonyloxy may be optionally further substituted by one or more substituents selected from the group consisting of halogen, C1 to C4 alkyl, C1 to C4 alkoxy, nitro, hydroxyl, amino and C1 to C4 alkanoyl; L₁ is most preferably chlorine, bromine, methanesulfonyloxy, trifluoromethanesulfonyloxy, benzenesulfonyloxy, naphthalenesulfonyloxy, methylbenzenesulfonyloxy, nitrobenzenesulfonyloxy, aminobenzenesulfonyloxy, chlorobenzenesulfonyloxy, bromobenzenesulfonyloxy or methoxybenzenesulfonyloxy;
PG is a substituted or unsubstituted benzyl group, acyl type amino protecting group or alkoxycarbonyl type amino protecting group, and the substituent on the benzyl group is one or more independently selected from the group consisting of halogen, trifluoromethyl, C1 to C6 alkyl, C1 to C6 alkoxy and nitro, preferably the acyl type amino protecting group is formyl, acetyl, propionyl, benzoyl, haloacetyl or phthaloyl, and the alkoxycarbonyl type amino protecting group is tert-butoxycarbonyl, benzyloxycarbonyl or 9-fluorenylmethyloxycarbonyl,
Method 3:
a compound represented by formula (V) is coupled with a compound represented by formula (III), and then the amino protecting group is removed to obtain a compound of formula (Ic), which is optionally alkylated or reductive aminated to obtain a compound represented by formula (If) , as shown in Scheme 3: 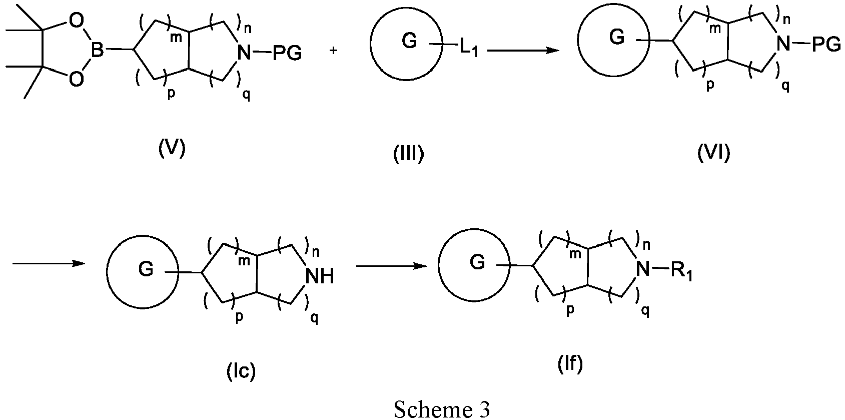
wherein, G ring, m, n, p, q are defined the same as those defined in claim 1; R₁ is C1 to C20 alkyl, L₁ represents halogen, C1 to C6 alkylsulfonyloxy, benzenesulfonyloxy or naphthalenesulfonyloxy, the above C1 to C6 alkylsulfonyloxy, benzenesulfonyloxy and naphthalenesulfonyloxy may be optionally further substituted with one or more substituents selected from the group consisting of halogen, C1 to C6 alkyl, C1 to C6 alkoxy, nitro, hydroxy, amino and C1 to C6 alkanoyl; L₁ is preferably halogen, C1 to C4 alkylsulfonyloxy, benzenesulfonyloxy or naphthalenesulfonyloxy, the above C1 to C4 alkylsulfonyloxy, benzenesulfonyloxy and naphthalenesulfonyloxy may be optionally further substituted by one or more substituents selected from the group consisting of halogen, C1 to C4 alkyl, C1 to C4 alkoxy, nitro, hydroxyl, amino and C1 to C4 alkanoyl; L₁ is most preferably chlorine, bromine, methanesulfonyloxy, trifluoromethanesulfonyloxy, benzenesulfonyloxy, naphthalenesulfonyloxy, methylbenzenesulfonyloxy, nitrobenzenesulfonyloxy, aminobenzenesulfonyloxy, chlorobenzenesulfonyloxy, bromobenzenesulfonyloxy or methoxybenzenesulfonyloxy;
PG is a substituted or unsubstituted benzyl group, acyl type amino protecting group or alkoxycarbonyl type amino protecting group, and the substituent on the benzyl group is one or more independently selected from the group consisting of halogen, trifluoromethyl, C1 to C6 alkyl, C1 to C6 alkoxy and nitro, preferably the acyl type amino protecting group is formyl, acetyl, propionyl, benzoyl, haloacetyl or phthaloyl, and the alkoxycarbonyl type amino protecting group is tert-butoxycarbonyl, benzyloxycarbonyl or 9-fluorenylmethyloxycarbonyl,
Method 4:
a compound represented by formula (II-a) is reacted with a compound represented by formula (X) through carbonyl addition to obtain a compound represented by formula (VIII), and then the amino protecting group is removed to obtain a compound of formula (Id), as shown in Scheme 4: 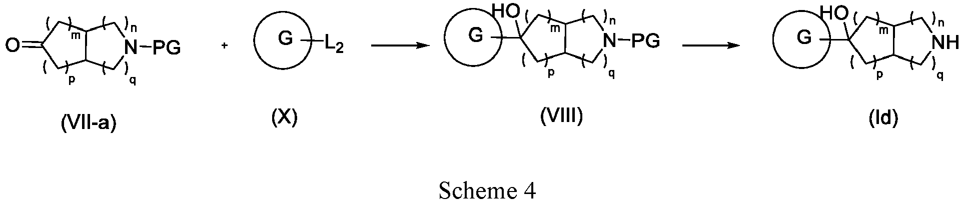
wherein, G ring, m, n, p, q are defined the same as those defined in claim 1; L₂ represents Li, MgBr, MgCl, MgI, ZnBr, ZnCl or ZnI;
PG is an acyl type amino protecting group or alkoxycarbonyl type amino protecting group, preferably the acyl type amino protecting group is formyl, acetyl, propionyl, benzoyl, haloacetyl or phthaloyl, and the alkoxycarbonyl type amino protecting group is tert-butoxycarbonyl, benzyloxycarbonyl or 9-fluorenylmethyloxycarbonyl,
Method 5:
a compound represented by formula (VII-a) is reacted with a compound represented by formula (X) through carbonyl addition to obtain a compound of formula (VIII), and then dehydration and amino deprotection are simultaneously performed to obtain a compound of formula (Ie), which is optionally alkylated or reductive aminated to obtain a compound represented by formula (Ig), as shown in Scheme 5: 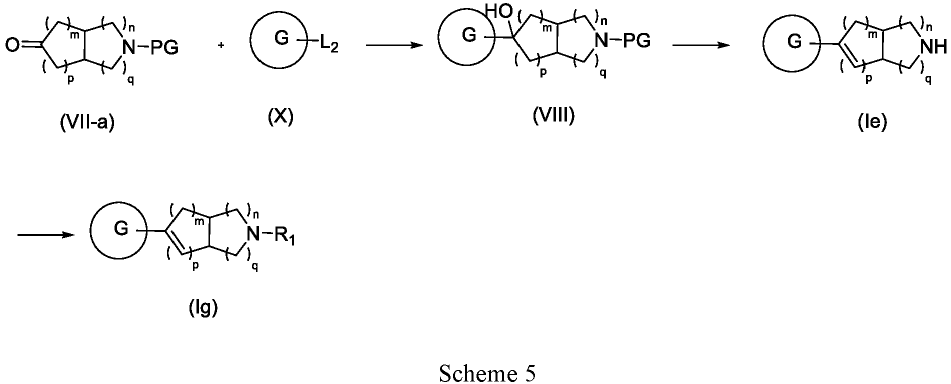
wherein, G ring, m, n, p, q are defined the same as above; R₁ is C1-C20 alkyl; L₂ represents Li, MgBr, MgCl, MgI, ZnBr, ZnCl or ZnI;
PG is an acyl type amino protecting group or alkoxycarbonyl type amino protecting group, preferably the acyl type amino protecting group is formyl, acetyl, propionyl, benzoyl, haloacetyl or phthaloyl, and the alkoxycarbonyl type amino protecting group is tert-butoxycarbonyl, benzyloxycarbonyl or 9-fluorenylmethyloxycarbonyl,
Method 6:
a compound represented by formula (VII-b) is reacted with a compound represented by formula (X) through carbonyl addition to obtain a compound represented by formula (Ih), which is then dehydrated to obtain a compound of formula (Ig), as shown in Scheme 6: 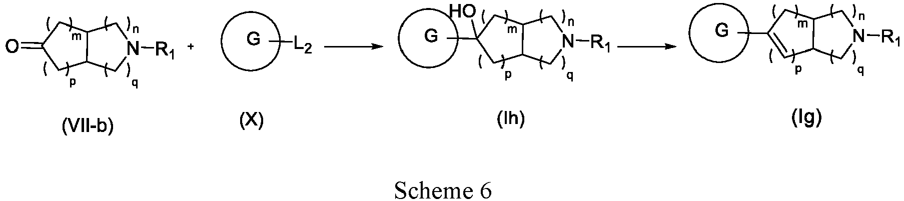
wherein, G ring, m, n, p, q are defined the same as above; R₁ is C1-C20 alkyl; L₂ represents Li, MgBr, MgCl, MgI, ZnBr, ZnCl or ZnI;
Method 7:
a compound represented by formula (Ie) or (Ig) is subjected to hydrogenation reduction to obtain a compound represented by formula (Ic) or (If) respectively: 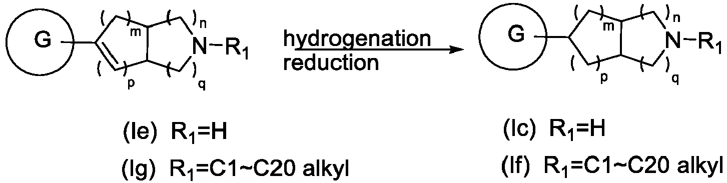
wherein, G ring, m, n, p, q are defined the same as above.

8. A pharmaceutical composition, comprising a therapeutically effective amount of one or more selected from the group consisting of the above compound represented by formula (I), the stereoisomers thereof and the pharmaceutically acceptable salts thereof, and optionally a pharmaceutically acceptable carrier.

9. Use of the compound according to claim 1, or a stereoisomer or pharmaceutically acceptable salt thereof, or the pharmaceutical composition according to claim 8 in preparation of a drug for prevention and/or treatment of a central nervous system disease.

10. Use according to claim 9, wherein the central nervous system disease is selected from the group consisting of affective disorder; mental disorder; mood disorder; depression; intrinsic depression; major depression; uncontrollable depression; dysthymic disorder; cyclic affective disorder; panic attack; panic disorder; social phobia; obsessive-compulsive and behavioral disorders; impulsive disorders; post-traumatic stress disorders; anxiety disorders; acute stress disorders; hysteria; anorexia nervosa; sleep disorders; adaptive disorders; autism; neuropathic headache; mania; hyperactivity; fibromyalgia; neuropathic pain; attention deficit/hyperactivity diseases and tics, **etc.,** preferably, the sleep disorder is sleep apnea, insomnia, narcolepsy, or cataplexy; preferably, the central nervous system disease is selected from the group consisting of depression; anxiety; and obsessive-compulsive and behavioral disorders, preferably, the neuropathic pain includes, but is not limited to, postherpetic neuralgia, reflex sympathetic dystrophy/causalgia or nerve trauma, prosthetic pain, and peripheral neuropathy, preferably, the herpes is herpes zoster; the peripheral neuropathy is diabetic neuropathy or neuropathy caused by long-term drinking of alcohol.
